# EUROPEAN PATENT SPECIFICATION

(11) **EP 3 691 686 B1**
(45) Date of publication and mention of the grant of the patent: **18.12.2024**
(21) Application number: 18865283.8
(22) Date of filing: 08.10.2018
(51) Int. Cl.: A61K 45/06, A61K 47/34, A61K 47/42, A61L 31/14, A61P 35/00, C12Q 1/6886

(54) **DETECTION OF METASTATIC DISEASE AND RELATED METHODS**
NACHWEIS VON METASTATISCHER ERKRANKUNG UND ZUGEHÖRIGE VERFAHREN
DÉTECTION DE MALADIE MÉTASTASIQUE ET PROCÉDÉS ASSOCIÉS

(30) Priority: 06.10.2017 US 201762569460 P; 12.10.2017 US 201762571702 P
(43) Date of publication of application: 12.08.2020
(73) Proprietor: The Regents Of The University Of Michigan, Ann Arbor, MI 48109-2590 (US)
(72) Inventor: SHEA, Lonnie D., Ann Arbor, MI 48109 (US); OAKES, Robert S., College Park, MD 20742 (US); BUSHNELL, Grace, Ann Arbor, MI 48109 (US); JERUSS, Jacqueline S., Ann Arbor, MI 48109 (US)
(74) Representative: Barker Brettell LLP
(86) International application number: PCT/US2018/054859
(87) International publication number: WO 2019/071257

(56) References cited:
- WO-A1-2017/120486
- WO-A2-2016/057702
- WO-A2-2016/172710
- US-A1- 2014 072 510
- US-A1- 2014 274 769
- US-A1- 2015 285 810
- US-A1- 2018 050 068
- SIEH SHIRLY ET AL: "Paracrine interactions between LNCaP prostate cancer cells and bioengineered bone in 3D in vitro culture reflect molecular changes during bone metastasis", BONE, vol. 63, 1 June 2014 (2014-06-01), GB, pages 121 - 131, XP055813952, ISSN: 8756-3282, DOI: 10.1016/j.bone.2014.02.001
- OAKES ROBERT S. ET AL: "Metastatic Conditioning of Myeloid Cells at a Subcutaneous Synthetic Niche Reflects Disease Progression and Predicts Therapeutic Outcomes", CANCER RESEARCH, vol. 80, no. 3, 1 February 2020 (2020-02-01), US, pages 602 - 612, XP055813911, ISSN: 0008-5472, Retrieved from the Internet <URL:https://www.ncbi.nlm.nih.gov/pmc/articles/PMC7002274/pdf/nihms-1541533.pdf> DOI: 10.1158/0008-5472.CAN-19-1932
- AGUADO ET AL.: "Engineering the Pre-Metastatic Niche", NATURE BIOMEDICAL ENGINEERING, vol. 1, 13 June 2017 (2017-06-13), pages 1 - 12, XP055693924
- XI ET AL.: "RNA Biomarkers: Frontier of Precision Medicine for Cancer", NON-CODING RNA, vol. 3, no. 1, 20 February 2017 (2017-02-20), pages 1 - 17, XP055589364

## Description

### CROSS REFERENCE TO RELATED APPLICATIONS

This application claims priority to U.S. Provisional Application No. 62/569,460, filed on October 6, 2017, and U.S. Provisional Application No. 62/571,702, filed on October 12, 2017.

### INCORPORATION BY REFERENCE OF MATERIAL SUBMITTED ELECTRONICALLY

Incorporated by reference in its entirety is a computer-readable nucleotide/amino acid sequence listing submitted concurrently herewith and identified as follows: 230,480 byte ASCII (Text) file named "52538_SeqListing.txt," created on October 3, 2018.

### BACKGROUND

Cancer is the second leading cause of death in the United States, claiming more than half a million American lives every year. Cancer's lethality is due to its ability to spread throughout the body, or its ability to become metastatic. Metastatic cancer does not always cause symptoms, and, even if symptoms do occur, the nature and frequency of the symptoms depend on the size and location of the metastatic tumors. Also, some metastatic cancer symptoms, such as pain, headache, dizziness, shortness of breath, and swelling of the belly, are common to other diseases and disorders and not unique to metastatic cancer. Once cancer becomes metastatic, it is often difficult to control and most metastatic cancers are not curable.

For these reasons and others, much effort has gone into developing methods for detecting metastatic cancer in the early stages. Current methodologies for metastatic cancer detection include blood tests to identify any metastases in the liver or bones, bone scans, X-rays, and computerized tomography (CT) scans. Improvements in these basic techniques include designing better imaging agents. For example, Karathanasis, Efstathios "Detecting Early Onset of Metastatic Disease Using MRI", Imaging Technology News, May 15, 2013, describes a detection method using an imaging agent that targets a specific biomarker (alpha(v)beta(3) integrin) expressed by metastatic cancer cells. As discussed in Robinson, "The Early Detection of Liver Metastases" Cancer Imaging 2(2): 1-3 (2002), certain methodologies are limited to the size of the metastatic lesions detected; some imaging techniques (e.g., CT, magnetic resonance imaging (MRI)), detect relatively larger lesions, while other techniques (e.g., radionuclide, or doppler perfusion) detect smaller lesions. Azarin et al., Nat Commun 6: 8094 (2015) describes biomaterial scaffolds that recruit or capture metastatic cells and the subsequent identification or detection of such metastatic cells in the scaffolds through a label-free detection system using inverse spectroscopic optical coherence tomography. (IS-OCT). Common to all of these strategies is the reliance of the imaging, detection, identification or characterization of the metastatic cells.

US201407251 0A1 relates to synthetic scaffolds for metastasis detection.

For multiple reasons, it would be advantageous to detect metastatic cancer at even earlier stages than the stage(s) at which the above methodologies detect. It would be beneficial, for example, to detect metastatic cancer before cancer cell homing, migration, and/or colonization occur.

### SUMMARY

The present invetion is defined by the appended claims. The present disclosure is directed generally to synthetic scaffolds that form engineered pre-metastatic niches (pMN) and methods of use and diagnosis related thereto. The disclosure includes methods for identifying a subject's metastatic status, risk or predisposition via detection of changes in the expression profile of cells captured in the synthetic scaffolds.

Presented herein are data demonstrating that expression profiles, such as, for example, gene expression profiles, RNA expression profiles, protein expression profiles (e.g., cytokine and chemokine expression profiles) of cells captured in synthetic engineered pMN change over time after tumorigenesis and that such expression profiles of tumor-bearing animals differ from those of tumor-free animals. Also presented herein are data showing that such expression profiles of cells localized in the scaffolds change over time after treatment (e.g., surgical tumor resection) and that the expression profiles of treated animals differ from those of untreated animals.

Without being bound to any particular theory, these expression profiles of the cells of the scaffolds are representative of a subject's status with regard to metastatic disease and the potential, risk, or likelihood therefor, and therefore, such expression profiles allow for early diagnosis or detection of metastatic cancer. For example, such profiles may be used to monitor an animal with a tumor or cancer before, during or after the onset of metastatic disease or before, during or after the onset of tumor cell colonization at a metastatic site. Because the expression profiles represent the subject's status in this regard, the expression profiles may be used to determine a subject's therapy, e.g., determine a subject's need for therapy for metastatic disease. In related aspects, the expression profiles may also be useful for determining the efficacy of a treatment administered to or performed on a subject receiving such treatment. In yet other related aspects, the expression profiles may be used in methods of preventing or delaying the onset of metastatic disease or reducing metastatic potential.

Accordingly, disclosed, but not part of the claimed invention, are methods of determining a subject's status with regard to metastatic disease. In exemplary embodiments, the subject has a tumor or cancer. Provided herein are methods of determining a subject's metastatic potential. In exemplary aspects, the method comprises measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level.

Also disclosed, but not part of the claimed invention, are methods of detecting metastatic disease, or a predisposition thereto, in a subject in need thereof. In exemplary embodiments, the method comprises measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level.

The present disclosure provides methods which are not part of the claimed invention, of monitoring a subject's metastatic potential or metastatic disease. In exemplary embodiments, the methods comprise measuring a level of expression of a gene, an RNA, or a protein, or a combination thereof, in a sample obtained from a synthetically-engineered pMN implanted in the subject at a first time point and measuring the expression level of the gene, RNA, or protein in a sample obtained from the synthetically-engineered pMN at a second time point, wherein the expression level measured at the first time point is compared to the expression level measured at the second time point. In exemplary aspects, the first time point occurs before a treatment and the second time point occurs after a treatment and the method is a method of determining the efficacy of a treatment for metastatic disease.

Methods of determining treatment for a subject with a tumor or cancer are provided by the present disclosure , but not as part of the claimed invention. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level.

The present disclosure also provides a method which is not part of the claimed invention, for determining a subject's need for metastatic disease therapy. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level.

The present disclosure also provides methods which are not part of the claimed invention, of preventing or delaying the onset of metastatic disease. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA or protein in the sample is compared to a control level, and administering a metastatic disease therapy based on the measured level of expression.

Further provided herein but not as part of the claimed invention, are systems comprising: a processor; a memory device coupled to the processor, and machine readable instructions stored on the memory device. Computer-readable storage media having stored thereon machine-readable instructions executable by a processor and methods implemented by a processor in a computer are additionally provided herein.

### BRIEF DESCRIPTION OF THE DRAWINGS

Figure 1 is an illustration of the process of a circulating tumor cell (CTC) homing to and colonizing at a secondary site and the influence of the pMN immune microenvironment.
Figure 2 demonstrates that microporous polymer scaffolds create a synthetic pMN in vivo.
Figure 3 is an outline for a pMN signature and monitoring of metastatic potential. Metastatic potential as a diagnostic based on the magnitude of conditioning in distal pMN sites.
Figure 4 is an outline of an experiment involving mice having an implanted scaffold and later inoculated with tumor cells. Gene expression of scaffold samples is analyzed at different time points after tumorigenesis.
Figure 5 is a volcano plot for all genes analyzed via qRT-PCR.
Figure 6 is a pair of exemplary heat maps demonstrating PLS-DA classification and clustering base on scaffold gene expression in tumor-bearing (TB) or tumor-free (TF) mice 7 or 21 days post tumor inoculation.
Figure 7 is a pair of box plots demonstrating the change in average gene expression in tumor-bearing (TB) mice over time post tumor inoculation.
Figure 8 is a set of box plots demonstrating the changes in average gene expression in tumor-bearing (TB) mice over time post tumor inoculation.
Figure 9 is a box plot of gene expression scores from SVD 1^{st} singular vector.
Figure 10 is a box plot of random forest prediction of Healthy Control animals (HC) or tumor-bearing (TB) mice.
Figure 11 is a graph of random forest prediction (% tumor bearing probability) vs. gene expression score from SVD.
Figure 12 is a graph of random forest prediction (% tumor bearing probability) vs. gene expression score from SVD for mice that were treated by surgical resection.
Figure 13 is a graph of normalized S100A8 gene expression as a function of time.
Figure 14 is a graph of cytokine/chemokine expression for Healthy control animals and tumor-bearing animals.
Figure 15 is a graph of gene expression change relative to protein expression.
Figure 16 is a system diagram of a processing system for performing the techniques described herein, including, assessing a subject's metastatic potential, in accordance with an example.

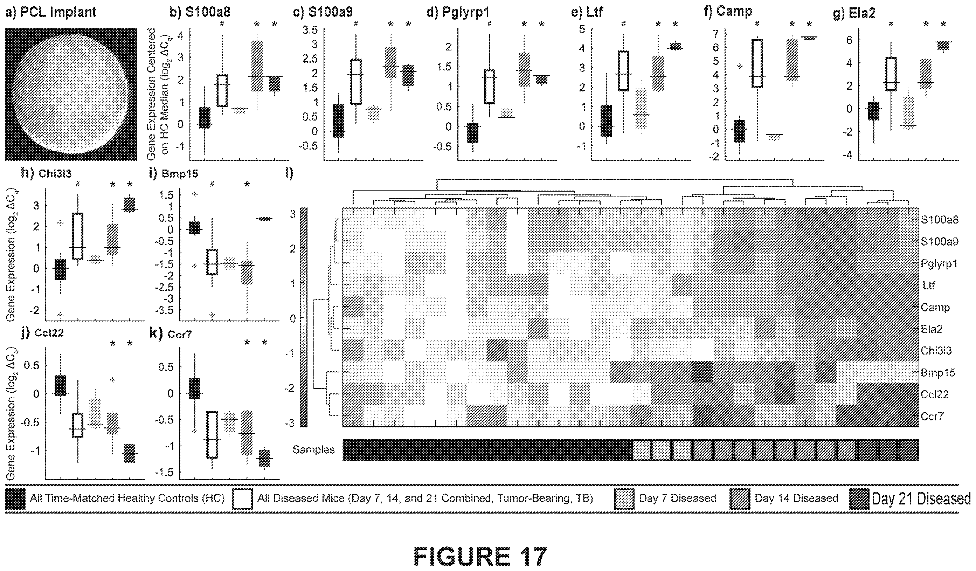

Figure 17 demonstrates that progressive gene expression changes within implant-derived tissue during metastatic disease course. BALB/c mice were implanted with microporous polymer implants (Day -14) then inoculated with syngeneic 4T1 tumor cells at Day 0. Implants were biopsied at Days 7, 14, and 21 and tissue was analyzed with a high-throughput RT-qPCR platform (OpenArray^{™}, 632 target and 16 reference genes). a) Microporous polymer (PCL) implant with a diameter of 5 mm, thickness of 2 mm, and interconnected 250-425 µm pores. b-k) Box plots for genes of interest show the median, 25th-75th percentiles and most extreme data points that were not considered outliers (outliers are visually indicated by red +). Diseased cohort expression is centered by the time-matched healthy cohort median. Scale depicts the log2-transformed fold change. A two-way MANOVA showed a significant interaction effect between condition and time (Pillai's Trace=1.276, F(20,28)=2.465, p=0.014). Post hoc univariate ANOVA showed significant differences within the diseased cohort over time (indicated by #, df=(2,22), p<0.05). Simple effects analysis showed significant differences between diseased and time-matched healthy controls (indicated by *, Šidák adjusted p<0.05 adjusted, see Data S5 for exact F and p values). l) Heatmap and unsupervised hierarchal clustering of 10 genes of interest with expression levels for each gene depicted as standardized data (n=14 per condition). Cohort size at Days 7 (n=3),14 (n=8), and 21 (n=3) represent biological replicates.

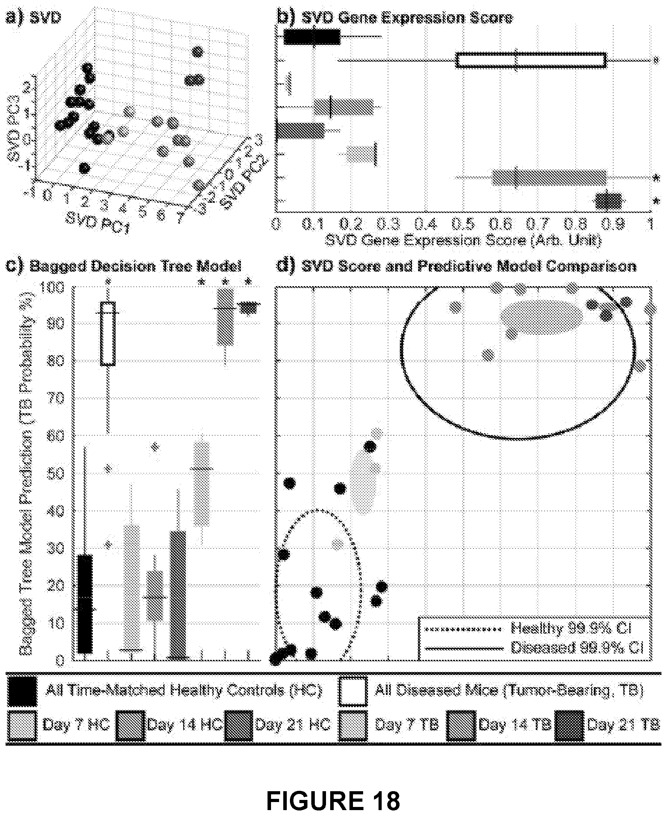

Figure 18 demonstrates that signatures reduce gene expression to scoring metrics and diagnostic predictions, a) Gene expression from analysis of implant-derived tissue was reduced to an unsupervised scoring metric through singular value decomposition (SVD), which was converted to a score b) by calculating Euclidean distance from the healthy centroid to each sample. Scores were scaled between 0 and 1. c) In parallel, gene expression data was used to train a bootstrap aggregated (bagged) decision tree ensemble with leave-one-out cross validation to predict the likelihood that a mouse was tumor-bearing, d) Plot of the SVD score versus the bagged tree prediction. Dashed and solid line ellipses indicate the 99.9% confidence intervals for healthy (n=14) and diseased (n=14) cohorts, respectively. Filled ellipses indicate average and SEM of Days 7 (n=3), 14 (n=8), and 21 (n=3) diseased cohorts, illustrating the signature tracks with disease course. A two-way MANOVA showed a significant interaction effect between condition and time (Pillai's Trace=0.568, F(4,44)=4.361, p=0.005). Post hoc univariate ANOVA showed significant differences within the diseased cohort over time (indicated by #, df=(2,22), p<0.001). Simple effects analysis showed significant differences between diseased and time-matched healthy controls (indicated by *, Šidák adjusted p<0.05 adjusted, see Data S5 for exact F and p values).

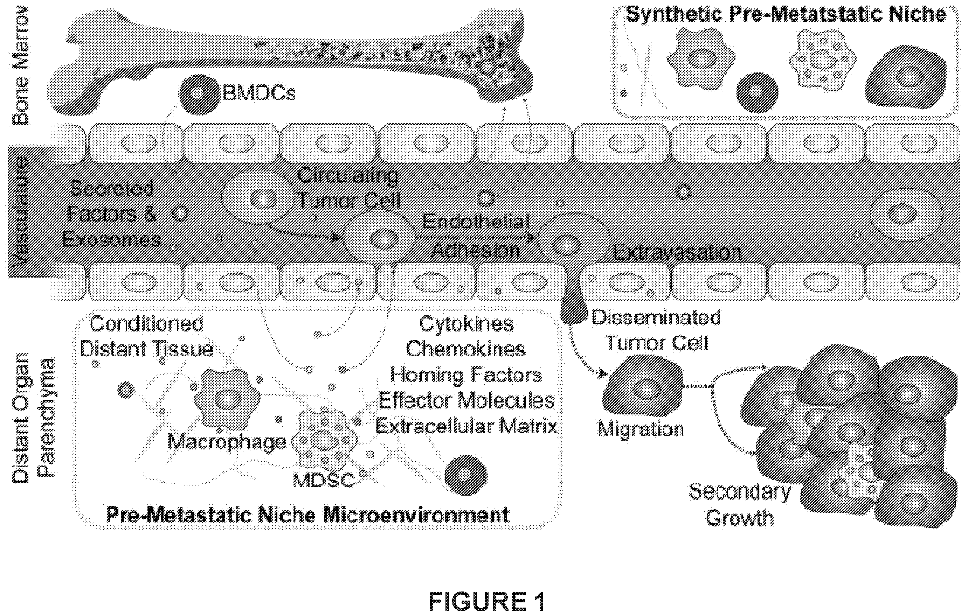

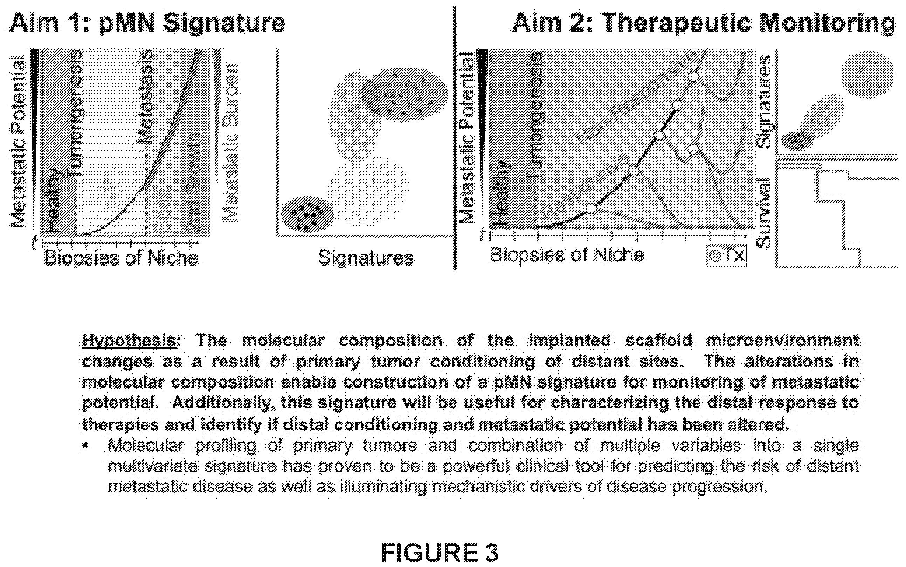

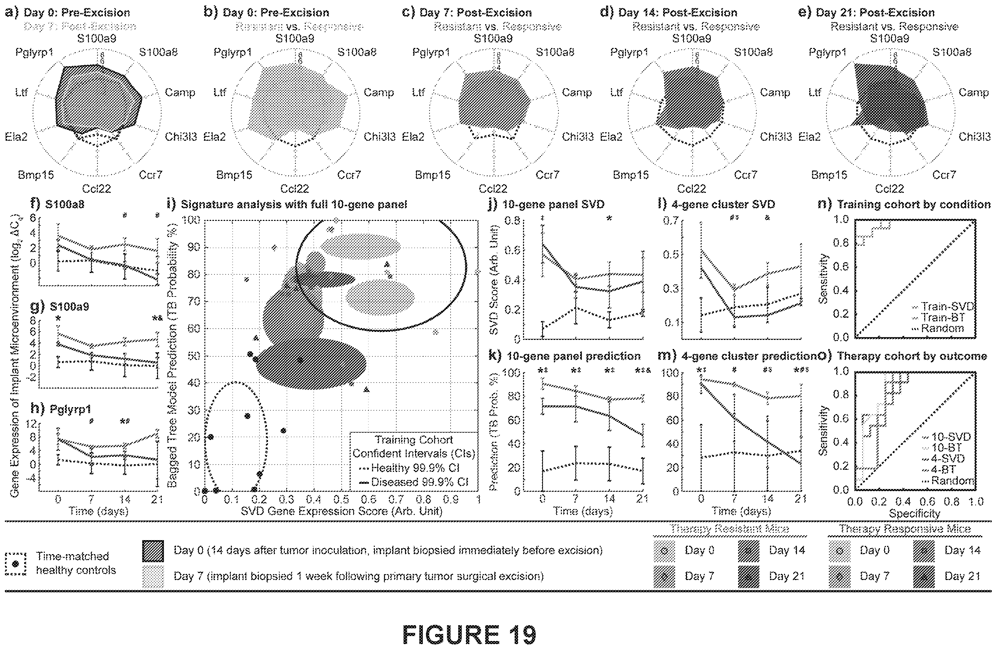

Figure 19 demonstrates that primary tumor mastectomy redirects gene expression and signature trajectory, which predicts therapeutic efficacy. Mice with an array of implants (n=8 sites per mouse, n=10 mice) and a primary tumor in their 4th mammary fat pad (n=7 mice) had implant #1 surgically biopsied then immediately had their primary tumor resected along with the surrounding fat pad (Day 0, tumor-burden equivalent to Day 14 in Fig. 1&3). Healthy mice (n=3) received sham inoculation and a mammary gland excision. All mice where then monitored by weekly (Days 7, 14, 21) SynDx biopsy, RT-qPCR, and signature analysis. a) For the 7 genes that showed an increased expression in the metastatic onset model, there was an organized regression in expression 1-week after therapy. b-e) Longitudinal survival monitoring stratified diseased mice into therapeutic resistant (n=4) and therapeutic responsive (n=3) groups. While a significant decrease in expression was evident in several genes (Fig. 18), the divergence between the resistant and responsive was most evident in f-h) S100a8, S100a9, and Pglyrp1. i-k) Associated alterations in SVD score and a significant divergence in bagged tree model prediction was observed. Creation of a modified 4-gene l) SVD and m) bagged tree signature from S100a8, S100a9, Pglyrp1, and Ltf. ROC curves of predicted performance for SVD and bagged tree models in the n) training cohort (n=28) for tumor-bearing and o) therapy cohort for resistance from Days 7 to 21. All area under the curve (AUC) calculations for the therapy cohort exceeded 0.8 with the 10-gene bagged tree (10-BT) having the highest AUC at .878 (95% Cl, 0.743 - 1.000). Filled ellipses indicate the average and SEM of Day 0, 7, 14, and 21. Error bars in line plots for gene expression and signature trajectory indicate SEM. The cohort size for each group is decreased by one at Day 21 due recurrence and animal censorship. For longitudinal data, statistics were performed via a linear mixed model. Post hoc simple effects analysis indicates significant differences (p<0.05) between (*) healthy and resistant, (‡) healthy and responsive, (&) resistant and responsive, and (#) responsive and responsive Day 0 (pre-excision) following significant (p<0.05) or trending (p<0.1) interactions in a two-way ANOVA (see Data S5 for exact F and p values for all genes).

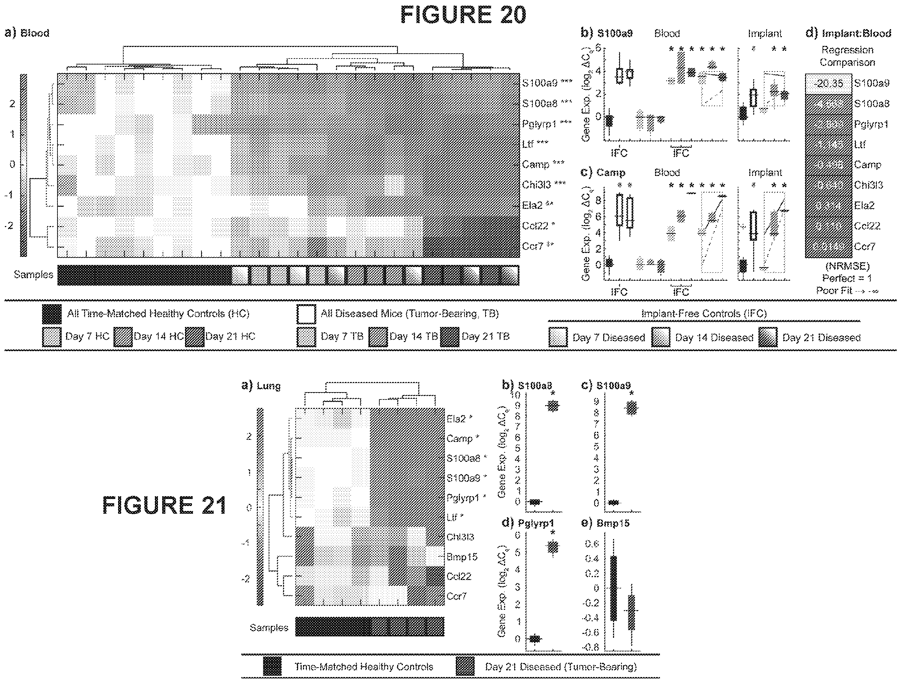

Figure 20 demonstrates gene expression changes found in blood following tumor inoculation. a) Heatmap of blood leukocyte RT-qPCR data from BALB/c mice inoculated with 4T1 tumor cells shows unsupervised clustering based on samples and genes. Implant-free control (IFC) samples were also analyzed in the cohort as indicated in the data. A two-way MANOVA showed a significant interaction effect between condition and time (Pillai's Trace=2.527, F(36,40)=1.907, p=0.024). Post hoc univariate and simple effects analysis showed significant differences within the diseased cohort over time and when comparing diseased and IFCs to time-matched controls for (***) all time points, (*) Day 21, (‡*) Day 21 and diseased versus control at Day 7, (&*) Day 21 and diseased versus control at Day 14 (Šidák adjusted p<0.05 adjusted). Bmp15 was omitted due to a lack of detection. To compare gene expression dynamics between blood and synthetic niches, regression analyses (insets) were performed between Days 7, 14, and 21. To illustrate, b) blood S100a9 compared to implant-derived tissue S100a9 expression has a worse fit when compared to c) blood Camp and implanted-derived tissue expression as determined by d) Normalized Root Mean Squared Error (NRMSE). The S100a8, S100a9, and Pglyrp1 gene cluster had the worst fits when comparing blood to implant-derived tissue. Significant differences within blood expression indicated as # (df=(2,15), p<0.001) and * indicates significant differences between diseased and time matched controls (Šidákadjusted p<0.05, see Data S5 for exact F and p values).
Figure 21 demonstrates gene expression changes in lung at Day 21 following tumor inoculation. BALB/c mice inoculated with 4T1 tumor cells at Day 0, had lung tissue biopsies taken and RT-qPCR analyzed at Day 21 from tumor inoculated and healthy mice. a) Heatmap of gene expressions for 10-gene panel (reference gene normalized, centered on healthy median, and standardized). Organization was based on unsupervised clustering of samples and genes. Genes of interest with significant changes included an increase in b) S100a8, c) S100a9, and d) Pglyrp1. e) Unlike the blood, Bmp15 was detectable but not significant. * indicates significance (Dunn-Šidák corrected to p<0.005) from independent two-tailed t-tests comparing diseased to healthy controls (n=4 per condition). Data in b-e panels is unstandardized, log2-transformed fold-changes.

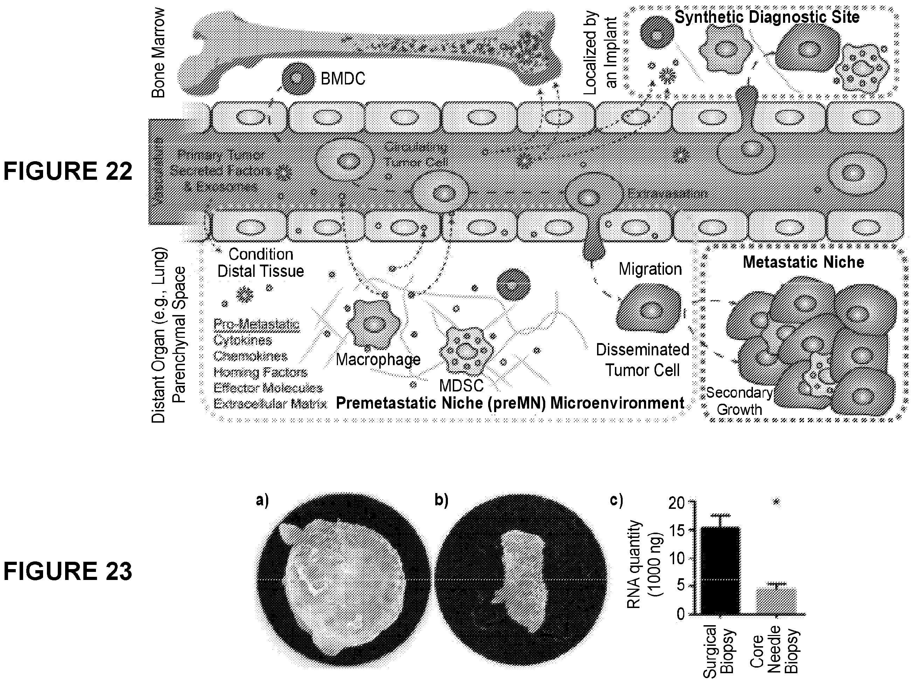

Figure 22 demonstrates the development of the pre-metastatic niche (preMN), metastatic niche, and synthetic diagnostic site. Circulating tumor cells (CTCs) exhibit a tropism for specific distal microenvironments, thus indicating that metastasis is pre-determined. These primed distal microenvironments are denoted as the pre-metastatic niche (preMN) and facilitate homing then colonization of disseminated tumor cells (DTCs). Development of the preMN is driven by systemic conditioning from the primary tumor and secretion of factors and exosomes, which is amplified by simultaneous conditioning of the bone-marrow and alterations in bone-marrow derived cells (BMDCs). Given that the preMN contains a unique mixture of soluble factors, extracellular matrix, stromal and immune cells (e.g., cancer-associated fibroblasts and myeloid-derived suppressor cells, MDSCs), it is reasonable that preMN function could be synthesized through the recapitulation these unique factors at a synthetic site *in vivo.*
Figure 23 demonstrates that implant microenvironment exhibits good tissue ingrowth and facilitates surgical and core-needle biopsies to acquire RNA for transcriptomic or gene expression analysis. a) A surgically-biopsied implant illustrating the intact, frozen condition prior to RNA isolation and RT-qPCR assessment, b) A sample derived from a scaffold using a core needle biopsy (CNB, Bard^{®} Mission^{®} Disposable Core Biopsy Instrument) which enables minimally invasive retrieval of samples, similar to clinical approaches for sampling suspicious tissue. In both samples tissue ingrowth into the microporous structure is evident but especially in the CNB as the cutting plane transected the inner core of the implant. Scaffolds as shown are in -80°C frozen state. Diameter of the black background circle is 7.5 mm. c) Comparing the RNA isolated from the full surgically biopsied scaffold compared to the CNB sample, there is a significant -1.812 log-transformed fold change, but sufficient material for numerous gene expression assessments (RT-qPCR, RNAseq, etc.) with the CNB samples containing an average of 4376 ng of total RNA. * indicates differences between surgically biopsied and CNB samples from an unpaired t-test (p<0.05, n=8).

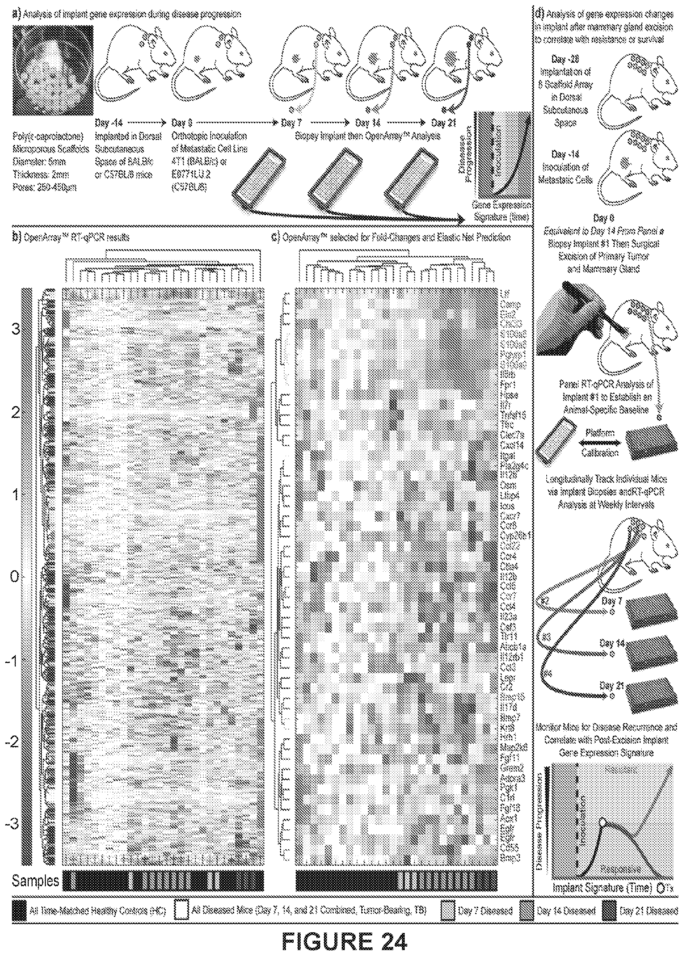

Figure 24 demonstrates the experimental designs and OpenArray^{™} output for select genes with high fold-change and predictive value. a) Experimental outline for model training study including the fabrication of microporous PCL scaffolds, their implantation into mice. Mice were then inoculated with tumor cells and the resulting changes in implant gene expression were studied via OpenArray^{™} RT-qPCR. b) Results from OpenArray for tumor-bearing mice at Days 7 (n=3), 14 (n=8), and 21 (n=3) along with time-matched healthy control mice (n=14). c) Results from OpenArray selected for fold change and predictive value of TF or TB by a derivative of the LASSO algorithm (Elastic Net). For panels b-c mice are indicated on x-axis and genes are indicated on y-axis with clustering done via Euclidian distance and average linkage. Genes indicated in red text on the y-axis were selected for the smaller 10-gene panel analysis is subsequent studies. d) Experimental outline for model testing in a post-excision model including the implantation of scaffolds, tumor inoculation, isolation of pre-treatment implant, tumor and mammary gland excision, then weekly isolation of a synthetic niche for analysis.

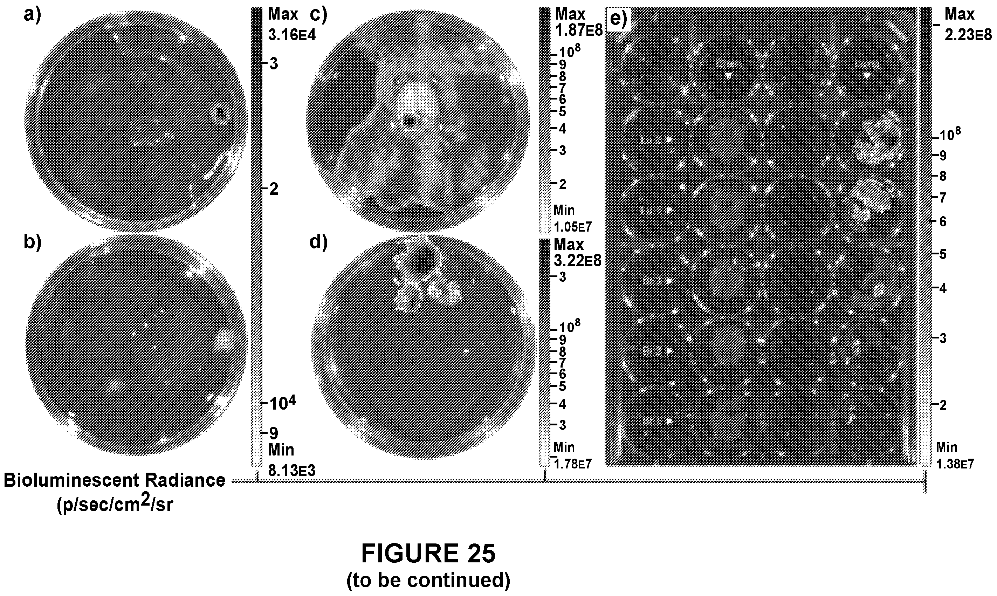

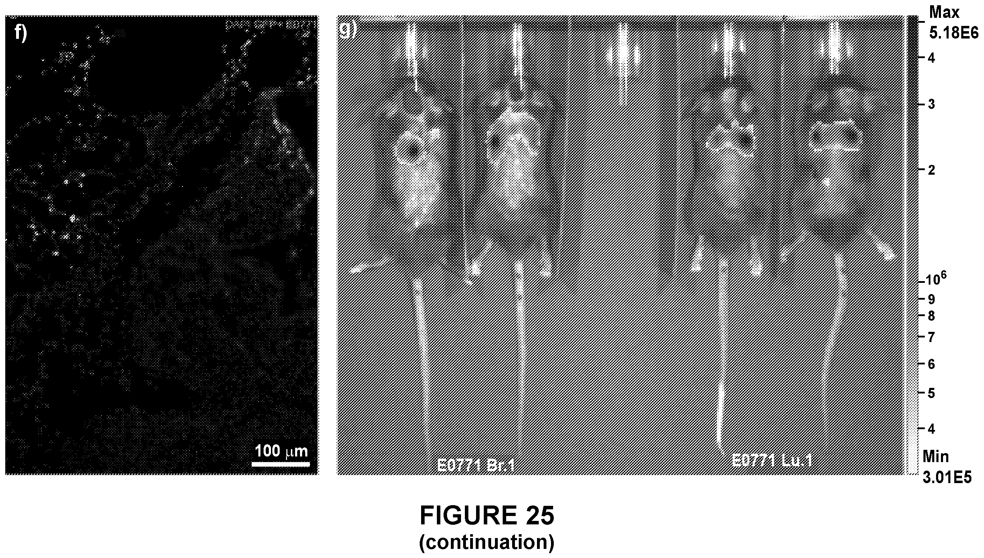

Figure 25 demonstrates the development of E0771 metastatic variants. Scaffolds from a) left and b) right side of of a mouse inoculated with parental E0771 which developed a high metastatic burden which metastases present in the c) brain and d) lungs. Metastatic lines Br.1 and Lu.1 were derived from the tumor cells from these brain and lung metastases, respectively. Serial inoculation via intracardiac injection and isolation of developed metastases e) indicated a propensity for the lung derived metastases have an organotropic bias toward lung tissue. f) Confirmation of fluorescent tumor cells in the scaffolds from a-b was verified by fluorescent microscopy of scaffold tissue sections. g) In vivo imaging of Br.1 and Lu.1 line 11 days following intracardiac inoculation.

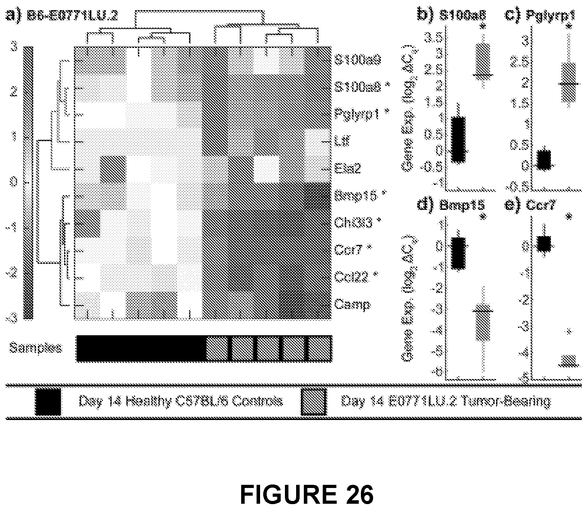

Figure 26 demonstrates synthetic niche gene expression in C57BL/6 mice inoculated with metastatic tumor cells. C57BL/6 mice were implanted with microporous PCL scaffolds (Day -14) and then inoculated with a metastatic derivative (developed through serial inoculations of explanted lung metastases) of the E0771 syngeneic line (Day 0). At Day 14, scaffolds were biopsied from inoculated mice and healthy controls. a) Heatmap of gene expressions for a 10-gene panel normalized to reference genes, centered on the healthy control median, and standardized. Organization is based on unsupervised clustering of samples and genes. Significant alterations include the increases in b) S100a8 and c) Pglyrp1 alongside significant decreases in d) Bmp15 and e) Ccr7. * indicates significance (Dunn-Šidák corrected to p<0.005) from independent two-tailed t-tests comparing diseased to healthy (n=5 per condition). Data in b-e panels illustrate unstandardized, log2-transformed fold-changes.

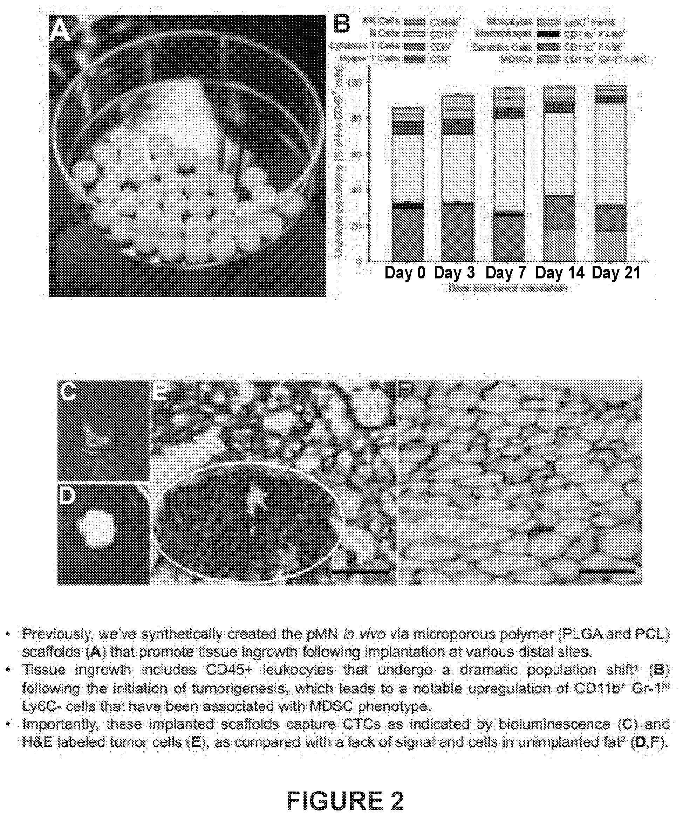

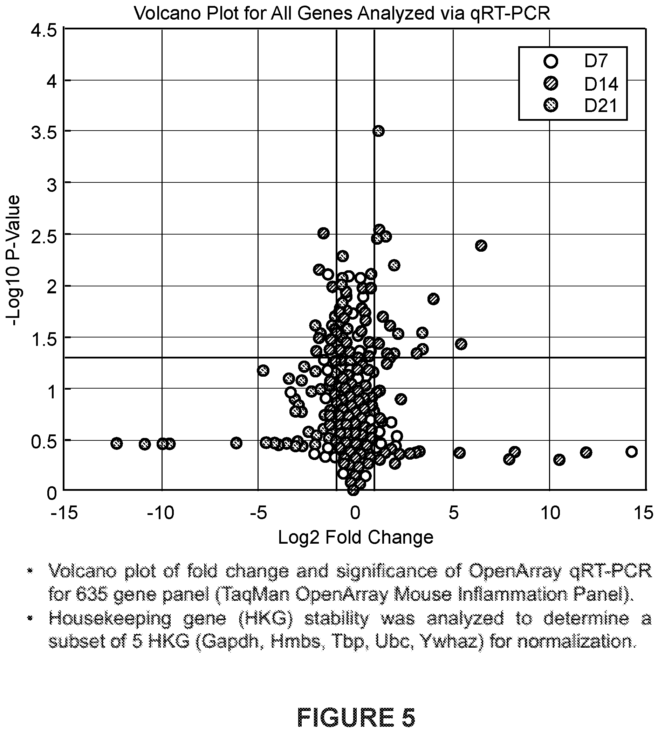

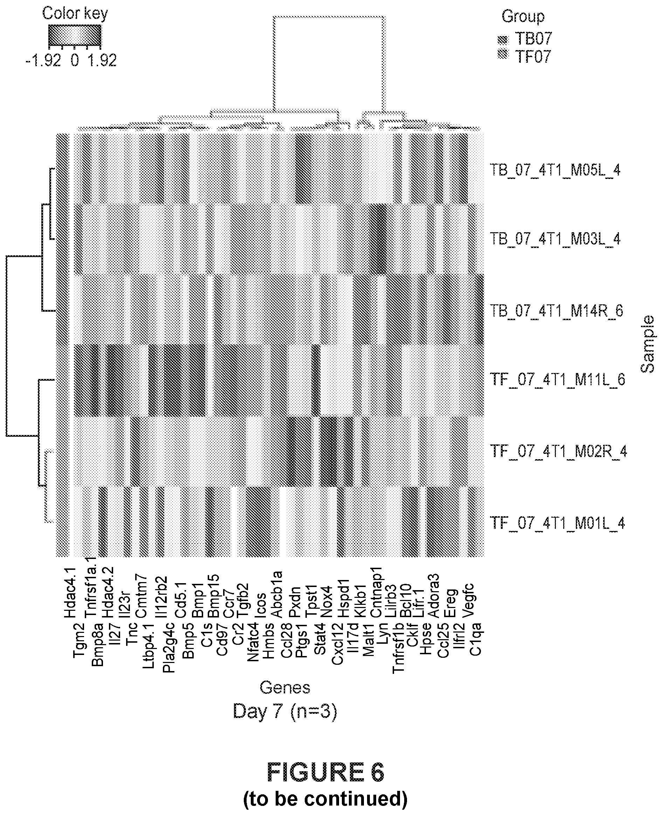

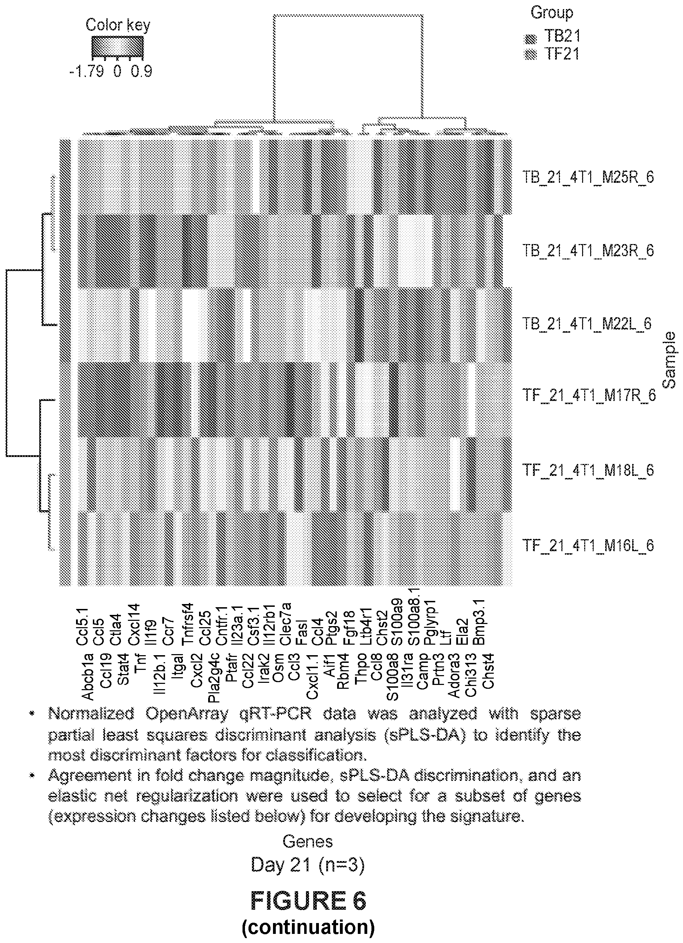

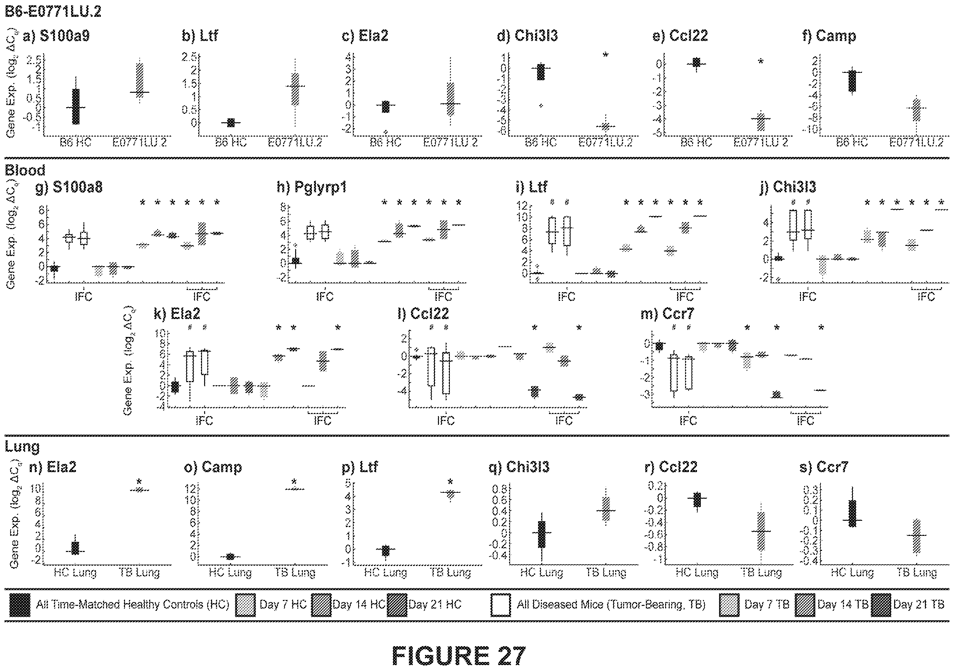

Figure 27 demonstrates additional box plots for C57BL/6, blood and lung gene expression, a-f) box plots of gene expression to complete the data in the B6-E0771Lu.2 heatmap from Fig. 2. g-m) box plots of gene expression to complete the data in the Blood heatmap from Fig. 5. n-s) box plots of gene expression to complete the data in the Lung heatmap from Fig. 6. For the C57BL/6 model in a-f) * indicates significance (Dunn-Šidák corrected to p<0.005) from independent two-tailed t-tests comparing diseased to healthy (n=5 per condition). For blood data n-s) a two-way MANOVA showed a significant interaction effect between condition and time (Pillai's Trace=2.527, F(36,40)=1.907, p=0.024). Post hoc univariate and simple effects analysis showed significant differences within the diseased cohort over time as indicated as ^{#} (df=(2,15), p<0.001) and * indicates significant differences compared to time matched controls (Šidák adjusted p<0.05 adjusted. For the lung data in n-s) * indicates significance (Dunn-Šidák corrected to p<0.005) from independent two-tailed t-tests comparing diseased to healthy (n=4 per condition).
Figure 28 demonstrates gene expression analysis and singular value decomposition, bagged decision tree, and sparse partial least squares discriminant analysis computational pipelines. Map of computational processes and the data outputs that are represented in the manuscript. For example, the calculation of the Euclidean distance from the first 3 principle components of the singular value decomposition (SVD) represented in Fig. 3b can be traced back through its processing steps to the total RNA isolation, quality control, and cDNA synthesis.

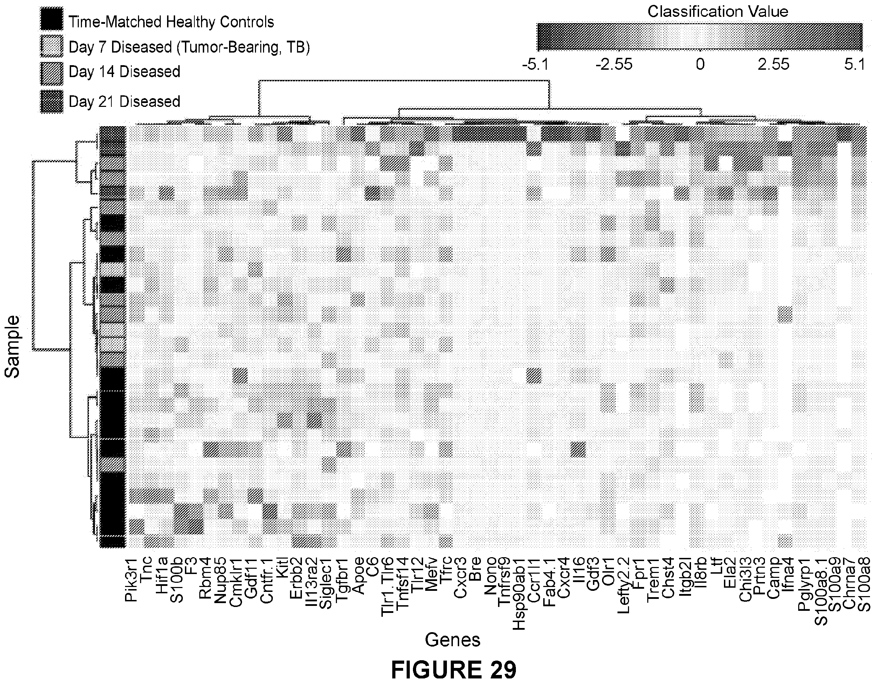

Figure 29 demonstrates Sparse Partial Least Squares Discriminant Analysis (sPLS-DA) of the entire OpenArray^{™} RT-qPCR data limited to selection of the 25 most valuable factors that increased and decreased. Normalized OpenArray qRT-PCR data was analyzed with sparse partial least squares discriminant analysis (sPLS-DA) to identify the most discriminant factors for classification. Agreement in fold change magnitude, sPLS-DA discrimination, and an elastic net regularization were used to select for a subset of genes (expression changes listed below) for developing the signature and validation experiments that used SVD and bagged tree decision tree ensembles. Genes indicated in red text on the x-axis were included in the computational signatures.

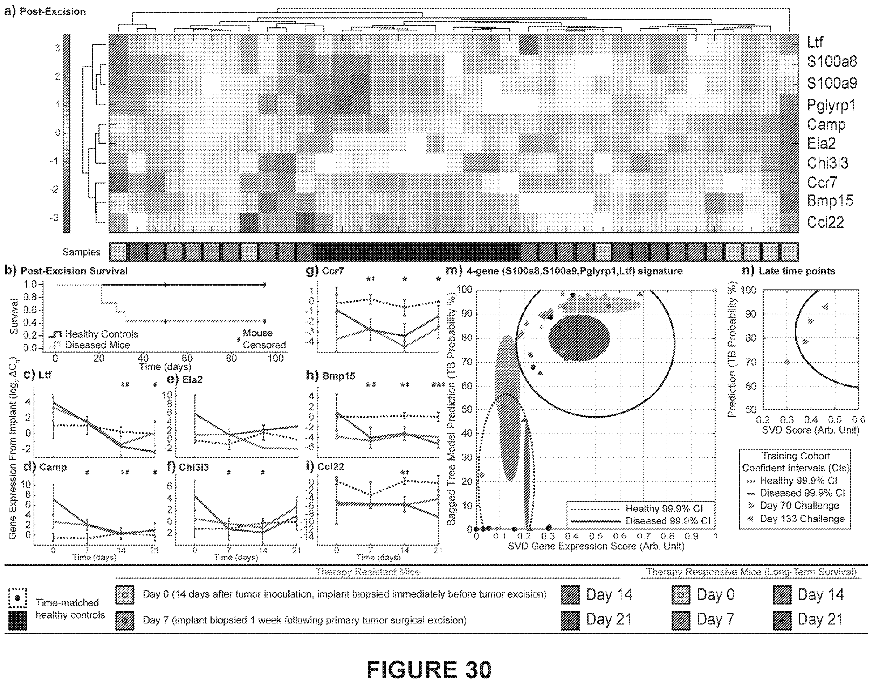

Figure 30 demonstrates additional data from post-excision model including: heatmap to indicate full distribution of data, survival curve, gene expression and signature trajectories, and modified signature including only S100a8, S100a9, Pglyrp1, and Ltf. a) Heatmap gene expression (C_{q}) for a 10-gene panel normalized to reference genes. Genes indicated on the y-axis present in similar fashion to the gene expression from the OpenArray training data (e.g., the highest levels of S100a9 are present in pre-resection samples and cluster with S100a8 and Pg!yrp1). Biopsied samples from pre-resection (Day 14) and post-resection (Days 21, 28, and 35) are indicated along the x-axis with unsupervised clustering showing a general aggregation of healthy samples with samples from specific mice moving towards healthy as a function of time. This time course, longitudinal analysis of gene expression trajectories as a function of survival b) is better conveyed as a line plot for c) Ltf, d) Camp, e) Ela2, f) Chi3l3, g) Ccr7, h) Bmp15, i) Ccl22. Error bars indicate SEM. m) Creation of a modified signature (SVD and bagged tree) from only S100a8, S100a9, Pglyrp1, and Ltf, which consistently cluster together across multiple models and in different tissues shows a stronger separation between mice that survived long-term and those that developed recurrence. n) To challenge the long-term dynamics in the synthetic niche, the healthy control mice (n=3) were inoculated at late time points (Day 56 and Day 119 after implantation) in their left 4^{th} mammary fat pad (contralateral to right excision at day 0). Synthetic niche gene expression and signature alignment was analyzed at Days 70 and 133 (133 data points are from different niches in the same mouse). Dashed and solid lines indicate the 99.9% confidence intervals for healthy and diseased training cohorts, respectively. Error bars in line plots for gene expression and signature trajectory indicate SEM. The cohort size for each group is decreased by one at Day 21 due recurrence and animal censorship. For longitudinal data, statistics were performed via a linear mixed model. Post hoc simple effects analysis indicates significant differences (p<0.05) between (*) healthy and resistant, (^{‡}) healthy and responsive, (^{&}) resistant and responsive, (^{#}) responsive and responsive Day 0 (pre-excision), and (^{$}) resistant and resistant Day 0 (pre-excision) following significant (p<0.05) or trending (p<0.1) interactions in a two-way ANOVA.

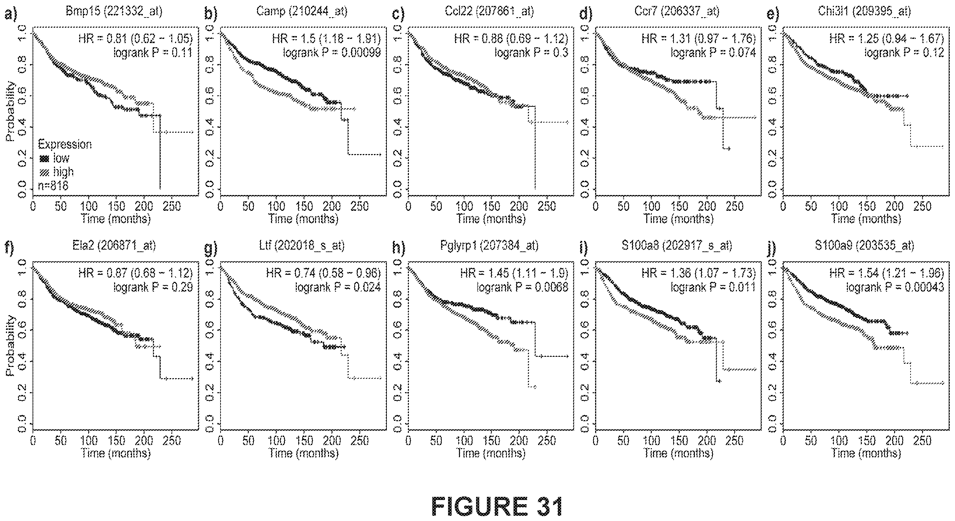

Figure 31 demonstrates Kaplan-Meier survival curves correlated with high and low gene expression from breast cancer patient samples. Microarray (GEO via KMPlot) and prognosis data a-j) were queried for genes selected for the 10-gene panel from the synthetic niche to determine the expression level relevance in primary tumors on the clinical outcome of systemically untreated breast cancer patients (n=818). Kaplan-Meier plots indicate as separation of two profiles separating high and low gene expression that are automatically divided by the median expression of the genes for all samples. Plots indicate the Hazard Ratio (HR) which is highest for S100a9 and logrank significance comparing high and low expression as a function of recurrence-free survival. (*) FDR 10% corrected significance (p<0.011) and (**) FDR 5% corrected significance (p<0.00099).

### DETAILED DESCRIPTION

### Metastatic Disease and Cancer

The present disclosure provides methods relating to a subject's status with regard to metastatic disease. As used herein, the term "metastatic disease" means "metastatic cancer". In the context of most cancer types, "metastatic cancer" is synonymous with "stage IV cancer". For purposes herein, the cancer may be any cancer known in the art, such as, for example, any of: acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vulva, chronic lymphocytic leukemia, chronic myeloid cancer, colon cancer, esophageal cancer, cervical cancer, gastrointestinal carcinoid tumor, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, ovarian cancer, pancreatic cancer, peritoneum, omentum, and mesentery cancer, pharynx cancer, prostate cancer, rectal cancer, renal cancer (e.g., renal cell carcinoma (RCC)), small intestine cancer, soft tissue cancer, stomach cancer, testicular cancer, thyroid cancer, uterine cancer, ureter cancer, and urinary bladder cancer. In particular aspects, the cancer is selected from the group consisting of: head and neck, ovarian, cervical, bladder and oesophageal cancers, pancreatic, gastrointestinal cancer, gastric, breast, endometrial and colorectal cancers, hepatocellular carcinoma, glioblastoma, bladder, lung cancer, e.g., non-small cell lung cancer (NSCLC), bronchioloalveolar carcinoma. In exemplary aspects, the metastatic cancer is triple-negative breast cancer, pancreatic cancer, prostate cancer, or melanoma.

During the process of metastasis, cancer cells localized at a vascularized primary tumor detach from the primary tumor and intravasate (or enter into the vasculature), thereby becoming a circulating tumor cell (CTC). A CTC can undergo homing and migration to a secondary site, which may be distant from the primary tumor. The CTC can subsequently adhere to the blood vessel wall near the secondary site and extravasate (or exit the vasculature) into the tissue at the secondary site. At the secondary site, the tumor cell may begin proliferating and begin the growth or colonization of a secondary tumor at this distant site. See, e.g., Wirtz et al., Nature Reviews Cancer 2011.

At each of these stages, tumor cells uniquely interact with their environment. CTCs exhibit a tropism for specific distal microenvironments, thus indicating that metastasis is pre-determined. These primed distal microenvironments are termed pre-metastatic niches (pMN) and facilitate homing then colonization of CTCs. Development of the pMN is driven by systemic conditioning from the primary tumor and its secretion of factors and exosomes, which is amplified by simultaneous conditioning of the bone marrow. See, Figure 1. Without being bound to any particular theory, the stage of metastasis is reflected by the expression profile of the cells of the pMN. The expression profile of the cells of the pMN serves as a molecular signature of the pMN, and data provided herein demonstrate that the molecular signature changes over the course of time after tumorigenesis and throughout the stages of metastasis. As supported by the data presented herein, the expression profile of the cells of the pMN are represented by the expression of the cells of engineered pMNs (also referred to herein and in the art as "biomaterial scaffolds" or "scaffolds"). Thus, the expression profiles of scaffolds are representative of the stage of metastatic disease and/or the potential, risk or likelihood therefor, and therefore, such profiles allow for early diagnosis, detection, and management (e.g., treatment) of metastatic cancer.

### Metastatic Potential and Disease Detection

Provided herein are methods of determining a subject's status with regard to metastatic disease. In exemplary embodiments, the subject has a tumor or cancer. The present disclosure provides methods of determining a subject's metastatic potential. As used herein, the term "metastatic potential" means the potential, risk, chance, or likelihood that a cancer will become metastatic. "Metastatic potential" is a measure of the likelihood for onset of metastatic disease or metastatic cancer. In exemplary aspects, the method comprises measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level. In exemplary instances, the methods of determining a subject's metastatic potential achieves or is similar to, if not the same as, determining a tumor's or cancer's metastatic stage in a subject. In exemplary embodiments, such methods of determining a tumor's or cancer's metastatic stage comprises measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level.

Also provided herein are methods of detecting metastatic disease, or a predisposition thereto, in a subject in need thereof. In exemplary embodiments, the method comprises measuring a level of expression of a gene, an RNA or a protein, or a combination thereof, in a sample obtained from a synthetically-engineered pMN implanted in the subject, wherein the measured expression level of the gene, RNA or protein in the sample is compared to a control level.

In exemplary instances, the methods comprise measuring the expression level of at least 2, 3, 4, 5 or more genes, at least 2, 3, 4, 5 or more RNA, and/or at least 2, 3, 4, 5 or more proteins in the sample. In exemplary instances, the methods comprise measuring the expression level of at least 10, 15, 20 or more genes, at least 10, 15, 20 or more RNA, and/or at least 10, 15, 20 or more proteins in the sample. In exemplary instances, the methods comprise measuring the expression level of at least 50, 100, 200 or more genes, at least 50, 100, 200 or more RNA, and/or at least 50, 100, 200 or more proteins in the sample. In exemplary instances, the methods comprise measuring the expression level of a plurality of different genes, a plurality of RNA, and/or a plurality of proteins. In exemplary aspects, the expression levels of the genes, RNA, and/or proteins are processed through an algorithm to obtain a single metric or single score of gene expression, RNA expression, or protein expression. In exemplary aspects, the expression levels are normalized to housekeeping gene expression levels. In exemplary aspects, the expression levels are processed through singular value decomposition, dynamic mode decomposition, principle component analysis, fisher linear discriminant, or linear combination. In exemplary aspects, the expression levels of the genes, RNA, and/or proteins (optionally normalized to housekeeping gene expression levels) or the single metric or single score is processed through a machine learning algorithm to obtain a score of prediction of disease state, e.g., a % chance of attaining a diseased state (metastatic potential). In exemplary aspects, the metric of gene expression, RNA expression, or protein expression is combined with the prediction score to obtain a graphical or numerical output, which may be used as a control (or a panel of controls) against which the measured levels are compared.

### Monitoring Metastatic Potential, Metastatic Disease, and Treatment Thereof

The present disclosure provides methods of monitoring a subject's metastatic potential or metastatic disease. In exemplary embodiments, the methods comprise measuring a level of expression of a gene, an RNA, or a protein, or a combination thereof, in a sample obtained from a synthetically-engineered pMN implanted in the subject at a first time point and measuring the expression level of the gene, RNA, or protein in a sample obtained from the synthetically-engineered pMN at a second time point, wherein the expression level measured at the first time point is compared to the expression level measured at the second time point. In exemplary aspects, the first time point occurs before a treatment and the second time point occurs after a treatment and the method is a method of determining the efficacy of a treatment for metastatic disease.

In exemplary instances, the methods comprise measuring the expression level of at least 2, 3, 4, 5 or more genes, at least 2, 3, 4, 5 or more RNA, and/or at least 2, 3, 4, 5 or more proteins in the sample. In exemplary instances, the methods comprise measuring the expression level of at least 10, 15, 20 or more genes, at least 10, 15, 20 or more RNA, and/or at least 10, 15, 20 or more proteins in the sample. In exemplary instances, the methods comprise measuring the expression level of at least 50, 100, 200 or more genes, at least 50, 100, 200 or more RNA, and/or at least 50, 100, 200 or more proteins in the sample. In exemplary instances, the methods comprise measuring the expression level of a plurality of different genes, a plurality of RNA, and/or a plurality of proteins. In exemplary instances, the methods comprise measuring the expression level of a plurality of different genes, a plurality of RNA, and/or a plurality of proteins. In exemplary aspects, the expression levels of the genes, RNA, and/or proteins are processed through an algorithm to obtain a single metric or single score of gene expression, RNA expression, or protein expression. In exemplary aspects, the expression levels are normalized to housekeeping gene expression levels. In exemplary aspects, the expression levels are processed through singular value decomposition, dynamic mode decomposition, principle component analysis, fisher linear discriminant, or linear combination. In exemplary aspects, the expression levels of the genes, RNA, and/or proteins (optionally normalized to housekeeping gene expression levels) or the single metric or single score is processed through a machine learning algorithm to obtain a score of prediction of disease state, e.g., a % chance of attaining a diseased state (metastatic potential). In exemplary aspects, the metric of gene expression, RNA expression, or protein expression is combined with the prediction score to obtain a graphical or numerical output, which may be used as a control (or a panel of controls) against which the measured levels are compared. Once metastatic potential is determined in this manner, a determination of whether or not the subject has metastatic disease may be made.

### Methods of Determining a Subject's Treatment or Need Therefor

As the methods of the present disclosures determine metastatic potential and/or detect metastatic disease in a subject, methods of determining treatment for a subject or determining a subject's need for a metastatic disease therapy are provided.

Methods of determining treatment for a subject with a tumor or cancer are further provided by the present disclosure. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA or protein in the sample is compared to a control level.

The present disclosure also provides a method for determining a subject's need for metastatic disease therapy. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA or protein in the sample is compared to a control level.

In exemplary instances, the methods comprise measuring the expression level of at least 2, 3, 4, 5 or more genes, at least 2, 3, 4, 5 or more RNA, and/or at least 2, 3, 4, 5 or more proteins in the sample. In exemplary instances, the methods comprise measuring the expression level of at least 10, 15, 20 or more genes, at least 10, 15, 20 or more RNA, and/or at least 10, 15, 20 or more proteins in the sample. In exemplary instances, the methods comprise measuring the expression level of at least 50, 100, 200 or more genes, at least 50, 100, 200 or more RNA, and/or at least 50, 100, 200 or more proteins in the sample. In exemplary instances, the methods comprise measuring the expression level of a plurality of different genes, a plurality of RNA, and/or a plurality of proteins. In exemplary instances, the methods comprise measuring the expression level of a plurality of different genes, a plurality of RNA, and/or a plurality of proteins. In exemplary aspects, the expression levels of the genes, RNA, and/or proteins are processed through an algorithm to obtain a single metric or single score of gene expression, RNA expression, or protein expression. In exemplary aspects, the expression levels are normalized to housekeeping gene expression levels. In exemplary aspects, the expression levels are processed through singular value decomposition, dynamic mode decomposition, principle component analysis, fisher linear discriminant, or linear combination. In exemplary aspects, the expression levels of the genes, RNA, and/or proteins (optionally normalized to housekeeping gene expression levels) or the single metric or single score is processed through a machine learning algorithm to obtain a score of prediction of disease state, e.g., a % chance of attaining a diseased state (metastatic potential). In exemplary aspects, the metric of gene expression, RNA expression, or protein expression is combined with the prediction score to obtain a graphical or numerical output, which may be used as a control (or a panel of controls) against which the measured levels are compared. Once metastatic potential is determined, a physician may have sufficient information to decide the best course of treatment for the subject and whether or not the course includes treatment for metastatic disease therapy.

In exemplary aspects, the score is indicative of aberrant immunological events. In exemplary instances, the score is indicative of an immunosuppressive and tumor cell hospitable environment. In some aspects, such an outcome is reflected by increased expression of S100a8/9. In certain aspects, the score is indicative of the need for inhibition of MDSC suppressive function. In exemplary aspects, it is determined that anti-metastatic disease therapy is warranted. In certain instances, it is determined that a systemic anti-metastatic disease therapy (e.g., PARP inhibitor) is needed.

### Treatment Methods

The references to methods of treatment in paragraphs 67-70 and 72 of this description are to be interpreted as references to the compounds, pharmaceutical compositions and medicaments for use in a method for treatment of the human (or animal) body by therapy (or for diagnosis).

As the early detection or diagnosis of metastatic disease enables proper management of the disease, the present disclosure provides a method of treating metastatic disease in a subject or a method of treating a subject with a tumor or cancer. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level, and administering an anti-metastatic disease treatment to the subject or performing anti-metastatic disease therapy on the subject, based on the measured level of expression.

As used herein, the term "treat," as well as words related thereto, do not necessarily imply 100% or complete treatment. Rather, there are varying degrees of treatment of which one of ordinary skill in the art recognizes as having a potential benefit or therapeutic effect. In this respect, the methods of treating cancer of the present disclosure can provide any amount or any level of treatment. Furthermore, the treatment provided by the method of the present disclosure can include treatment of one or more conditions or symptoms or signs of the cancer being treated. Also, the treatment provided by the methods of the present disclosure can encompass slowing the progression of the cancer. For example, the methods can treat cancer by virtue of enhancing the T cell activity or an immune response against the cancer, reducing tumor or cancer growth, reducing metastasis of tumor cells, increasing cell death of tumor or cancer cells, and the like. In exemplary aspects, the methods treat by way of delaying the onset or recurrence of the cancer by at least 1 day, 2 days, 4 days, 6 days, 8 days, 10 days, 15 days, 30 days, two months, 3 months, 4 months, 6 months, 1 year, 2 years, 3 years, 4 years, or more. In exemplary aspects, the methods treat by way increasing the survival of the subject.

The present disclosure provides methods of prophylactically treating (i.e., preventing) or delaying the onset of metastatic disease. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level, and administering an anti-metastatic disease treatment to the subject or performing anti-metastatic disease therapy on the subject, based on the measured level of expression.

Because cancer is lethal due to its nature of becoming metastatic cancer, delaying the onset of metastatic disease may effectively increase the survival of the subject. Accordingly, the present disclosure further provides methods of increasing the survival of a subject with a tumor or cancer. In exemplary embodiments, the methods comprise measuring a level of expression of a gene, an RNA, or a protein, or a combination thereof, in a sample obtained from a synthetically-engineered pMN implanted in the subject, wherein the measured expression level of the gene, RNA, or protein in the sample is compared to a control level, and administering an anti-metastatic disease treatment to the subject or performing anti-metastatic disease therapy on the subject, based on the measured level of expression.

In exemplary instances, the methods comprise measuring the expression level of at least 2, 3, 4, 5 or more genes, at least 2, 3, 4, 5 or more RNA, and/or at least 2, 3, 4, 5 or more proteins in the sample. In exemplary instances, the methods comprise measuring the expression level of at least 10, 15, 20 or more genes, at least 10, 15, 20 or more RNA, and/or at least 10, 15, 20 or more proteins in the sample. In exemplary instances, the methods comprise measuring the expression level of at least 50, 100, 200 or more genes, at least 50, 100, 200 or more RNA, and/or at least 50, 100, 200 or more proteins in the sample. In exemplary instances, the methods comprise measuring the expression level of a plurality of different genes, a plurality of RNA, and/or a plurality of proteins. In exemplary instances, the methods comprise measuring the expression level of a plurality of different genes, a plurality of RNA, and/or a plurality of proteins. In exemplary aspects, the expression levels of the genes, RNA, and/or proteins are processed through an algorithm to obtain a single metric or single score of gene expression, RNA expression, or protein expression. In exemplary aspects, the expression levels are normalized to housekeeping gene expression levels. In exemplary aspects, the expression levels are processed through singular value decomposition, dynamic mode decomposition, principle component analysis, fisher linear discriminant, or linear combination. In exemplary aspects, the expression levels of the genes, RNA, and/or proteins (optionally normalized to housekeeping gene expression levels) or the single metric or single score is processed through a machine learning algorithm to obtain a score of prediction of disease state, e.g., a % chance of attaining a diseased state (metastatic potential). In exemplary aspects, the metric of gene expression, RNA expression, or protein expression is combined with the prediction score to obtain a graphical or numerical output, which may be used as a control (or a panel of controls) against which the measured levels are compared. Once metastatic potential is determined, an appropriate course of treatment for the subject may be provided to the subject and the treatment may be provided to the subject. An exemplary method is described herein at Example 7.

As used herein "anti-metastatic disease treatment" refers to the administration of a systemic agent targeting mechanistic pathways specific to metastasis. As used herein, "anti-metastatic disease therapy" refers to a treatment program that may include multiple dosing regimen or combinations of agents indicated as anti-metastatic disease treatments. Examples of anti-metastatic disease treatment include for instance, a poly ADP ribose polymerase (PARP) inhibitor (e.g., Olaparib) that targets metastatic tumor cells or Gemcitabine that may deplete myeloid-derived suppressor cells. Examples of anti-metastatic disease therapy include for example multiple doses of Gemcitabine or a combination therapy that includes both Gemcitabine and Olaparib. In exemplary instances, the anti-metastatic disease treatment comprises a PARP inhibitor or Gemcitabine. In various aspects, the anti-metastatic disease treatment comprises a compound that targets MDSCs through inhibiting MDSC suppressive function, inhibiting MDSC expansion, inhibiting MDSC recruitment, and/or inducing MDSC differentiation. Such treatments are described in the art, e.g., Albeituni et al., Cancer J 19(6): 490-501 (2013). In exemplary aspects, the anti-metastatic disease treatment comprises an inhibitor of reactive nitrogen species (RNS), a nitroaspirin, triterpenoid, very small size proteoliposome (VSSP), a phosphodiesterase-5 (PDE-5) inhibitor, e.g., sildenafil, an exosome formation inhibitor (e.g., an amiloride), a combination of gemcitabine and 5-fluorouracil, a cyclooxygenase-2 (COX-2) inhibitor, prostaglandin E2 (PGE2) inhibitor, sunitinib, amino bisphosphonate (e.g., zoledronate, pamidronate), a combination of doxorubicin and a cyclophosphamide, vemurafenib, a CXCR2 or CXCR4 antagonist, vitamin D3, an anti-G-CSF antibody, an anti-Bv8 antibody, an anti-CSF-1 antibody, an anti-CCL2 antibody, a taxane (e.g., docetaxel, paclitaxel), an all trans-retinoic acid (ATRA), a TLR9 activator, curcumin, whole-glucan particles (WGP).

### Samples

The samples of the methods of the present disclosure are samples obtained from a synthetically-engineered pMN. At some point in time relative to when the sample is obtained, the synthetically-engineered pMN was implanted in the subject. In exemplary aspects, the sample is obtained from a synthetically-engineered pMN that has been removed from the subject prior to the sample being taken. In exemplary aspects, the sample is obtained from a synthetically-engineered pMN while still implanted into a subject. In exemplary instances, the sample is a core-needle biopsy obtained from the synthetically-engineered pMN while still implanted into a subject. In exemplary aspects, the sample is a fine-needle aspiration obtained from the synthetically-engineered pMN while still implanted into a subject. Such methods of obtaining samples are known in the art. See, e.g., Eom et al., AJNR Am J Neuroradiol. 2015 Jun;36(6):1188-93.

In exemplary aspects, the sample comprises immune cells, stromal cells, or a combination thereof. In exemplary aspects, the sample comprises immune cells, including, but not limited to, macrophages, dendritic cells, MDSCs, neutrophils, monocytes, helper T cells, cytotoxic T cells, B cells, NK cells, CD45+ cells, and the like. In exemplary aspects, the sample comprises a combination of two or more of the above immune cell types. In exemplary aspects, the sample comprises stromal cells, including, for example, fibroblasts, endothelial cells, pericytes, and the like. In exemplary aspects, the sample comprises a combination of two or more of the above stromal cell types. In exemplary aspects, the sample comprises a combination of immune cells and stromal cells. In exemplary aspects, the sample comprises CD45+ cells.

In exemplary aspects, the sample obtained from the synthetically-engineered pMN substantially lacks tumor cells or cancer cells. In exemplary aspects, less than 10% of the cells in the sample are tumor or cancer cells. In exemplary aspects, less than 5% of the cells in the sample are tumor or cancer cells. In exemplary aspects, less than 3% of the cells in the sample are tumor or cancer cells. In exemplary aspects, less than 2% of the cells in the sample are tumor or cancer cells. In exemplary aspects, less than 1% of the cells in the sample are tumor or cancer cells. In exemplary aspects, less than 0.5% of the cells in the sample are tumor or cancer cells. In exemplary aspects, less than 0.1% of the cells in the sample are tumor or cancer cells. The presently disclosed methods are advantageous, in part, because the methods are not based on the characterization of the metastatic cells. Rather, the methods are based (at least in part) on the non-cancer/non-tumor cells of the pMN (as represented by the scaffold).

### Synthetically-Engineered pMN

With regard to the methods of the present disclosure, the samples are obtained from a synthetically-engineered pMN, which is also referred to herein as "synthetic pMN" or "engineered pMN" or "synthetic scaffold" or "biomaterial scaffold" or "scaffold". The scaffold may be any scaffold which mimics the cellular and molecular components of the pMN and is able to capture or recruit metastatic cells. In exemplary aspects, the scaffold maintains residence in tissue for several weeks to years and facilitates ingrowth of tissue and the retrieval of that tissue at later time points. Such scaffolds are known in the art. See, e.g., Azarin et al., Nat Commun 6: 8094 (2015); Aguado et al., Sci Rep 5: 17566 (2015); Aguado et al., Acta Biomaterialia (2016); and Rao et al., Cancer Res 76(18): 5209-5218 (2016); U.S. Patent Application Publication No. 2014/0072510 A1; International Patent Application Publication No. WO 2017/120486.

In exemplary embodiments, the scaffold is porous and/or permeable. In exemplary embodiments, the scaffold comprises a polymeric matrix and acts as a substrate permissible for metastasis, colonization, cell growth, etc. In exemplary embodiments, the scaffold provides an environment for attachment, incorporation, adhesion, encapsulation, etc. of agents (e.g., DNA, lentivirus, protein, cells, etc.) that create a metastatic capture site within the scaffold. In exemplary embodiments, agents are released (e.g., controlled or sustained release) to attract circulating tumor cells, metastatic cells, or pre-metastatic cells. With regard to agents (e.g., therapeutic agents) and sustained release, for long term therapy (e.g., days, weeks or months) and/or to maintain the highest possible drug concentration at a particular location in the body, the present disclosure in certain embodiments provides a sustained release depot formulation with the following non-limiting characteristics: (1) the process used to prepare the matrix does not chemically or physically damage the agent; (2) the matrix maintains the stability of the agent against denaturation or other metabolic conversion by protection within the matrix until release, which is important for very long sustained release; (3) the entrapped agent is released from the hydrogel composition at a substantially uniform rate, following a kinetic profile, and furthermore, a particular agent can be prepared with two or more kinetic profiles, for example, to provide in certain embodiments, a loading dose and then a sustained release dose; (4) the desired release profile can be selected by varying the components and the process by which the matrix is prepared; and (5) the matrix is nontoxic and degradable. PEG scaffolds as disclosed herein are also contemplated to function as a scaffold that achieves sustained release of a therapeutically active agent. Accordingly, in some embodiments an agent is configured for specific release rates. In further embodiments, multiple different agents are configured for different release rates. For example, a first agent may release over a period of hours while a second agent releases over a longer period of time (e.g., days, weeks, months, etc.). In some embodiments, and as described above, the scaffold or a portion thereof is configured for sustained release of agents. In some embodiments, the sustained release provides release of biologically active amounts of the agent over a period of at least 30 days (e.g., 40 days, 50 days, 60 days, 70 days, 80 days, 90 days, 100 days, 180 days, etc.).

In exemplary embodiments, the scaffold is partially or exclusively composed of a micro-porous poly(e-caprolactone) (PCL), forming a PCL scaffold. Such PCL scaffolds have a greater stability than the micro-porous poly(lactide-co-glycolide) (PLG) biomaterial scaffolds. In exemplary embodiments, the scaffold comprises PCL and/or PEG and/or alginate.

In some embodiments, the scaffold is a controlled release scaffold formed partially or exclusively of hydrogel, e.g., a poly( ethylene glycol) (PEG) hydrogel to form a PEG scaffold. Any PEG is contemplated for use in the compositions and methods of the disclosure. In general, the PEG has an average molecular weight of at least about 5,000 daltons. In further embodiments, the PEG has an average molecular weight of at least 10,000 daltons, 15,000 daltons, and is preferably between 5,000 and 20,000 daltons, or between 15,000 and 20,000 daltons. Also preferred is PEG having an average molecular weight of 5,000, of 6,000, of 7,000, of 8,000, of 9,000, of 10,000, of 11,000, of 12,000 of 13,000, of 14,000, or of 25,000 daltons. In further embodiments, the PEG is a four-arm PEG. In preferred embodiments, each arm of the four-arm PEG is terminated in an acrylate, a vinyl sulfone, or a maleimide. It is contemplated that use of vinyl sulfone or maleimide in the PEG scaffold renders the scaffold resistant to degradation. It is further contemplated that use of acrylate in the PEG scaffold renders the scaffold susceptible to degradation.

In some embodiments, one or more agents are associated with a scaffold to establish a hospitable environment for metastasis and/or to provide a therapeutic benefit to a subject. Agents may be associated with the scaffold by covalent or non-covalent interactions, adhesion, encapsulation, etc. In some embodiments, a scaffold comprises one or more agents adhered to, adsorbed on, encapsulated within, and/or contained throughout the scaffold. The present disclosure is not limited by the nature of the agents. Such agents include, but are not limited to, proteins, nucleic acid molecules, small molecule drugs, lipids, carbohydrates, cells, cell components, and the like. In various embodiments, the agent is a therapeutic agent. In some embodiments, two or more (e.g., 3, 4, 5, 6, 7, 8, 9, 10 ...20 ... 30 ... 40 ..., 50, amounts therein, or more) different agents are included on or within the scaffold. In some embodiments, agents associated with a scaffold include metastatic markers, such as: CD133 (which generally defines all progenitors), VEGF -1 (hematopoietic progenitor cells (HPCs)), VEGFR-2 (endothelial progenitor cells (EPCs)), CDIIb and GR1 (myeloid-derived suppressor cells), F4/80 and CDIIb (macrophages), and CDllb+CD115+Ly6c+(inflammatory monocytes). In some embodiments, agents associated with a scaffold include lentivirus encoding a gene that aids in establishing a hospitable environment for metastasis and/or providing a therapeutic benefit to a subject. In other embodiments, no agents are provided with the scaffold.

In further embodiments, it is contemplated that a scaffold of the disclosure recruits more and/or different cells relative to a scaffold that comprises, e.g., PLG. For example, in some embodiments a scaffold of the disclosure recruits more tumor cells than a scaffold that comprises, e.g., PLG. In various embodiments, a scaffold of the disclosure recruits and/or captures about 5, 10, 20, 50, 100, 200, 500, 1000 or more cells relative to a scaffold that comprises, e.g., PLG. In some embodiments, the types of cells that associate with a scaffold of the disclosure are different from a scaffold that comprises, e.g., PLG. For example and without limitation, a higher percentage of CD49b cells are found in association with a PCL scaffold relative to a PLG scaffold; further, there are about equal quantities of F4/80 and CDIIc cells in association with a PLG scaffold, whereas there are three times as many CDIIc cells as F4/80 cells in association with a PCL scaffold.

In certain embodiments, the scaffold comprises a polymeric matrix. In some embodiments, the matrix is prepared by a gas foaming/particulate leaching procedure, and includes a wet granulation step prior to gas foaming that allows for a homogeneous mixture of porogen and polymer and for sculpting the scaffold into the desired shape.

Thus, in some aspects, the scaffolds may be formed of a biodegradable polymer, e.g., PCL, that is fabricated by emulsifying and homogenizing a solution of polymer to create microspheres. Other methods of microsphere production are known in the art and are contemplated by the present disclosure. See, e.g., U.S. Patent Application Publication Numbers 2015/0190485 and 2015/0283218. The microspheres are then collected and mixed with a porogen (e.g., salt particles), and the mixture is then pressed under pressure. The resulting discs are heated, optionally followed by gas foaming. Finally, the salt particles are removed. The fabrication provides a mechanically stable scaffold which does not compress or collapse after in vivo implantation, thus providing proper conditions for cell growth.

In some aspects, the scaffolds are formed of a substantially non-degradable polymer, e.g., PEG. Degradable hydrogels encapsulating gelatin microspheres may be formed based on a previously described Michael-Type addition PEG hydrogel system with modifications [Shepard et al., Biotechnol Bioeng. 109(3): 830-9 (2012)]. Briefly, four-arm polyethylene glycol) vinyl sulfone (PEG-VS) (20 kDa) is dissolved in 0.3 M triethanolamine (TEA) pH 8.0 at a concentration of 0.5 mg^L to yield a final PEG concentration of 10%. The plasmin-degradable trifunctional (3 cysteine groups) peptide crosslinker (Ac-GCYKN CGYKN CG) is dissolved in 0.3 M TEA pH 10.0 to maintain reduction of the free thiols at a concentration that maintain a stoichiometrically balanced molar ratio of VS:SH. Prior to gelation, gelatin microspheres are hydrated with 10 µí sterile Millipore or lentivirus solution. Subsequently, the PEG and peptide crosslinking solutions are mixed well and immediately added to the hydrated gelatin microspheres for encapsulation. In some embodiments, and as described above, salt is used as the porogen instead of gelatin microspheres. In this case, the PEG solution is made in a saturated salt solution, so that the porogen does not significantly dissolve.

In some embodiments, UV crosslinking is used instead of peptide crosslinking. Ultraviolet crosslinking is contemplated for use with PEG-maleimide, PEG-VS, and PEG-acrylate.

Scaffolds of the present disclosure may comprise any of a large variety of structures including, but not limited to, particles, beads, polymers, surfaces, implants, matrices, etc. Scaffolds may be of any suitable shape, for example, spherical, generally spherical (e.g., all dimensions within 25% of spherical), ellipsoidal, rod-shaped, globular, polyhedral, etc. The scaffold may also be of an irregular or branched shape.

In some embodiments, a scaffold comprises nanoparticles or microparticles (e.g., compressed or otherwise fashioned into a scaffold). In various embodiments, the largest cross-sectional diameters of a particle within a scaffold is less than about 1,000 µm, 500 µm, 200 µm, 100 µm, 50 µm , 20 µm, 10 µm, 5 µm, 2 µm, 1 µm, 500 nm, 400 nm, 300 nm, 200 nm or 100 nm. In some embodiments, a population of particles has an average diameter of: 200-1000 nm, 300-900 nm, 400-800 nm, 500-700 nm, etc. In some embodiments, the overall weights of the particles are less than about 10,000 kDa, less than about 5,000 kDa, or less than about 1,000 kDa, 500 kDa, 400 kDa, 300 kDa, 200 kDa, 100 kDa, 50 kDa, 20 kDa, 10 kDa.

In some embodiments, a scaffold comprises PCL. In further embodiments, a scaffold comprises PEG. In certain embodiments, PCL and/or PEG polymers and/or alginate polymers are terminated by a functional group of chemical moiety (e.g., ester-terminated, acid-terminated, etc.).

In some embodiments, the charge of a matrix material (e.g., positive, negative, neutral) is selected to impart application-specific benefits (e.g., physiological compatibility, beneficial interactions with chemical and/or biological agents, etc.). In certain embodiments scaffolds are capable of being conjugated, either directly or indirectly, to a chemical or biological agent). In some instances, a carrier has multiple binding sites (e.g., 2, 3, 4, 5, 6, 7, 8, 9, 10 ... 20 ... 50 ... 100, 200, 500, 1000, 2000, 5000, 10,000, or more).

In exemplary embodiments, the life times of the scaffolds are well within the timeframe of clinical significance are demonstrated. For example, stability lifetimes of greater than 90 days are contemplated, with percent degradation profiles of less than about 50%, 45%, 40%, 35%, 30%, 25%, 20%, 15%, 10%, 5%, and 1% respectively, where the percent degradation refers to the scaffolds' ability to maintain its structure for sufficient cell capture as a comparison of its maximum capture ability. Such ability is measured, for example, as the change in porous scaffold volume over time, the change in scaffold mass over time, and/or the change in scaffold polymer molecular weight over time. These long life times mean that scaffolds can now be applied in patient-friendly conditions that allow subjects to wear the scaffold under normal daily living conditions, inside and outside the clinical environment.

Further still, with the present techniques scaffolds are provided that remain functionalized long enough to provide targeted treatment sites in vivo, that is, locations where metastatic cells are not merely just detected, but over time target, to provide a specific location for cell resection and possible removal of all metastatic cells.

The ability of the present scaffolds to remain functionalized over greater periods of time has, in some examples, provided for formation of a sustained or controllable release scaffold. These scaffolds, for example, may comprise protein responsive materials that are non-degradable when implanted and recruiting metastatic cells. When exposed to activating proteins (e.g., an enzyme), however, these scaffolds degrade to then release the captured metastatic cells. In some examples, such a property is contemplated for use in vitro to facilitate the recovery of the captured cells. For example and without limitation, in some examples the scaffold is an alginate scaffold and the activating protein is alginate lyase.

In some embodiments, the scaffold or a portion thereof is configured to be sufficiently porous to permit metastasis of cells into the pores. The size of the pores may be selected for particular cell types of interest and/or for the amount of ingrowth desired and are, for example without limitation, at least about 20 µm, 30 µm, 40 µm, 50 µm, 100 µm, 200 µm, 500 µm, 700 µm, or 1000 µm. In some embodiments, the PEG gel is not porous but is instead characterized by a mesh size that is, e.g., 10 nanometers (nm), 15 nm, 20 nm, 25 nm, 30 nm, 40 nm, or 50 nm.

The effectiveness of the longer lifetime scaffolds herein, especially for use as targeted treatment sites, relates to Paget's "seed and soil" paradigm which proposes that, prior to colonization by metastatic cells, supportive cells (e.g., fibroblasts, immune cells, endothelial cells), soluble factors, and extracellular matrix (ECM) components establish a microenvironment conducive to tumor cell homing and colonization. The importance of this paradigm is that metastasis to specific organs is not random, but rather is influenced by the properties of the local environment. The use of longer lifetime scaffolds, in vivo, provides sufficient time for these support cells to establish the microenvironment, at the scaffold, to which metastatic cells are attracted. The initial translation of these principals led to the development and implementation of micro-porous poly(lactide-co-glycolide) (PLG) biomaterial scaffolds, as described in the literature. There, the recruitment of metastatic breast cancer cells through the local immune response in vivo was demonstrated. However, PLG scaffolds were degradable over time scales considered too short for clinical translation.

### Sites of Metastasis and Scaffold Implantation Sites

While metastatic cancer can spread to essentially any part of the body, different types of cancers tend to spread to particular body parts. Common sites of metastasis are shown in the table below.

| **Cancer Type** | **Main Sites of Metastasis** |
|---|---|
| Bladder | Bone, liver, lung |
| Breast | Bone, brain, liver, lung |
| Colon | Liver, lung, peritoneum |
| Kidney | Adrenal gland, bone, brain, liver, lung |
| Lung | Adrenal gland, bone, brain, liver, other lung |
| Melanoma | Bone, brain, liver, lung, skin, muscle |
| Ovary | Liver, lung, peritoneum |
| Pancreas | Liver, lung, peritoneum |
| Prostate | Adrenal gland, bone, liver, lung |
| Rectal | Liver, lung, peritoneum |
| Stomach | Liver, lung, peritoneum |
| Thyroid | Bone, liver, lung |
| Uterus | Bone, liver, lung, peritoneum, vagina |

| | |
|---|---|
| Source: National Cancer Institute website at cancer.gov/types/metastatic-cancer. | |

The site at which the scaffold is implanted desirably is one of the above common sites of metastasis. In exemplary aspects, the site at which the scaffold is implanted in the subject is a lung, liver, brain, bone, peritoneum, omental fat, muscle, or lymph node of the subject. Additionally or alternatively, in some embodiments, the scaffold is implanted subcutaneously. In exemplary aspects, more than one scaffold is implanted in the subject. In exemplary aspects, the subject comprises more than one scaffold. In further examples, at least 1, at least 2, at least 3, at least 4, at least 5, at least 6, at least 7, at least 8, at least 9, or at least 10 scaffolds are implanted in a subject. In exemplary aspects, samples from each of the scaffolds implanted in the subject are obtained and expression profiles of each sample are measured.

### Measuring Expression Levels

The methods of the present disclosure relate to measuring a level of expression of a gene, an RNA, e.g., a messenger RNA (mRNA), or a protein, in a sample obtained from a scaffold implanted in a subject. In exemplary embodiments, the methods comprise measuring a combination of at least two of an expression level of a gene, an RNA, and a protein. In exemplary embodiments, the methods comprise measuring the expression level of at least one gene, at least one RNA, and at least one protein. In exemplary instances, the methods comprise measuring the expression level of at least 2, 3, 4, 5 or more genes, at least 2, 3, 4, 5 or more RNA, and/or at least 2, 3, 4, 5 or more proteins in the sample. In exemplary instances, the methods comprise measuring the expression level of at least 10, 15, 20 or more genes, at least 10, 15, 20 or more RNA, and/or at least 10, 15, 20 or more proteins in the sample. In exemplary instances, the methods comprise measuring the expression level of at least 50, 100, 200 or more genes, at least 50, 100, 200 or more RNA, and/or at least 50, 100, 200 or more proteins in the sample. In exemplary instances, the methods comprise measuring the expression level of a plurality of different genes, a plurality of RNA, and/or a plurality of proteins.

In exemplary aspects, the methods comprise measuring the expression level of more than 10 different genes, more than 100 different genes, more than 1000 different genes, more than 5000-10,000 different genes, and the expression levels of the different genes constitute a gene signature.

In exemplary aspects, the methods comprise measuring the expression level of more than 10 different RNA, more than 100 different RNA, more than 1000 different RNA, more than 5000-10,000 different RNA, and the expression levels of the different RNA constitute an RNA signature, or a transcriptome.

In exemplary aspects, the methods comprise measuring the expression level of more than 10 different proteins, more than 100 different proteins, more than 1000 different proteins, more than 5000-10,000 different proteins, and the expression levels of the different proteins constitute a protein signature, or a proteome.

In exemplary aspects, the methods comprise measuring the expression level of the S100A8 gene or the S100A9 gene, or an expression product (e.g., an RNA or protein) encoded thereby. In exemplary aspects, the methods comprise measuring the expression level of the S100A8 gene or the S100A9 gene, the S100A8 RNA or the S100A9 RNA, or the S100A8 protein or the A100A9 protein. In exemplary aspects, the method comprises measuring a level of expression of both the S100A8 gene and the S100A9 gene or the expression products of both genes. In exemplary aspects, the method comprises measuring a level of expression of the RNA or protein encoded by the S100A8 gene or the S100A9 gene or both.

In exemplary aspects, the methods comprise measuring a level of expression of one or more of the following genes: Ccl22, Cxcl2, Ccr7, Csf3, Bmp15, IL-23a, S100A9, Chi3I3, Pglryp1, S100A8, Ltf, Ela2, and Camp. In exemplary aspects, the methods comprise measuring the level of expression at the gene level, the RNA level, or the protein level, or a combination thereof. In exemplary aspects, the method comprises measuring a level of expression of the RNA or protein encoded by one or more of the following genes: Ccl22, Cxcl2, Ccr7, Csf3, Bmp15, IL-23a, S100A9, Chi3I3, Pglryp1, S100A8, Ltf, Ela2, and Camp. In exemplary aspects, the method comprises measuring a level of expression of one or more of Csf3, IL-1a, IL-12p70, IL-6, Cxcl5, IL-15, Cxcl10, Ccl2, Cxcl9, and Ccl5 at the gene, RNA and/or protein level.

In exemplary aspects, the methods comprise measuring a level of expression of one or more of the following genes: Ccl22, Ccr7, Bmp15, S100A9, Chi3I3, Pglryp1, S100A8, Ltf, Ela2, and Camp. In exemplary aspects, the methods comprise measuring the level of expression at the gene level, the RNA level, or the protein level, or a combination thereof. In exemplary aspects, the method comprises measuring a level of expression of the RNA or protein encoded by one or more of the following genes: Ccl22, Ccr7, Bmp15, S100A9, Chi3I3, Pglryp1, S100A8, Ltf, Ela2, and Camp.

In exemplary aspects, the methods comprise measuring a level of expression of one or more of the following genes or the RNA or protein encoded by one or more of the following genes: Gapdh, Hmbs, Tbp, Ubc, Ywhaz, Bmp15, Camp, Ccl22, Ccr7, Chi3I3, Csf3, Cxcl2, Ela2, Il23a, Ltf, Pglyrp1, S100a8, S100a9. In additional or alternative exemplary aspects, the methods comprise measuring a level of expression of one or more of the following genes or the RNA or protein encoded by one or more of the following genes: Actb, B2m, Cdkn1a, Gusb, Hprt1, Ipo8, Pgk1, Polr2a, Ppia, Rplp2, Tfrc, 5730403B10Rik, A2m, Abcb1a, Abcf1, Acvr1, Acvr2b, Acvrl1, Adipoq, Adora1, Adora2a, Adora3, Adrb2, Afap1I2, Aif1, Aimp1, Aimp1, Akt1, Alox15, Alox5, Alox5, Alox5, Alox5, Alox5, Alox5, Alox5ap, Anxa1, Aoah, Aoc3, Aox1, Apcs, Apoa1, Apoa1, Apoa4, Apoe, Areg, Atrn, Axl, B4galt1, Bad, Bcl10, Bcl6, Bdkrb1, Bdkrb2, Blnk, Bmp1, Bmp10, Bmp2, Bmp3, Bmp3, Bmp4, Bmp5, Bmp6, Bmp6, Bmp7, Bmp7, Bmp7, Bmp8a, Bmp8b, Bmpr1b, Bre, C1qa, C1qb, C1qc, C1rl, C1s, C2, C3, C3ar1, C3ar1, C6, C8a, C8b, C8g, C9, Casp1, Casp8, Cav1, Ccbp2, Cell, Ccl11, Ccl17, Ccl19, Ccl2, Ccl20, Ccl24, Ccl25, Ccl26, Ccl28, Ccl3, Ccl4, Ccl5, Ccl5, Ccl6, Ccl7, Ccl7, Ccl8, Ccr1, Ccr1I1, Ccr2, Ccr3, Ccr3, Ccr4, Ccr5, Ccr6, Ccr8, Ccr8, Ccr9, Ccrl1, Ccrl2, Cd14, Cd163, Cd180, Cd1d1, Cd24a, Cd274, Cd28, Cd4, Cd40, Cd40lg, Cd44, Cd46, Cd55, Cd70, Cd74, Cd80, Cd86, Cd97, Cdk5, Cdo1, Cebpa, Cebpb, Cer1, Cfb, Cfd, Cfh, Cfhr1, Cfi, Cfp, Cftr, Chrna7, Chst1, Chst2, Chst4, Cklf, Clcf1, Clec7a, Clu, Cmklr1, Cmtm2a, Cmtm2b, Cmtm3, Cmtm4, Cmtm5, Cmtm6, Cmtm7, Cmtm8, Cntfr, Cntfr, Cntnap1, Cr1I, Cr2, Crh, Crlf1, Crp, Csf1, Csf2, Csf2, Csf2ra, Csf2rb, Csf3, Ctf1, Ctla4, Cx3cl1, Cx3cr1, Cxcl1, Cxcl1, Cxcl10, Cxcl11, Cxcl12, Cxcl14, Cxcl16, Cxcl17, Cxcl3, Cxcl5, Cxcl9, Cxcr3, Cxcr3, Cxcr4, Cxcr6, Cxcr7, Cybb, Cyp26b1, Darc, Ddx58, Dnajc8, Ebi3, Eda2r, Egfr, Egfr, Ephx2, Epo, Erap1, Erbb2, Ereg, F11r, F2, F2r, F2rl1, F3, Fabp4, Fabp4, Fam3b, Fam3c, Fas, Fasl, Fceria, Fcerlg, Fcgr2b, Fgf10, Fgf11, Fgf12, Fgf18, Fgf2, Fgf20, Fgf23, Figf, Flt3I, Fn1, Fos, Fos, Foxp3, Fpr1, Fpr3, Gal, Gdf11, Gdf15, Gdf2, Gdf3, Gdf5, Gdf6, Gdf9, Gh, Ghrl, Ghsr, Glmn, Gpr17, Gpr68, Gpx1, Gpx4, Grem1, Grem2, Grn, Gsk3b, Gusb, Gusb, H2Q10, H47, Havcr2, Hc, Hdac4, Hdac4, Hdac4, Hdac5, Hdac5, Hdac5, Hdac7, Hdac7, Hdac9, Hgf, Hgf, Hgf, Hif1a, Hmgb1, Hmox1, Hprt1, Hprt1, Hprt1, Hpse, Hrh1, Hrh1, Hsp90ab1, Hspd1, Icos, Icosl, Ifih1, Ifna12, Ifna14, Ifna2, Ifna4, Ifnar1, Ifnar2, Ifnb1, Ifne, Ifng, Ifng, Ifngr1, Ifngr2, Ifnk, Igf1, Igfbp4, Ik, Il10, Il10ra, Il10rb, Il12a, Il12a, Il12b, Il12b, Il12rb1, Il12rb2, Il13, Il13, Il13ra1, Il13ra1, Il13ra2, Il15, Il15ra, Il16, Il17a, Il17c, Il17d, Il17f, Il17ra, Il17rb, Il18, Il18bp, Il18rap, Il19, Il1a, Il1b, Il1b, Il1f10, Il1f5, Il1f6, Il1f9, Il1r1, Il1rap, Il1rapl2, Il1rl1, Il1rl2, Il1rn, Il2, Il2, Il20, Il21, Il22;Iltifb, Il22ra1, Il22ra2, Il23a, Il23r, Il24, Il27, Il27ra, Il28b, Il28ra, Il28ra, Il2rg, Il3, Il31ra, Il33, Il3ra, Il4, Il4ra, Il5, Il5ra, Il6, Il6, Il6, Il6ra, Il6st, Il7r, Il8ra, Il8rb, Il9, Inha, Inhba, Inhbb, Ins2, Irak1, Irak2, Irak3, Irak4, Irf3, Irf3, Irf7, Itgal, Itgam, Itgb2, Itgb2I, Itgb6, Jak1, Jak2, Jak3, Jun, Kit, Kitl, Klf6, Klkb1, Klrg1, Kng1, Krt1, Krt7, Krt8, Lbp, Lefty1;Lefty2, Lefty2, Lefty2, Lefty2, Lep, Lepr, Lif, Lifr, Lifr, Lilrb3, Lrp8, Lta4h, Ltb, Ltb4r1, Ltbp4, Ltbp4, Ltbr, Ly75, Ly86, Lyn, Malt1, Map2k3, Map2k6, Mapk14, Mapk8, Mapkapk2, Masp1, Masp2, Mbl2, Mefv, Mgll, Mmp25, Mrc1, Mstn, Muc1, Muc4, Myd88, Ncam1, Ncf1, Ndst1, Nfam1, Nfatc3, Nfatc4, Nfe2I1, Nfkb1, Nfrkb, Nfx1, Nlrc4, Nlrp3, Nmi, Nod1, Nod2, Nodal, Nono, Nos2, Nox4, Nr3c1, Nup85, Oit1, Olr1, Orm1, Osm, Oxgr1, P2rx1, P2rx7, P2ry1, Pdgfb, Pik3r1, Pla2g2d, Pla2g2e, Pla2g4c, Pla2g7, Plaa, Plp2, Pparg, Pparg, Ppbp, Ppia, Ppia;E030024N20Rik, Prdx5, Prg2, Prl7d1, Procr, Prok2, Prok2, Prtn3, Ptafr, Pten, Ptges, Ptgs1, Ptgs2, Ptn, Ptpn6, Ptprc, Ptx3, Ptx3, Pxdn, Pxmp2, Pycard, Rac1, Rbm4, Rcan1, Reg3a, Reg3g, Rela, Retn, Rhoa, Rhoa, Rhoa, Ripk2, Ryr1, S100a8, S100b, S1pr1, S1pr3, Saa1, Saa3, Scgb3a1, Scn9a, Scube1, Sdcbp, Sectm1b, Sele, Selp, Serpina1a, Serpina3c, Serpinf2, Serping1, Sftpa1, Sh2b2, Siglec1, Siva1, Slco1a4, Slurp1, Socs1, Socs2, Sod1, Sod1, Spp1, Spred1, Spred2, Stab1, Stat1, Stat3, Stat4, Stat5b, Stat6, Tacr1, Tgfb1, Tgfb2, Tgfbr1, Tgfbr1, Tgm2, Thpo, Timm50, Tirap, Tlr1, Tlr1, Tlr1;Tlr6, Tlr11, Tlr12, T!r13, Tlr2, Tlr3, Tlr4, Tlr5, Tlr6, Tlr7, Tlr9, Tnc, Tnf, Tnfaip3, Tnfaip6, Tnfrsf11b, Tnfrsf14, Tnfrsf18, Tnfrsf19, Tnfrsf1a, Tnfrsf1a, Tnfrsf1b, Tnfrsf25, Tnfrsf4, Tnfrsf8, Tnfrsf9, Tnfsf10, Tnfsf11, Tnfsf 12;Tnfsf 12-tnfsf 13, Tnfsf13;Tnfsf12-tnfsf13, Tnfsf13b, Tnfsf14, Tnfsf15, Tnfsf18, Tnfsf4, Tnfsf8, Tnfsf9, Tollip, Tpst1, Trend, Trf, Trip6, Trp53, Trpv1, Tslp, Twist1, Txlna, Unc13d, Vegfa, Vegfb, Vegfc, Vip, Vps45, Wnt16, Xcl1, Xcr1, Zfp36.

The above genes, as well as the RNA and proteins encoded by the genes, are known in the art. The sequences of each are available at the website for the National Center for Biotechnology Information (see Table A), some sequences of which are provided in the sequence listing submitted herewith.

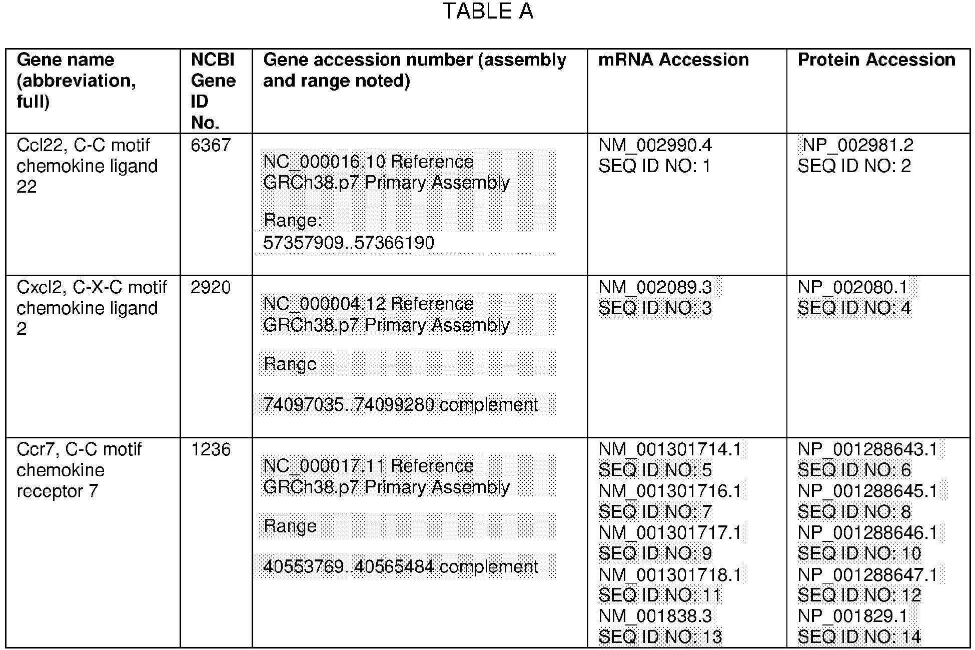

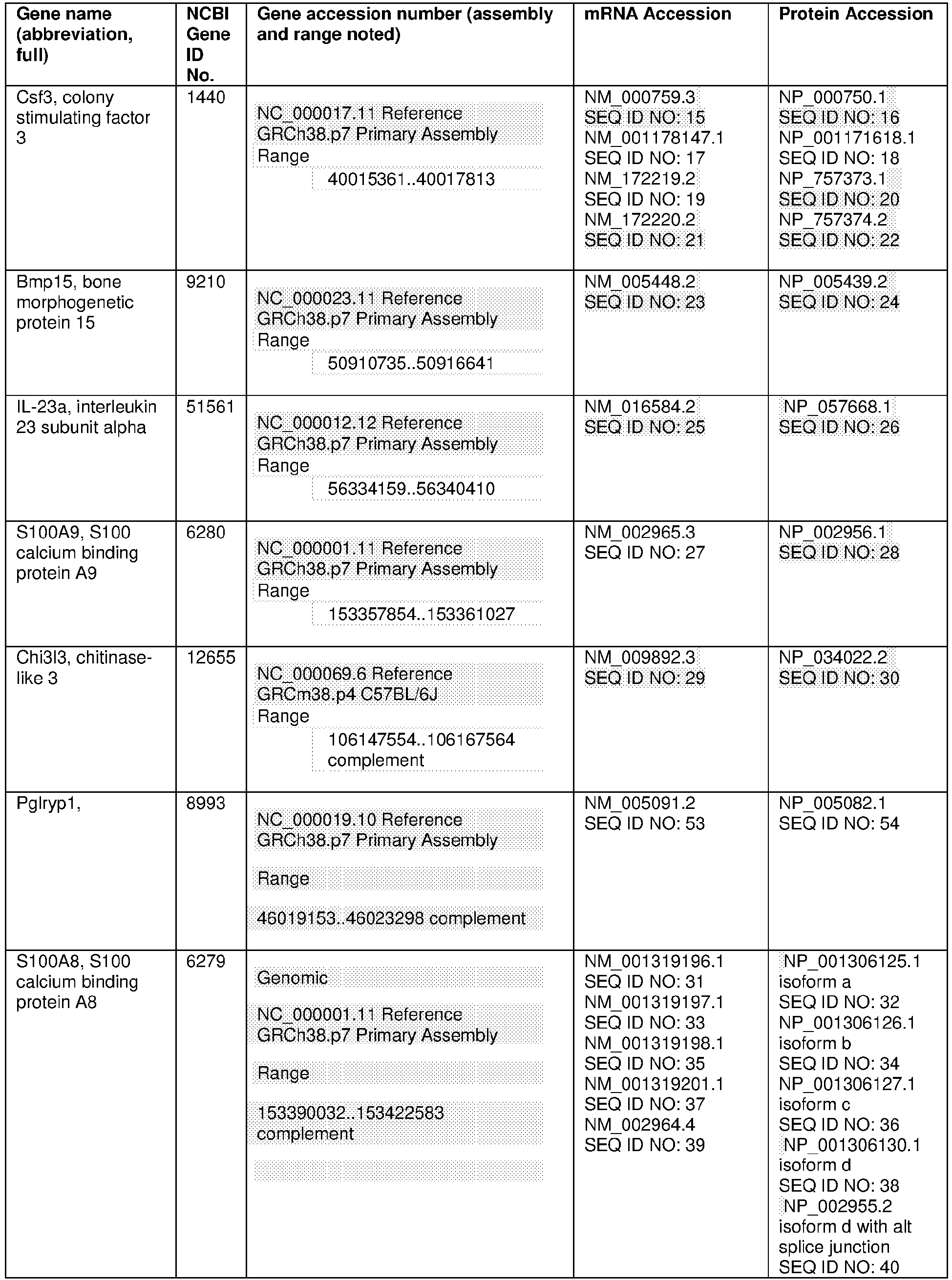

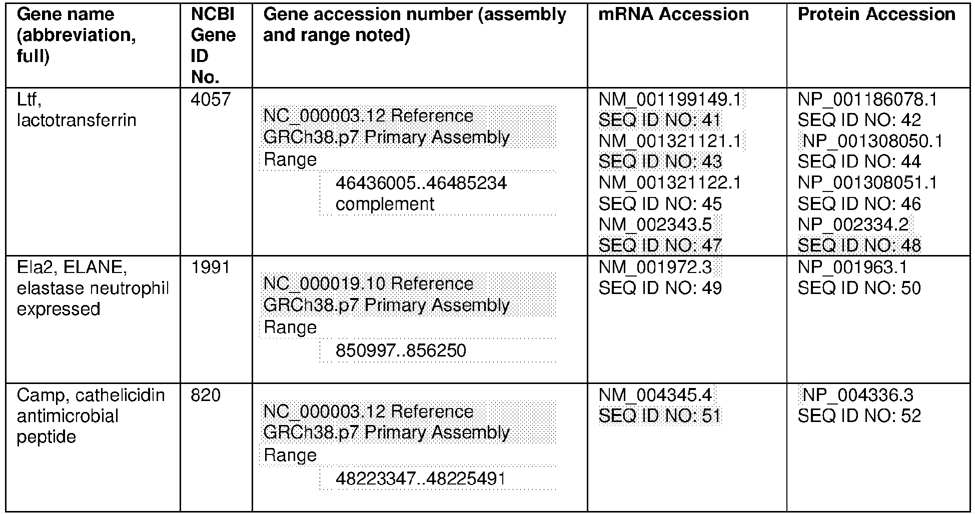

Suitable methods of determining expression levels of nucleic acids (e.g., mRNA) are known in the art and include quantitative polymerase chain reaction (qPCR), including, but not limited to, quantitative real-time PCR (qRT-PCR), RNAseq, Northern blotting and Southern blotting. Techniques for measuring gene expression include, for example, gene expression assays with or without the use of gene chips are described in Onken et al., J Molec Diag 12(4): 461-468 (2010); and Kirby et al., Adv Clin Chem 44: 247-292 (2007). Affymetrix gene chips and RNA chips and gene expression assay kits (e.g., Applied Biosystems^{™} TaqMan^{®} Gene Expression Assays) are also commercially available from companies, such as ThermoFisher Scientific (Waltham, MA). Suitable methods of determining expression levels of proteins are known in the art and include immunoassays (e.g., Western blotting, an enzyme-linked immunosorbent assay (ELISA), a radioimmunoassay (RIA), and immunohistochemical assay) or bead-based multiplex assays, e.g., those described in Djoba Siawaya JF, Roberts T, Babb C, Black G, Golakai HJ, Stanley K, et al. (2008) An Evaluation of Commercial Fluorescent Bead-Based Luminex Cytokine Assays. PLoS ONE 3(7): e2535. Proteomic analysis which is the systematic identification and quantification of proteins of a particular biological system are known. Mass spectrometry is typically the technique used for this purposes. Protein expression Suitable methods of measuring expression are described herein. See the section entitled EXAMPLES.

### Controls

In the methods described herein, the level that is measured may be the same as a control level or a cut off level or a threshold level, or may be increased or decreased relative to a control level or a cut off level or a threshold level. In some aspects, the control level is that of a control subject which may be a matched control of the same species, gender, ethnicity, age group, smoking status, BMI, current therapeutic regimen status, medical history, or a combination thereof, but differs from the subject being diagnosed in that the control does not suffer from the disease in question or is not at risk for the disease. Thus, in exemplary aspects, the control level(s) of the gene(s), RNA, or protein(s) is/are level(s) of a subject known to not have metastatic disease. In alternative aspects, the control level(s) of the gene(s), RNA, or protein(s) is/are level(s) of a subject known to have metastatic disease. In exemplary aspects, as further described herein, the measured level is compared to both a control level of a subject known to not have metastatic disease and a control level of a subject known to have metastatic disease. In exemplary aspects, as further described herein, the measured level is compared to multiple control levels: a control level of a subject known to not have metastatic disease and several control levels of subjects known to have metastatic disease but differ from one another by the stage of metastatic disease. For example, a panel of control levels may be used to compare the measured level and the panel of control levels include a control level of a subject who does not have a tumor or a cancer, a control level of a subject known to have a tumor that has not metastasized, a control level of a subject known to have advanced metastatic disease, a control level of a subject known to be in an early stage of metastatic disease, and a control level of a subject known to be in a mid-stage of metastatic disease. In exemplary aspects, the panel of control levels are graphically displayed as elliptical areas, wherein each elliptical area represents the average and standard deviation of the control level in regards to probability of metastatic disease (e.g., along the y-axis) and a score of metastatic potential (e.g., along the x-axis). In some instances, the measured level is plotted on this graphic and the distance from the nearest control elliptical area is indicative of the subject's metastatic potential.

In exemplary aspects, the method comprises measuring the level of expression of a gene, RNA, or protein at a first time point and at a second time point and the measured level of the first time point serves as a control level or establishes a baseline.

Relative to a control level, the level that is determined may an increased level. As used herein, the term "increased" with respect to level (e.g., expression level, *biological* activity level) refers to any % increase above a control level. The increased level may be at least or about a 5% increase, at least or about a 10% increase, at least or about a 15% increase, at least or about a 20% increase, at least or about a 25% increase, at least or about a 30% increase, at least or about a 35% increase, at least or about a 40% increase, at least or about a 45% increase, at least or about a 50% increase, at least or about a 55% increase, at least or about a 60% increase, at least or about a 65% increase, at least or about a 70% increase, at least or about a 75% increase, at least or about a 80% increase, at least or about a 85% increase, at least or about a 90% increase, at least or about a 95% increase, relative to a control level.

Relative to a control level, the level that is determined may a decreased level. As used herein, the term "decreased" with respect to level (e.g., expression level, *biological* activity level) refers to any % decrease below a control level. The decreased level may be at least or about a 5% decrease, at least or about a 10% decrease, at least or about a 15% decrease, at least or about a 20% decrease, at least or about a 25% decrease, at least or about a 30% decrease, at least or about a 35% decrease, at least or about a 40% decrease, at least or about a 45% decrease, at least or about a 50% decrease, at least or about a 55% decrease, at least or about a 60% decrease, at least or about a 65% decrease, at least or about a 70% decrease, at least or about a 75% decrease, at least or about a 80% decrease, at least or about a 85% decrease, at least or about a 90% decrease, at least or about a 95% decrease, relative to a control level.

### Housekeeping Genes

For purposes herein, the levels of expression are measured and the measured levels may be normalized or calibrated to a level of a housekeeping gene. The housekeeping gene in some aspects is GAPDH, Hmbs, Tbp, Ubc, Ywhaz. In exemplary aspects, the housekeeping gene is any one of those set forth in the Table B or any one of those comprising a sequence of SEQ ID NOs: 55-90.

**TABLE B**

| Gene Name | NCBI Accession No. |
|---|---|
| Actin, beta (ACTB) | NM_001101 |
| aldolase A, fructose-bisphosphate (ALDOA) | NM_000034 |
| Glyceraldehyde-3-phosphate dehydrogenase (GAPDH) | NM_002046 |
| Phosphoglycerate kinase 1 (PGK1) | NM_000291 |
| Lactate dehydrogenase A (LDHA), | NM_005566 |
| Ribosomal protein S27a (RPS27A) | NM_002954 |
| Ribosomal protein L19 (RPL19) | NM_000981 |
| Ribosomal protein L11 (RPL11) | NM_000975 |
| Non-POU domain containing, octamer-binding | NM_007363 |
| (NONO) | |
| Rho GDP dissociation inhibitor (GDI) alpha (ARHGDIA) | NM_004309 |
| Ribosomal protein L32 (RPL32) | NM_000994 |
| Ubiquitin C (UBC) | NM_021009 |
| HMBS | NM_000190.3 |
| | NM_001024382.1 |
| | NM_001258208.1 |
| | NM_001258209.1 |
| TBP | NM_001172085.1 |
| | NM_003194.4 |
| Ywhaz | NM_001135699.1 |
| | NM_001135700.1 |
| | NM_001135701.1 |
| | NM_001135702.1 |
| | NM_003406.3 |
| | NM_145690.2 |

### Subjects

In exemplary aspects, the subject is a mammal, including, but not limited to, mammals of the order Rodentia, such as mice and hamsters, and mammals of the order Logomorpha, such as rabbits, mammals from the order Carnivora, including Felines (cats) and Canines (dogs), mammals from the order Artiodactyla, including Bovines (cows) and Swines (pigs) or of the order Perssodactyla, including Equines (horses). In some aspects, the mammals are of the order Primates, Ceboids, or Simoids (monkeys) or of the order Anthropoids (humans and apes). In some aspects, the mammal is a human. In some aspects, the human is an adult aged 18 years or older. In some aspects, the human is a child aged 17 years or less. In exemplary aspects, the subject has a tumor or cancer. The tumor or cancer may be any of those known in the art or described herein.

### Additional Steps

With regard to the methods of the disclosure, the methods may include additional steps. For example, the method may include repeating one or more of the recited step(s) of the method. Accordingly, in exemplary aspects, the method comprises measuring a level of expression of a gene, an RNA, or a protein, in a sample obtained from a scaffold and re-measuring the level, e.g., at a different time point, for accuracy. In exemplary aspects, the method comprises implanting the scaffold into the subject. In exemplary aspects, the method comprises obtaining the sample from the subject. In exemplary embodiments, more than one sample is obtained from the scaffold. In exemplary embodiments, 2, 3, 4, 5, 6, 7, 8, 9, 10, or more samples are obtained from the scaffold, each sample obtained at a different point in time. In exemplary aspects, a sample is obtained from the scaffold once a day, 2x per day, 3x per day, 4x per day or more frequently. In exemplary aspects, a sample is obtained from the scaffold every 2, 3, 4, 5, or 6 days. In exemplary aspects, a sample is obtained from the scaffold once a week or once every 2 weeks, 3 weeks, 4 weeks, 5 weeks, 6 weeks, 7 weeks, or 8 weeks or less frequently. In exemplary aspects, a sample is obtained on a regular basis based on the analysis of a first sample. In exemplary aspects, a sample is obtained on a regular basis until a pre-determined goal is met. In exemplary aspects, the pre-determined goal is the determination of the subject as exhibiting a complete therapeutic response to a treatment, e.g., chemotherapy, immune therapy, gene therapy, radiation, surgical resection.

In exemplary aspects, the method comprises measuring an expression level for every sample obtained. In exemplary aspects, the expression level is measured within 1, 4, 6, 8, 12, 16, or 24 hours of obtaining the sample. In exemplary aspects, the sample is cryopreserved and expression of the sample is determined at a later time.

In exemplary aspects, the methods comprise processing the sample for measurement of expression. For example, the methods may comprise RNA isolation from cells of the scaffold. The methods may comprises homogenizing in a Trizol reagent for RNA isolation or in a detergent for protein isolation. In exemplary aspects, it is desirable to fractionate the cells of the sample into cell type specific sub-populations. In this regard, the methods comprise in exemplary instances a step for separating the cells of the scaffold into cell type specific sub-populations. In exemplary aspects, the cells of the scaffold are separated into subpopulations of immune cells and stromal cells. In exemplary instances, the cells of the scaffold are separated into fibroblasts, endothelial cells, dendritic cells, CD45+ cells, T-cells, B-cells, monocytes, macrophages, MDSCs, neutrophils, and natural killer cells. In exemplary aspects, the methods comprise measuring or quantifying the different cell populations in the sample and/or measuring tumor cell populations in the sample, in addition to or instead of measuring a level of expression of a gene, an RNA or a protein, in a sample obtained from a synthetically-engineered pre-metastatic niche (pMN) implanted in the subject. In exemplary aspects, the methods comprise determining the cell population profile of the sample. In exemplary aspects, the methods comprises determining the cell signature of the sample. Methods of measuring cell populations in a sample may be performed by known techniques, including, fluorescent-assisted cell sorting (FACS), magnetic-assisted cell sorting, histological techniques, e.g., fluorescent immunohistochemistry or multiplexed fluorescent imaging technologies. In exemplary aspects, the methods comprise monitoring the cell populations over time.

In exemplary aspects, the method comprises measuring an expression level of the sample in more than one way. In exemplary instances, the methods comprise measuring expression using a gene chip and an ELISA or other immunoassay. In exemplary instances, the methods comprise measuring expression levels of one or more housekeeping genes and comparing the measured levels of genes to housekeeping genes. In exemplary aspects, the methods comprises normalizing the expression level data to expression levels of one or more housekeeping genes.

In exemplary aspects, when the method comprises, e.g., measuring a level of expression of a plurality of genes, RNA, and/or proteins, the methods comprise constructing a matrix of expression levels, e.g., a matrix of gene expression levels, a matrix of RNA expression levels, a matrix of protein expression levels. The matrices can be further categorized into biological pathways or functions, e.g., a sub-matrix of genes, RNA, and/or proteins involved in cell cycle control, immune cell proliferation and activation, metastatic intravasation, extravasation, homing, migration, or colonization, or a sub-matrix for extracellular matrix (ECM) components. The matrices may be manipulated to remove data points or add data points from a second source of data, an ELISA vs. a multiplex bead-based assay. The expression levels of the matrices may be normalized to housekeeping genes and/or to total protein concentrations. In exemplary aspects, the methods comprise analyzing the expression level matrices for expression level changes, significance, false-detection rate of key variables, and for the identification of analytes of particular interest via regularization, supervised discriminate analysis (e.g., using Lasso or Elastic Net; Partial Least-Squares Discriminate Analysis (PLS-DA); feature selection).

In exemplary aspects, the matrices are compressed into a single metric or score. In exemplary aspects, such decompression of multiple variables into a single metric or score is accomplished via linear combination of multiple gene or protein expression levels, singular value decomposition (SVD), dynamic mode decomposition (DMD), principle component analysis (PCA), or Fisher linear discriminant. Methods of SVD for similar purposes are described in Alter et al., Proc Natl Acad Sci USA. 2000 Aug 29; 97(18): 10101-10106.

In exemplary aspects, a machine learning algorithm is employed to attain a prediction of metastatic disease state or status. The algorithm may comprise one or more of logistic regression, discriminant analysis (linear or quadratic), random forest generation or other ensemble/decision tree classification, neural networks pattern recognition (hidden layers), support vector machines, nearest neighbor (fine-course weighted) and Bayesian networks.

In exemplary aspects, the method comprises providing to the subject a treatment or therapy purposed for treating metastatic disease. The treatment or therapy purposed for treating metastatic disease may be any of those known in the art or described herein. In exemplary instances, the therapy is surgical resection or radiation therapy. In exemplary aspects, the treatment comprises administration of one or more chemotherapeutic agents.

In exemplary aspects, the method further comprises combining multiple metrics into a multivariate signature. This step may comprise combining multiple scoring and prediction algorithms to a graphical or numerical output for identification of trends, correlations and outliers. Visual outputs for correlation include side by side box or bar plots for each algorithm or a combination thereof into a multidimensional output (e.g., multivariable plot of SVD score and RF prediction). Example figures show the SVD score plotted on the x-axis and the RF prediction plotted on the y-axis. This outline is flexible. The general concept is to establish a parameter space composed of these multivariate metrics with high and low end to aid categorization of test samples.

Further details relating to data processing are provided below.

### Data Processing

The present techniques further include measuring levels of the genes, RNA, or proteins and determining a pMN expression signature for a subject using a processing system. For example, in exemplary embodiments, metastatic potential is determined or metastatic disease, or a predisposition thereto, is detected by measuring a level of expression of a gene, a RNA or a protein, or a combination thereof, in a sample obtained from a synthetically-engineered pMN implanted in a subject. In exemplary embodiments, a level of expression of a plurality of genes, RNA, and/or proteins, are measured. The measured expression level data are then provided to a processing system that compares the measured expression level data to control levels of the expression data. In some exemplary embodiments, the processing system compares measured expression level data of a first time instance to measured expression level data taken at a second time instance, or to a plurality of time instances. In exemplary aspects, the first time point occurs before a treatment and the second time point occurs after a treatment and the method is a method of determining the efficacy of a treatment for metastatic disease.

The processing system may compare measured expression level data using different algorithm-based analyses subsystems. In some examples, one type of subsystem employs a compression algorithm, such as a single value decomposition (SVD) algorithm that receives a plurality of different measured expression level data and compresses that data into a single scoring metric. In some examples, one type of subsystem employs a probability determination, such as a Random Forest algorithm, that indicates a probability of metastases for a pMN. In some examples, that probability indicates a subject's metastatic potential or metastatic disease.

An example implementation of the processing system to determine pMN expression signature for a subject is as follows.

At an initial processing stage, sample implantation is performed, followed by retrieval. An implant polymer scaffold, in accordance with the teachings herein and other example scaffolds including those described above is inserted into a subject, such as a predetermined high-risk subject, prior to disease onset. The implant is then used for early detection of metastatic potential or disease. In some examples, implantation occurs following therapeutic intervention and is used for monitoring therapeutic efficacy.

At the time point of interest, e.g., after a given number of days or after a therapeutic intervention, a biopsy is performed to remove the scaffold, either surgically removal of the entire scaffold or removal of only a portion thereof, such as performing a core-needle biopsy of the scaffold microenvironment.

With the scaffold removed or a scaffold microenvironment removed, the scaffold contents are processed for molecular content determination. In some examples, such processing includes isolate RNA and/or protein from scaffold biopsy (e.g., homogenize in Trizol reagent or detergent, respectively). Optionally, in some examples, fractionate cell populations within the scaffold for cell type specific analysis may be performed. While cell population analysis techniques will be understood, in some examples, separate cell populations based on fluorescent-assisted cell sorting or magnetic-assisted cell sorting may be performed. Analysis of gene or protein expression may be achieved, in some examples, using either qRT-PCR or RNAseq, for gene expression, and either ELISA or Luminex (bead-based multiplex assays), for protein expression. The result from this initial processing stage is measured expression level data that is provided to a processing system.

At a next stage, measured expression level data is pre-processed in the processing system. In exemplary embodiments, the processing system analyzes the measured expression level data and performs an alignment of various analytes into a format for either signature development or test sample characterization. The alignment may be in the form of a matrix with or without categorical indicators, for example. An example output of fold change from a significance from a compiled matrix is shown in Figure 5.

The processing system may include numerous pre-processing data analysis and correction processes. For example, compensation for missing data points may be used depending upon the computational algorithm chosen to perform the alignment. Furthermore, removal of an entire analyte may be used, if too many data points are missing or a nearest neighbor average may be employed, if cohort size is suitable.

For gene expression, the processing system may perform gene stability analysis and normalization. For example, a panel of genes may be compared against each other for stability within the set then a subset may be determined for sample normalization.

For protein expression, the processing system may normalize total sample protein concentration.

Further pre-processing by the processing system may include, analyzing fold change, significance, and false-detection rate of key variables for interpretation of single analyte dynamics.

The processing system, with the foregoing pre-processing, then identifies analytes (gene or protein expression) of interest, for example, via regularization or supervised discriminate analysis. Techniques that may be used include a Lasso or Elastic Net process and/or a Partial-Least Squares Discriminate Analysis (PLS-DA).

At a next stage, a reduction of the variables from the second stage is performed to determine a single metric, i.e., a compressed metric of the measured expression level data.

In exemplary embodiments, a process of decomposition matrix of gene expressions is performed and a gene expression score is attained. An example decomposition matrix is a singular value decomposition (SVD) process. In an example implementation, a single score was obtained by the processing system from the first left singular vector, which forms an orthonormal basis for the assay expression profiles. An examples decomposition matrix that may be used is a dynamic mode decomposition (DMD).

With a decomposition performed on the measured expression level data, the processing system may be further configured to employ a machine learning process to attain a prediction of disease state. An example, machine learning process is a Random forest (RF) generation or other ensemble/decision tree classification algorithm. In some examples, the machine learning process employs a logistic regression analysis. In some examples, the machine learning process employs a discriminant analysis (linear or quadratic). In some examples, the machine learning process employs a support vector machines analysis. In some examples, the machine learning process employs a nearest neighbor (fine-course, weighted) analysis.

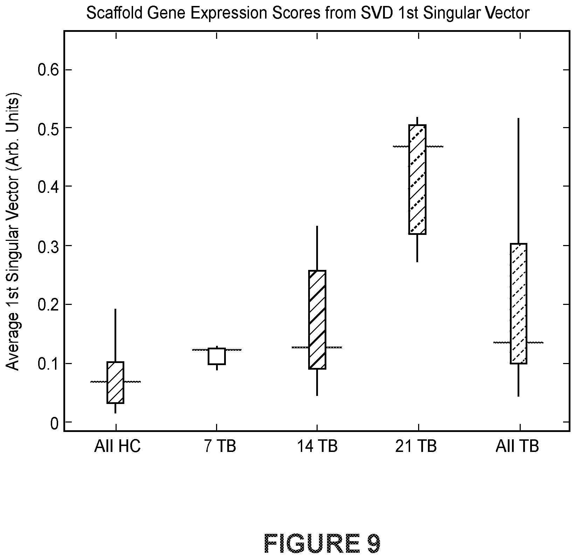

Figures 9-12 illustrate example outputs from this third stage.

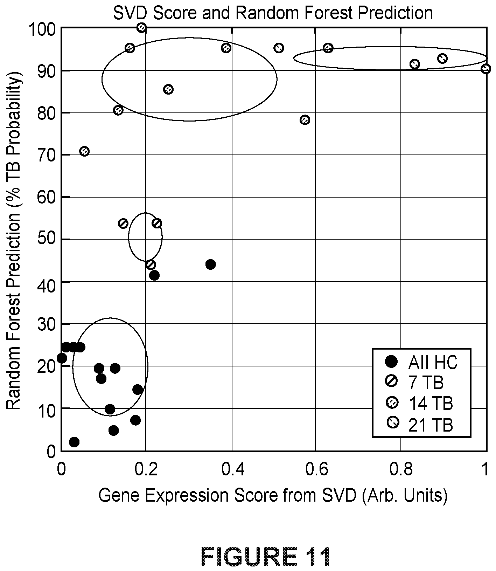

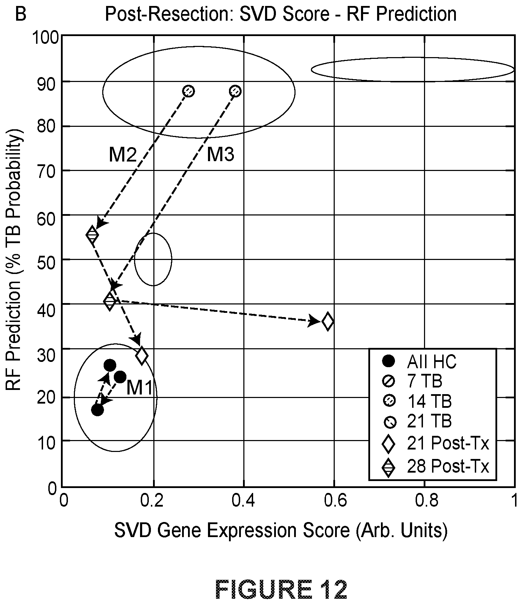

At a next stage, a combination is performed on multiple scores and a predicted metastatic potential is determined. In an exemplary embodiment, the SVD score and the Random Forest percentage prediction are combined to analyze the measured expression level data in a multidimensional process. For example, the SVD score and the Random Forest percentage prediction may be combined into an interrelation plot showing a multivariate analysis, such as a graphical output as in the example of Figures 11 and 12. In some examples, the two may be combined to form a numerical output. In either example, the multidimensional process may be used by the processing system to identify trends, correlations, and outliers and to assess pre metastasis. Figures 11 and 12 illustrate an example multivariate plot that may be generated and displayed by the processing system. The plot provides a visual indication of the outputs for correlation of the SVD and Random Forest values, including side by side box or bar plots for each algorithm or a combination of algorithms into a multidimensional output (e.g., scatter plot of SVD score and RF prediction).

In exemplary aspects, as described above, the measured levels of the genes, RNA, or proteins form a gene expression signature, an RNA signature, or a protein signature, respectively. In further aspects, the measurement of cell types in the sample form a cell signature. In exemplary instances, each of the signatures are processed into a single value.

### Systems, Computer-Readable Storage Media, and Methods Implemented by a Computer Processor

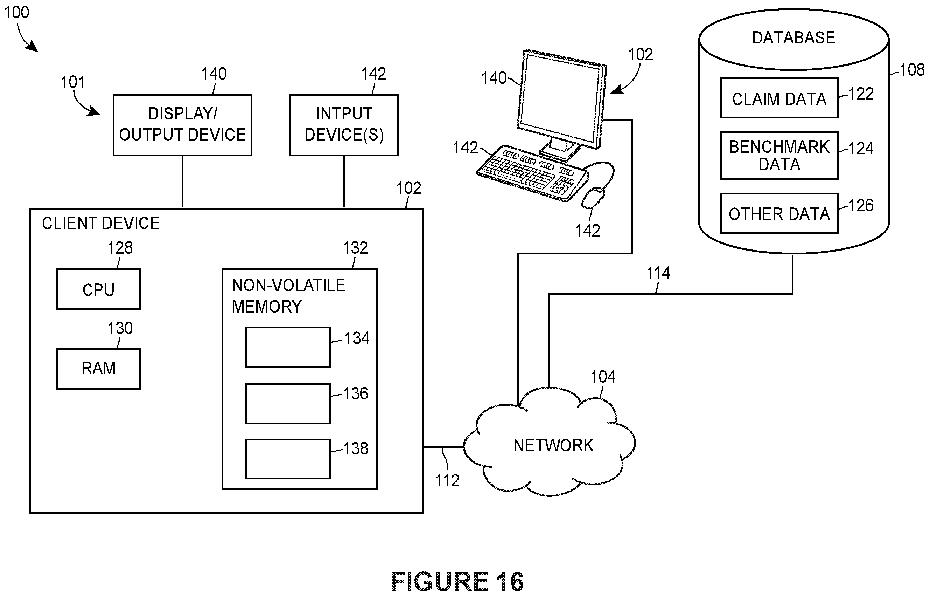

Figure 16 illustrates an exemplary embodiment 101 of a system 100 for assessing a subject's metastatic potential. Generally, the system 100 may include one or more client devices 102, a network 104, and a database 108. Each client device 102 may be communicatively coupled to the network 104 by one or more wired or wireless network connections 112, which may be, for example, a connection complying with a standard such as one of the IEEE 802.11 standards ("Wi-Fi"), the Ethernet standard, or any other appropriate network connection. Similarly, the database 108 may be communicatively coupled to the network 104 via one or more connections 114. (Of course, the database could alternatively be internal to one or more of the client devices 102.) The database 108 may store data related to the expression profiles for a variety of subjects, including, but not limited to, data of a sample obtained from a scaffold implanted in the subject, data of a sample obtained from a scaffold implanted in a control population, etc. In exemplary aspects, the control is a population of subjects known to not have a tumor or cancer. In exemplary aspects, the control is a population of subjects known to have a non-metastatic tumor or cancer. In exemplary aspects, the control is a population of subjects known to have metastatic cancer.

The data of the samples may be, for example, related to one or more of a level of expression of an S100A8 gene, RNA, or protein, a level of expression of an S100A9 gene, RNA, or protein, a level of expression of a gene, RNA, or protein of or encoded by one of: Ccl22, Cxcl2, Ccr7, Csf3, Bmp15, IL-23a, S100A9, Pglryp1, S100A8, Ltf, Ela2, and Camp, etc., as described in greater detail below. The data of the samples may be, for example, related to one or more of a level of expression of an S100A8 gene, RNA, or protein, a level of expression of an S100A9 gene, RNA, or protein, a level of expression of a gene, RNA, or protein of or encoded by one of: Ccl22, Ccr7, Bmp15, S100A9, Pglryp1, S100A8, Ltf, Ela2, and Camp.

As will be understood, the network 104 may be a local area network (LAN) or a wide-area network (WAN). That is, network 104 may include only local (e.g., intra-organization) connections or, alternatively, the network 104 may include connections extending beyond the organization and onto one or more public networks (e.g., the Internet). In some embodiments, for example, the client device 102 and the database 108 may be within the network operated by a single company (Company A). In other embodiments, for example, the client device(s) 102 may be on a network operated by Company A, while the database 108 may be on a network operated by a second company (Company B), and the networks of Company A and Company B may be coupled by a third network such as, for example, the Internet.

Referring still to Figure 16, the client device 102 includes a processor 128 (CPU), a RAM 130, and a non-volatile memory 132. The non-volatile memory 132 may be any appropriate memory device including, by way of example and not limitation, a magnetic disk (e.g., a hard disk drive), a solid state drive (e.g., a flash memory), etc. Additionally, it will be understood that, at least with regard to Figure 16, the database 108 need not be separate from the client device 102. Instead, in some embodiments, the database 108 is part of the non-volatile memory 132 and the data 122, 124, 126 may be stored as data within the memory 132. For example, the data 122 may be included as data in a spreadsheet file stored in the memory 132, instead of as data in the database 108. In addition to storing the records of the database 108 (in some embodiments), the memory 132 stores program data and other data necessary to analyze data of one or more sample and/or control populations, etc. For example, in an embodiment, the memory 132 stores a first routine 134, a second routine 136, and a third routine 138. The first routine 134 may receive data values related to a measured expression level of a gene, RNA, or protein of a sample obtained from a scaffold implanted in a test subject, and may process the data values received by the routine 134 through an algorithm to obtain a score. The second routine 136 may computer one or more statistical parameters of the data collected by the first routine 134, such as determining a mean value, a standard deviation value, etc. Additionally and/or alternatively, the second routine 136 may plot a score on a graphical or numerical output. Regardless, each of the routines is executable by the processor 128 and comprises a series of compiled or compilable machine-readable instructions stored in the memory 132. Additionally, the memory 132 may store generated reports or records of data output by one of the routines 134 or 136. Alternatively, the reports or records may be output to the database 108. One or more display/output devices 140 (e.g., printer, display, etc.) and one or more input devices 142 (e.g., mouse, keyboard, tablet, touch-sensitive interface, etc.) may also be coupled to the client device 102, as is generally known.

As will be understood, although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the present disclosure.

For example, the network 104 may include but is not limited to any combination of a LAN, a MAN, a WAN, a mobile, a wired or wireless network, a private network, or a virtual private network. Moreover, while only two clients 102 are illustrated in Figure 16 to simplify and clarify the description, it is understood that any number of client computers are supported and can be in communication with one or more servers (not shown).

Additionally, certain embodiments are described herein as including logic or a number of routines. Routines may constitute either software routines (e.g., code embodied on a machine-readable medium or in a transmission signal) or hardware routines. A hardware routine is tangible unit capable of performing certain operations and may be configured or arranged in a certain manner. In example embodiments, one or more computer systems (e.g., a standalone, client or server computer system) or one or more hardware routines of a computer system (e.g., a processor or a group of processors) may be configured by software (e.g., an application or application portion) as a hardware routine that operates to perform certain operations as described herein.

Similarly, the methods or routines described herein may be at least partially processor-implemented. For example, at least some of the operations of a method may be performed by one or processors or processor-implemented hardware modules. The performance of certain of the operations may be distributed among the one or more processors, not only residing within a single machine, but deployed across a number of machines. In some example embodiments, the processor or processors may be located in a single location (e.g., within a home environment, an office environment or as a server farm), while in other embodiments the processors may be distributed across a number of locations.

The performance of certain of the operations may be distributed among the one or more processors, not only residing within a single machine, but deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations.

Some embodiments may be described using the expression "coupled" and "connected" along with their derivatives. For example, some embodiments may be described using the term "coupled" to indicate that two or more elements are in direct physical or electrical contact. The term "coupled," however, may also mean that two or more elements are not in direct contact with each other, but yet still co-operate or interact with each other. The embodiments are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description should be read to include one or at least one and the singular also includes the plural unless it is obvious that it is meant otherwise.

Still further, the figures depict preferred embodiments of a map editor system for purposes of illustration only. One skilled in the art will readily recognize from the following discussion that alternative embodiments of the structures and methods illustrated herein may be employed without departing from the principles described herein

Upon reading this disclosure, those of skill in the art will appreciate still additional alternative structural and functional designs for a system and a process for identifying terminal road segments through the disclosed principles herein. Thus, while particular embodiments and applications have been illustrated and described, it is to be understood that the disclosed embodiments are not limited to the precise construction and components disclosed herein. Various modifications, changes and variations, which will be apparent to those skilled in the art, may be made in the arrangement, operation and details of the method and apparatus disclosed herein.

The present disclosure provides systems not part of the claimed invention, comprising: a processor; a memory device
coupled to the processor, and machine readable instructions stored on the memory device. In exemplary embodiments, the machine readable instructions that, when executed by the processor, cause the processor to
(i) receive a plurality of data values, each data value is a measured expression level of a different gene of a sample obtained from a synthetically-engineered pMN implanted in a test subject;
(ii) process the plurality of data values though (a) a decomposition algorithm to obtain a single score for gene expression for the test subject, (b) a machine learning algorithm to obtain a score of prediction of disease state for the test subject, or (c) a combination of (a) and (b) to obtain a combined score for the test subject;
(iii) plot the single score for gene expression for the test subject, the score of prediction of disease state for the test subject, or the combined score for the test subject on a graphical or numerical output, wherein the graphical or numerical output comprises an average single score for gene expression for a control, an average score of prediction of disease state for a control, or an average combined score for a control.

In exemplary aspects, the control is a population of subjects known to not have a tumor or cancer. In exemplary aspects, the control is a population of subjects known to have a non-metastatic tumor or cancer. In exemplary aspects, the control is a population of subjects known to have metastatic cancer. In exemplary aspects, the graphical or numerical output comprises the average score (single score for gene expression, score of prediction of disease state, or combined score) for multiple controls. In exemplary aspects, the graphical or numerical output comprises the average score (single score for gene expression, score of prediction of disease state, or combined score) for subjects known to have a non-metastatic tumor or cancer, the average score for subjects known to not have a tumor or cancer, and the average score for subjects known to have metastatic cancer.

In alternative or additional aspects, the system of the disclosure comprises machine readable instructions that, when executed by the processor, cause the processor to:
(i) receive a plurality of data values, each data value is a measured expression level of a different RNA of a sample obtained from a synthetically-engineered pMN implanted in a test subject;
(ii) process the plurality of data values though (a) a decomposition algorithm to obtain a single score for RNA expression for the test subject, (b) a machine learning algorithm to obtain a score of prediction of disease state for the test subject, or (c) a combination of (a) and (b) to obtain a combined score for the test subject;
(iii) plot the single score for RNA expression for the test subject, the score of prediction of disease state for the test subject, or the combined score for the test subject on a graphical or numerical output, wherein the graphical or numerical output comprises an average single score for RNA expression for a control, an average score of prediction of disease state for a control, or an average combined score for a control.

In exemplary aspects, the control is a population of subjects known to not have a tumor or cancer. In exemplary aspects, the control is a population of subjects known to have a non-metastatic tumor or cancer. In exemplary aspects, the control is a population of subjects known to have metastatic cancer. In exemplary aspects, the graphical or numerical output comprises the average score (single score for RNA expression, score of prediction of disease state, or combined score) for multiple controls. In exemplary aspects, the graphical or numerical output comprises the average score (single score for RNA expression, score of prediction of disease state, or combined score) for subjects known to have a non-metastatic tumor or cancer, the average score for subjects known to not have a tumor or cancer, and the average score for subjects known to have metastatic cancer.

In yet further alternative or additional aspects, the system of the disclosure, comprises machine readable instructions that, when executed by the processor, cause the processor to:
(i) receive a plurality of data values, each data value is a measured expression level of a different protein of a sample obtained from a synthetically-engineered pMN implanted in a test subject;
(ii) process the plurality of data values though (a) a decomposition algorithm to obtain a single score for protein expression for the test subject, (b) a machine learning algorithm to obtain a score of prediction of disease state for the test subject, or (c) a combination of (a) and (b) to obtain a combined score for the test subject;
(iii) plot the single score for protein expression for the test subject, the score of prediction of disease state for the test subject, or the combined score for the test subject on a graphical or numerical output, wherein the graphical or numerical output comprises an average single score for protein expression for a control, an average score of prediction of disease state for a control, or an average combined score for a control.

In exemplary aspects, the control is a population of subjects known to not have a tumor or cancer. In exemplary aspects, the control is a population of subjects known to have a non-metastatic tumor or cancer. In exemplary aspects, the control is a population of subjects known to have metastatic cancer. In exemplary aspects, the graphical or numerical output comprises the average score (single score for protein expression, score of prediction of disease state, or combined score) for multiple controls. In exemplary aspects, the graphical or numerical output comprises the average score (single score for protein expression, score of prediction of disease state, or combined score) for subjects known to have a non-metastatic tumor or cancer, the average score for subjects known to not have a tumor or cancer, and the average score for subjects known to have metastatic cancer.

Also provided herein are computer-readable storage media not part of the claimed invention, having stored thereon machine-readable instructions executable by a processor. In exemplary embodiments, the instructions comprise:
(i) instructions for receiving a plurality of data values, each data value is a measured expression level of a different gene of a sample obtained from a synthetically-engineered pMN implanted in a test subject
(ii) instructions for processing the plurality of data values though (a) a decomposition algorithm to obtain a single score for gene expression for the test subject, (b) a machine learning algorithm to obtain a score of prediction of disease state for the test subject, or (c) a combination of (a) and (b) to obtain a combined score for the test subject;
(iii) instructions for plotting the single score for gene expression for the test subject, the score of prediction of disease state for the test subject, or the combined score for the test subject on a graphical or numerical output, wherein the graphical or numerical output comprises an average single score for gene expression for a control, an average score of prediction of disease state for a control, or an average combined score for a control.

In alternative or additional embodiments, the instructions comprise:
(i) instructions for receiving a plurality of data values, each data value is a measured expression level of a different RNA of a sample obtained from a synthetically-engineered pMN implanted in a test subject
(ii) instructions for processing the plurality of data values though (a) a decomposition algorithm to obtain a single score for RNA expression for the test subject, (b) a machine learning algorithm to obtain a score of prediction of disease state for the test subject, or (c) a combination of (a) and (b) to obtain a combined score for the test subject;
(iii) instructions for plotting the single score for RNA expression for the test subject, the score of prediction of disease state for the test subject, or the combined score for the test subject on a graphical or numerical output, wherein the graphical or numerical output comprises an average single score for RNA expression for a control, an average score of prediction of disease state for a control, or an average combined score for a control.

In yet further alternative or additional embodiments, the instructions comprise:
(i) instructions for receiving a plurality of data values, each data value is a measured expression level of a different protein of a sample obtained from a synthetically-engineered pMN implanted in a test subject
(ii) instructions for processing the plurality of data values though (a) a decomposition algorithm to obtain a single score for protein expression for the test subject, (b) a machine learning algorithm to obtain a score of prediction of disease state for the test subject, or (c) a combination of (a) and (b) to obtain a combined score for the test subject;
(iii) instructions for plotting the single score for protein expression for the test subject, the score of prediction of disease state for the test subject, or the combined score for the test subject on a graphical or numerical output, wherein the graphical or numerical output comprises an average single score for protein expression for a control, an average score of prediction of disease state for a control, or an average combined score for a control.

The controls may be as those described above.

Further provided herein are methods not part of the claimed invention, implemented by a processor in a computer. In exemplary embodiments, the method comprises the steps of:
(i) receiving a plurality of data values, each data value is a measured expression level of a different gene of a sample obtained from a synthetically-engineered pMN implanted in a test subject;
(ii) processing the plurality of data values though (a) a decomposition algorithm to obtain a single score for gene expression for the test subject, (b) a machine learning algorithm to obtain a score of prediction of disease state for the test subject, or (c) a combination of (a) and (b) to obtain a combined score for the test subject;
(iii) plotting the single score for gene expression for the test subject, the score of prediction of disease state for the test subject, or the combined score for the test subject on a graphical or numerical output, wherein the graphical or numerical output comprises an average single score for gene expression for a control, an average score of prediction of disease state for a control, or an average combined score for a control.

In alternative or exemplary embodiments, the method comprises the steps of:
(i) receiving a plurality of data values, each data value is a measured expression level of a different RNA of a sample obtained from a synthetically-engineered pMN implanted in a test subject;
(ii) processing the plurality of data values though (a) a decomposition algorithm to obtain a single score for RNA expression for the test subject, (b) a machine learning algorithm to obtain a score of prediction of disease state for the test subject, or (c) a combination of (a) and (b) to obtain a combined score for the test subject;
(iii) plotting the single score for RNA expression for the test subject, the score of prediction of disease state for the test subject, or the combined score for the test subject on a graphical or numerical output, wherein the graphical or numerical output comprises an average single score for RNA expression for a control, an average score of prediction of disease state for a control, or an average combined score for a control.

In yet further alternative or exemplary aspects, the method comprises the steps of:
(i) receiving a plurality of data values, each data value is a measured expression level of a different protein of a sample obtained from a synthetically-engineered pMN implanted in a test subject;
(ii) processing the plurality of data values though (a) a decomposition algorithm to obtain a single score for protein expression for the test subject, (b) a machine learning algorithm to obtain a score of prediction of disease state for the test subject, or (c) a combination of (a) and (b) to obtain a combined score for the test subject;
(iii) plotting the single score for protein expression for the test subject, the score of prediction of disease state for the test subject, or the combined score for the test subject on a graphical or numerical output, wherein the graphical or numerical output comprises an average single score for protein expression for a control, an average score of prediction of disease state for a control, or an average combined score for a control.

The controls may be as those described above.

### Anti-Cancer Therapy and Treatments

Therapy and treatments for cancer, e.g., metastatic cancer, are known in the art. In exemplary aspects, the anti-cancer therapy or treatment comprises administration of one or more chemotherapeutic agents.

Chemotherapeutic agents are known in the art and include, but not limited to, platinum coordination compounds, topoisomerase inhibitors, antibiotics, antimitotic alkaloids and difluoronucleosides, as described in U.S. Pat. No. 6,630,124.

In some embodiments, the chemotherapeutic agent is a platinum coordination compound. The term "platinum coordination compound" refers to any tumor cell growth inhibiting platinum coordination compound that provides the platinum in the form of an ion. In some embodiments, cisplatin is the platinum coordination compound employed in the compositions and methods of the present disclosure. In some embodiments, the chemotherapeutic agent is a topoisomerase inhibitor. In some aspects, the topoisomerase inhibitor is camptothecin or a camptothecin analog. In still yet other embodiments of the present disclosure, the chemotherapeutic agent is an antibiotic compound. Suitable antibiotic include, but are not limited to, doxorubicin, mitomycin, bleomycin, daunorubicin and streptozocin. In some embodiments, the chemotherapeutic agent is an antimitotic alkaloid. In general, antimitotic alkaloids can be extracted from Cantharanthus roseus, and have been shown to be efficacious as anticancer chemotherapy agents. In other embodiments of the present disclosure, the chemotherapeutic agent is a difluoronucleoside. 2'-deoxy-2',2'-difluoronucleosides are known in the art as having antiviral activity. Such compounds are disclosed and taught in U.S. Pat. Nos. 4,526,988 and 4,808614. European Patent Application Publication 184,365 discloses that these same difluoronucleosides have oncolytic activity.

More than a dozen monoclonal antibodies are currently approved by the U.S. Food and Drug Administration to treat cancers. Among these agents are alemtuzumab (Campath^{®}), which is indicated for chronic lymphocytic leukemia (CLL), and trastuzumab (Herceptin^{®}), which is used for treating breast cancer. Some antibodies are labeled with chemotherapeutic drugs, including, for example, brentuximab vedotin (Adcetris^{®}) and Ado-trastuzumab emtansine (Kadcyla^{®}). Others are described in Guo et al., Clin Oncol Cancer Res (2011) 8: 215-219. Andrew Simpson and Otavia Caballero, BMC Proceedings Monoclonal antibodies for the therapy of cancer, October 2014, 8:O6.

In exemplary embodiments, the present disclosure provides a method which is not part of the claimed invention, of determining a subject's metastatic potential, determining a tumor's or cancer's metastatic stage, or detecting metastatic disease, or a predisposition thereto, in a subject in need thereof, comprising measuring a level of expression of a gene, an RNA or a protein, in a sample obtained from an engineered pre-metastatic niche (pMN) implanted in the subject, wherein the measured expression level of the gene, RNA or protein in the sample is compared to a control level. In exemplary aspects, the method comprises measuring the expression level of at least two genes, RNA, or proteins in the sample, optionally, comprising measuring the expression level of a plurality of genes, RNA, or proteins in the sample, wherein the measured expression levels are compared to control levels. In certain instances, the control levels of the genes, RNA, or proteins form a control pMN expression signature indicative of no metastatic disease. In various aspects, the control pMN expression signature indicative of no metastatic disease is processed through a decomposition algorithm (e.g., a singular value decomposition) to obtain a single score for gene expression and/or a machine learning algorithm (e.g., a random forest generation) to obtain a score of prediction of disease state. In various instances, the control pMN expression signature is processed through a decomposition algorithm to obtain a single control score for gene expression and a machine learning algorithm to obtain a control score of prediction of disease state, and, optionally, the single control score for gene expression and the control score of prediction of disease state are combined to provide a combined control score of no metastatic disease. In various aspects, the combined score of metastatic potential is compared to the combined control score of no metastatic disease to determine the subject's metastatic potential. In various aspects, when the combined score of metastatic potential is low, relative to the combined control score, the subject's metastatic potential is low and/or the subject is deemed healthy, when the combined score of metastatic potential is intermediate, relative to the combined control score, the subject's metastatic potential indicates an early stage of metastatic disease, and when the combined score of metastatic potential is high, relative to the combined control score, the subject's metastatic potential indicates metastatic disease. The presently disclosed method of determining a subject's metastatic potential, determining a tumor's or cancer's metastatic stage, or detecting metastatic disease, or a predisposition thereto, in a subject in need thereof, enables the determination of the appropriate treatment for a subject with a tumor or cancer and/or the determination of a subject's need for metastatic disease therapy. Accordingly, the present disclosure provides methods of determining treatment for a subject with a tumor or cancer and/or determining a subject's need for metastatic disease therapy, comprising determining a subject's metastatic potential, determining a tumor's or cancer's metastatic stage, or detecting metastatic disease, or a predisposition thereto, as described herein. In various aspects, when the combined score of metastatic potential is intermediate, relative to the combined control score, the subject's metastatic potential indicates an early stage of metastatic disease, and the subject is determined as needing a therapy that targets the immune cells at the metastatic niche, such as phosphodiesterase 5 (PDE-5) or COX-2 inhibitors that inhibit the functionality of myeloid derived suppressor cells.

The present disclosure further provides methods which are not part of the claimed invention, of treating metastatic disease or delaying the onset of metastatic disease, comprising determining a subject's need for metastatic disease therapy, as described herein, and providing the metastatic disease therapy based on the subject's metastatic potential. In various aspects, the present disclosure provides a method of treating a subject, comprising administering to the subject an anti-metastatic disease treatment, optionally, wherein the anti-metastatic disease treatment comprises a PARP inhibitor or Gemcitabine, when the subject exhibits a high combined score of metastatic potential, relative to the combined control score, or administering to the subject a therapy that targets the immune cells at the metastatic niche, such as phosphodiesterase 5 (PDE-5) or COX-2 inhibitors that inhibit the functionality of myeloid derived suppressor cells, when the subject exhibits an intermediate combined score of metastatic potential, relative to the combined control score.

The references to methods of treatment in paragraph 187 of this description are to be interpreted as references to the compounds, pharmaceutical compositions and medicaments for use in a method for treatment of the human (or animal) body by therapy.

The following examples are given merely to illustrate the present invention and not in any way to limit its scope.

### EXAMPLES

### EXAMPLE 1

The following example demonstrates that gene expression profiles of cells caught in synthetic engineered pre-metastatic niche (pMN) scaffolds change over time after tumorigenesis and that the gene expression profiles of tumor-bearing animals differ from those of tumor-free animals.

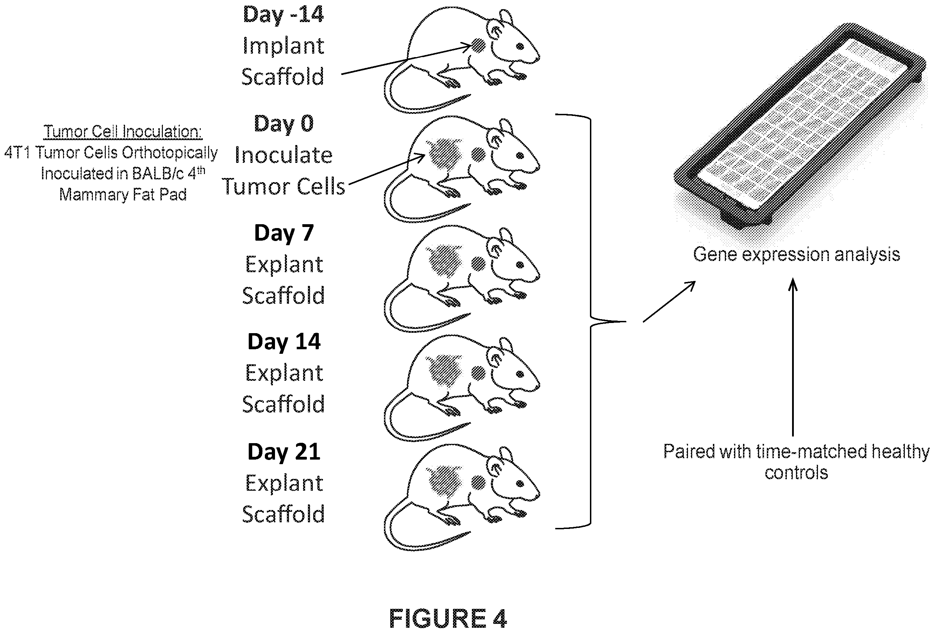

A polymer scaffold comprising polycaprolactone (PCL) was implanted into the subcutaneous space of two groups of BALB/c mice. Fourteen days after scaffold implantation, 4T1 tumor cells (2E6 cells suspended in 50µL of PBS) were orthotopically inoculated into the 4^{th} mammary fat pad of one group of mice. In the other group of mice, PCL scaffolds were implanted in the healthy mice but no inoculation was performed. This healthy, or control cohort, simulates the acquisition of an average gene expression from healthy patients. At Days 7, 14, and 21 following tumor cell inoculation, an explant was taken from the scaffold of each animal (of both the tumor-bearing and healthy control cohort) either through surgical resection or via use of a core needle biopsy. Figure 4 shows an experimental outline of the above described steps.

To derive genomic content from the implanted scaffold, the explanted cells and polymer scaffold were homogenized with a mechanical dispersing instrument in Trizol reagent. RNA isolation was carried out via centrifugation filter kits that include a DNA enzymatic degradation. Quality and quantity of the RNA was assessed via a Bioanalyzer 2100 and Nanodrop 2000c, respectively. RNA was synthesized into cDNA via reverse transcription, following suggested preparation protocols for high-throughput qRT-PCR using OpenArray panels.

High-throughput qRT-PCR was carried out using a TaqMan^{®} OpenArray^{®} Mouse Inflammation Panel on a QuantStudio^{™} 12K Flex. The OpenArray panel analyzes the expression of 635 genes that are associated with immunological pathways. Instrument parameters and quality control was managed by the University of Michigan DNA Sequencing Core. Additionally, single tube qRT-PCR was carried out on a subset of genes (determined from the OpenArray results) using Taqman probes to validate the results found using the high-throughput results as well as expand our tests. Gene expressions were normalized to a combination of 5 different housekeeping genes as determined by a house gene stability analysis of the 16 housekeeping genes included in the OpenArray panel.

Gene expression data were processed to generate a variety of graphical outputs. A volcano plot (a compilation of log-transform fold changes (x-axis) and log-transformed p-values (y-axis)) was generated to compare tumor-bearing cohorts and time-matched healthy controls. As shown in Figure 5, the volcano plot illustrated the magnitude and significance of gene changes associated with metastatic conditioning by the primary tumor. From this plot, the dynamic nature of gene expression was evident.

Heatmaps generated through partial least squares discriminate analysis (PLS-DA), a supervised discriminate analysis algorithm, aided in the selection of features of most importance. Additionally, it clustered the samples based on selected features and conveyed the strength of relationships between different samples. Exemplary heat maps from explants obtained on Day 7 and Day 21 are shown in Figure 6

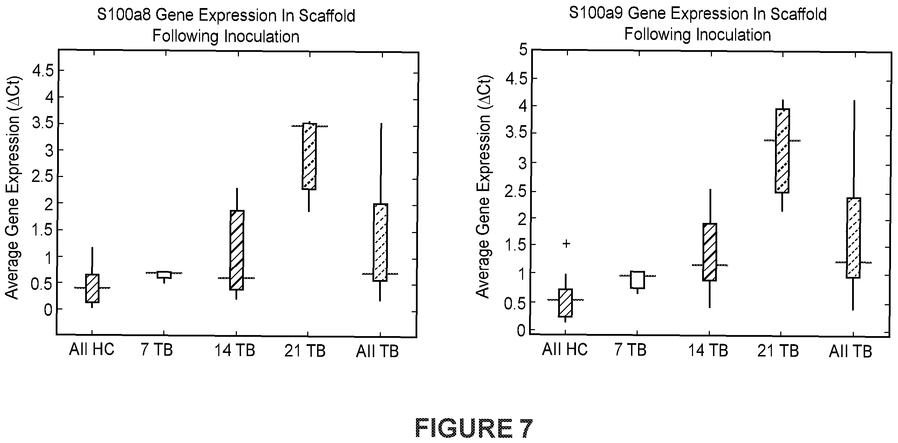

Box plots illustrated the progression of changes in the gene expression level of particular genes during metastatic conditioning. S1 00a8 and S100a9 have been indicated as fundamental to conditioning of the pMN and a microenvironment cytotoxic to T cells. The expression level of these two genes progressively changed between days 7, 14, and 21 (Figure 7) during onset of metastatic disease. All healthy controls are grouped in the first column, time-point specific expression is indicated in columns 2-4 for days 7-14-21, all tumor-bearing controls are grouped in the last column.

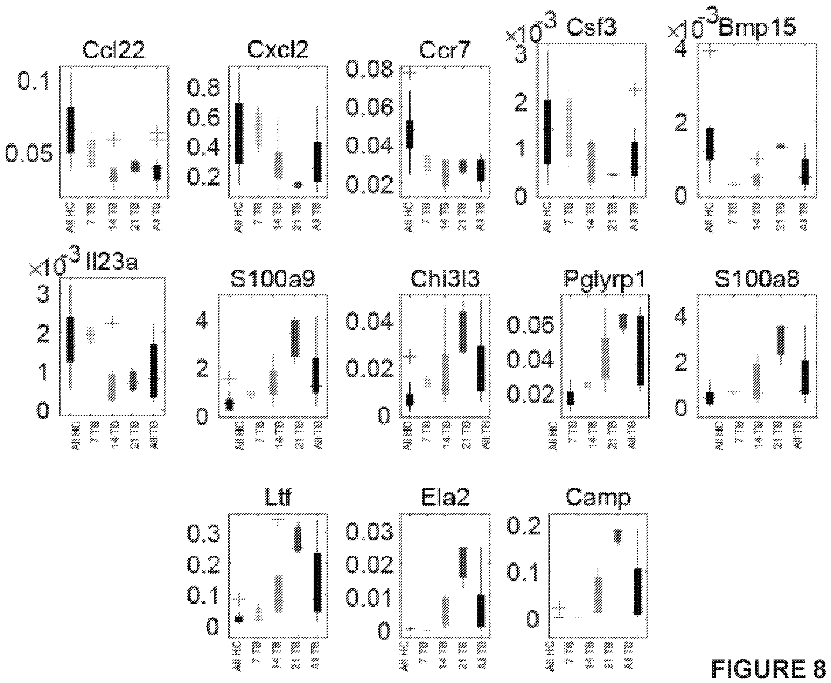

Similar to S1 00a8/a9, several other genes showed progressive alterations (increasing or decreasing) in expression over time as compared to time-matched healthy controls, with several of the upregulated being related to MDSC phenotype. These alterations are exemplified by the box plots shown in Figure 8.

### EXAMPLE 2

This example demonstrates an exemplary method of processing of gene expression profiles from cells in the synthetic pMN into a single diagnostic metric.

Following isolation of RNA and analysis of gene expression (as outlined in example 1), a multitude of alterations in the gene expression were combined into a single diagnostic metric through multiple dimensionality reduction and machine learning techniques. Results of these techniques are detailed below:

First, a method for reducing dimensionality of the data is the use of singular value decomposition (SVD) and extraction of the first left singular vector (an eigenvector) which provides a metric of the cumulative gene expression for each sample. This method serves to provide an unsupervised score for diagnosis of metastatic potential. The output of the SVD provides a score for each sample, when normalized the magnitude of the scores that were lowest are more closely associated with healthy mice on average, and the scores that are highest are more closely associated with tumor-bearing mice, with a progressive increase in score between days 7, 14, and 21. For illustration the averages for each cohort are shown in a box plot. Figure 9. Thus, from this single metric, one could infer metastatic potential. Other methods that fill this role of dimensionality reduction for developing a pMN signature includes linear combination of select factors, dynamic mode decomposition (DMD), principle component analysis (PCA), or Fisher's linear discriminate.

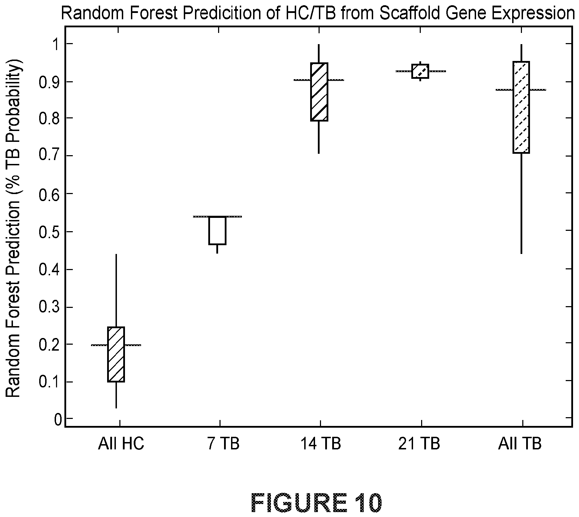

Second, a supervised, machine learning method for providing a prediction of metastatic potential (i.e. low or high likelihood that distal sites are primed for metastatic colonization) is the generation of random forest (RF) ensembles. This method aggregates a series of decision trees to provide a classification prediction. Probability of this prediction can be determined through cross validation. The output of the RF provides a probability for each sample, with the probabilities below 50% being predicted to be healthy mice on average, and the probabilities that are above 50% being more closely associated with tumor-bearing mice, with a progressive increase in probabilities between days 7, 14, and 21. For illustration the averages for each cohort are shown in a box plot. Figure 10. Thus, from this single metric, one could infer metastatic potential. Logistic regression, partial least squares discriminate analysis, discriminate analysis (linear or quadratic), neural networks pattern recognition, support vector machines, nearest neighbor, and Bayesian networks.

Last, multiple metrics can be combined into either a numerical or visual output to convey a multivariate signature of the metastatic potential. In the Figure 11, each sample from a healthy or tumor bearing mouse is plotted in the x-y plane. The x-axis correlates with SVD score and the y-axis correlates with the RF prediction. Average and first standard deviation of the SVD score and RF prediction for each cohort (all healthy control, days 7, 14, and 21 tumor bearing) are indicated by the larger faded ellipsoids in the background of the x-y plane. One can infer from the plot the clustering of the healthy cohorts, as well as the progressive increase in the multivariate signature of the tumor-bearing cohorts. It could additionally be inferred that any sample with a score/prediction outside of the healthy cohort average and standard deviation may warrant concern. Note that in general there is good agreement between the two metrics as there are no high scores with a low prediction, and few low scores with a high prediction. This demonstrates the benefit of combining multiple analytical approaches into a coherent output for diagnosis.

### EXAMPLE 3

This example demonstrates that the scaffold gene expression profiles and multivariate signature of cells caught in synthetic engineered pMN scaffolds change over time after treatment (surgical tumor resection) and that the gene expression profiles and multivariate signature of treated animals who remain healthy, differ from those who succumb to metastatic recurrence.

In the example, mice were implanted with scaffolds (as in Example 1), and a cohort of animals was inoculated with tumor cells (as in Example 1). One addition was that a series of 8 scaffolds were implanted into the subcutaneous space, which allow for more biopsies per mouse and thus enable the ability to longitudinally track each animal. While this presents a deviation from or previous approaches, it not expected to dramatically alter the diagnostic potential of the synthetic pMN.
- At Day 14 (following tumor inoculation, as aligned with day 14 in Example 2), a single scaffold was explanted from each mouse. These scaffolds were processed for qRT-PCR, genes of interest were analyzed, and based on the algorithms in Example 2 they were assigned a score and prediction. The scaffold from a healthy mouse at Day 14 was located within the average and standard deviation for the healthy control multivariate signature (SVD-RF combination). The scaffolds from tumor-bearing mice were located within the average and standard deviation for the Day 14 tumor-bearing. Immediately following biopsy of the scaffolds, the tumors were resected from the tumor-bearing mice and the healthy mice was given a sham mammary fat pad resection to account for any surgically inducted alterations.
- At Day 21, another scaffold was explanted from the same mice and similarly processed for gene expression and multivariate signature. The signature following resection of the primary tumor decreases in both score and prediction. Without the surgical resection it would have been expected to increase, and demonstrated in Example 2. For the healthy control that received a sham surgical resection there was very little change in the signature, and it was still located within the average and standard deviation for healthy.
- At Day 28, another scaffold was explanted from the same mice and similarly processed from gene expression and multivariate signature. For one of the mice that originally had a tumor, the trend towards the healthy cohort continued. This mouse remained healthy and did not exhibit signs of metastatic disease. However, one of the mice showed a dramatic increase in the SVD score and the magnitude, or trajectory of its signature shift away from the healthy cohort. This mouse developed symptoms of metastatic disease and died at Day 34 due to lung and plural metastases. For the healthy control that received a sham surgical resection there was very little change in the signature, and it was still located within the average and standard deviation for healthy.

By plotting the results of each sample in the multivariate signature subspace, one can infer from the plots comparing SVD and RF the trajectory of metastatic potential for each mouse. For the healthy mouse (M1) there was little change in trajectory. For the mouse that underwent therapeutic resection of the primary tumor and remained healthy (M2), there was maintained decrease in the trajectory of its metastatic signature. For the mouse that underwent therapeutic resection of the primary tumor and then subsequently died due to metastatic disease (M3), there was an initial decrease in the trajectory of the metastatic potential, however, this trajectory was alter away from healthy. Figure 12

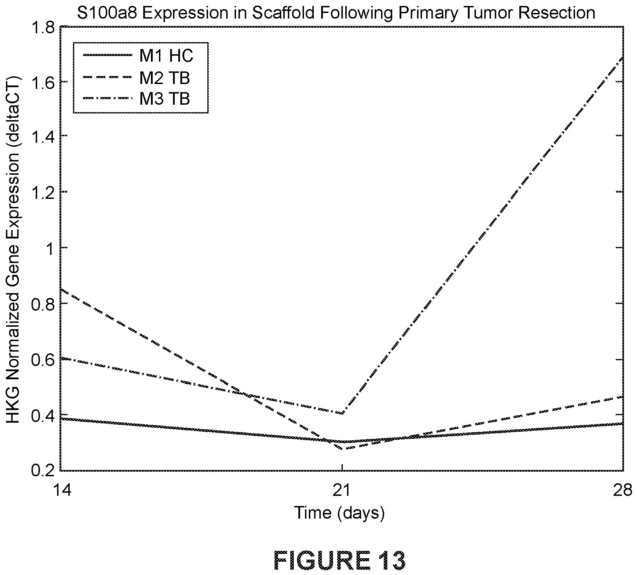

Analysis of individual gene expressions following resection of the primary tumor showed a divergence between the mouse that remained healthy and the mouse that developed metastatic disease. For example, analysis of S100a8 shows that a healthy mouse (M1) has little change in its gene expression over time. Conversely, S100a8 goes down in both tumor-bearing mice (M2 and M3) following tumor resection. M2 remains similar to the healthy control at Day 28, and indeed this mouse did not develop symptoms of metastatic disease. Expression of S100a8 in M3 deviates from both M1 and M2 at day 28, which preceded the symptomatic onset of metastatic disease at Day 34. Figure 13.

### EXAMPLE 4

This example demonstrates that the cytokine and chemokine expression profiles of immune cells caught in synthetic engineered pMN scaffolds change over time after tumorigenesis and that the cytokine and chemokine expression profiles of tumor-bearing animals differ from those of healthy, non-tumor-bearing animals.

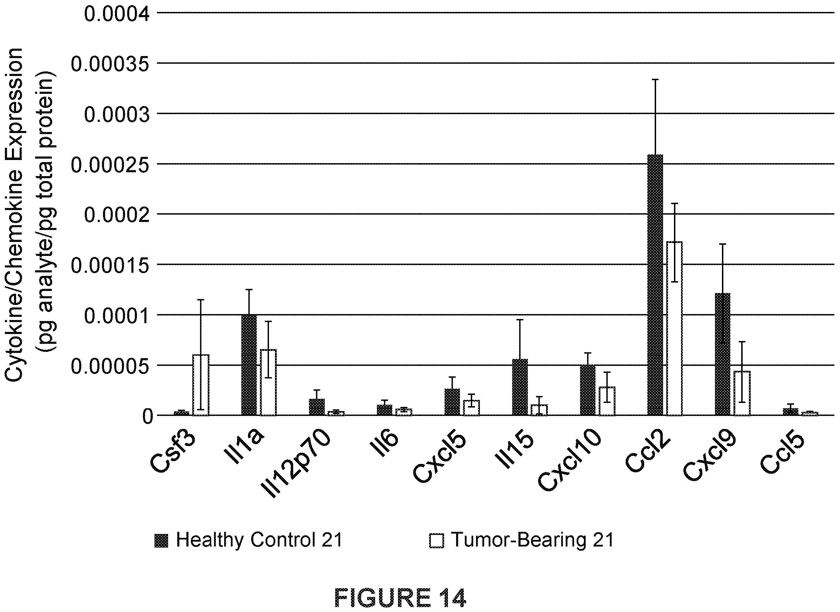

Another potential input into the synthetic pMN multivariate signature is protein expression. Of interest are cytokines and chemokines that may direct cell-cell signaling. To determine how the expression changes in tumor-bearing mice, compared to healthy mice we implanted PCL scaffold and inoculated tumor cells (As in Example 1). Then at Day 21 we isolated proteins from the scaffolds of tumor-bearing and healthy mice by homogenizing in PBS. Homogenized solutions were then sequentially centrifuged and filtered to remove debris. To analyze the cytokine expression levels, we employed a bead-based multiplexed analysis (Mouse Cytokine/ Chemokine Magnetic Bead Panel 32-plex) with analyzes 32 analytes per sample. Expression quantification was normalized to total protein concentration attained from a BCA assay. Several of the cytokines/chemokines showed a decrease in expression, which one (G-CSF) showed a dramatic upregulation in expression. Figure 14

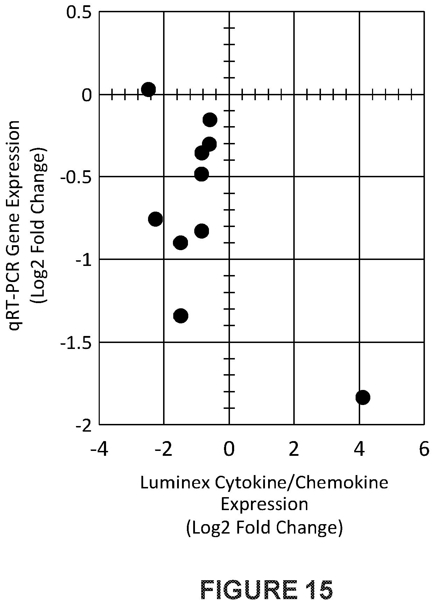

The drawing shows that many of the proteins that showed a decrease in expression also show a decrease in gene expression at Day 21 (as determined in Example 1). Figure 15

### EXAMPLE 5

This example demonstrates that synthetic diagnostic sites reflect disease progression and predict therapeutic response.

Engineering tissues *in vivo* that signal the status of host biology can extend regenerative medicine beyond the classical concepts of replacing lost or damaged tissues, towards creating engineered sites that serve as diagnostics. In metastatic breast cancer, the tissues to signify are those distal to the primary tumor that are in solid organs and recruit metastatic cells based on aberrant inflammatory processes.¹⁻⁴ Herein, it is demonstrated that the aberrant inflammatory processes of metastasis are captured in the chronic foreign body response mounted against biomaterial implants. The data herein show that the inflammation within the microporous polymer implants is progressively altered by disease, with inflammation analogous to that in diseased lung. Following therapy, longitudinal tracking of implants in individual mice differentiated resistance versus response to therapy. These results demonstrate the potential for implanted materials to detect context-specific pathological states that can be used for early detection of disease or therapeutic monitoring.

The concept of engineering a synthetic diagnostic site (SynDx) is predicated on generating a tissue that integrates with the host, whose properties will be modified based on the development of disease.^{5,6} Following implantation, a porous scaffold supports cell infiltration, vascularization, and a persistent inflammation as a consequence of the foreign body response (FBR)⁷. Herein, the inflammatory dynamics following the onset of disease are investigated as means to engineer a SynDx. It is hypothesized that the persistent inflammatory response at an implanted microporous polymer scaffold would acquire immunosuppressive trademarks in the context of metastatic cancer, thus mimicking those in disease-targeted organs.

In cancer metastasis and therapy, SynDx would facilitate next-generation diagnostics by signaling the condition and susceptibility of metastatic colonization in vital organs. The biological premise for creating a SynDx is based on evidence that circulating acellular material from the primary tumor alters discrete sites, termed pre-metastatic niche (preMN), in distal organs that precede and aid metastatic tumor cells (Fig. 22), thus making its occurrence diagnostically valuable.¹ Unfortunately, the longitudinal monitoring of a physiologic preMN is complex due to their indistinct and difficult to access localization within distant vital organs, such as the lung and liver. Data from SynDx would complement gene expression platforms designed for primary tumor samples that have driven the description of cancer subtypes, facilitated prediction of risk for distant recurrence, and empowered molecular signatures that guide decisions about systemic therapy for cancer patients.⁸ Unfortunately, the information obtained from the primary tumor is limited to a single measurement. Additionally, an understanding of distal tissue condition would complement liquid biopsies that face challenges in the relatively low numbers and phenotypic relevance as approximately 0.01% of circulating tumor cells will become metastatic foci.^{9,10} Collectively, SynDx that signal the condition metastatically-susceptible organs would evolve personalized patient care from primary tumor-based prognostics to routine monitoring of synthetic, predetermined, modular, and predictable diagnostic sites.

Studies from this research group and others have demonstrated that metastatic tumor cells colonize microporous polymer implants and focused on their early arrival as a metric of efficacy.¹¹⁻¹⁴ These implants (Figs. 17a, 23 & 24a) were composed of poly(ε-caprolactone) (PCL) polymer formed into a disc and designed with a microporous architecture (5mm diameter, 2mm thickness, interconnected 250-425µm pores) to support cellular ingrowth of stromal, immune, and epithelial cells that vastly outnumber the colonizing tumor cells. Additionally, persistent inflammation and the FBR at the microporous PCL scaffolds shows little change following two weeks after implantation in healthy mice.¹² For the studies herein, a microporous PCL scaffold was implanted into the easily accessible dorsal subcutaneous space of healthy BALB/c mice (Fig. 23) to allow tissue ingrowth for two weeks. Mice were then orthotopically inoculated at Day 0 with triple-negative 4T1 tumor cells (Fig. 24a).

A large-scale gene expression analysis of the tissue in microporous polymer implants was performed following primary tumor inoculation to characterize inflammatory dynamics. Weekly (Days 7, 14, and 21) changes in gene expression were screened via a high-throughput RT-qPCR platform, OpenArray^{™}, enabling parallel assessment of the expression of 632 target and 16 reference genes per sample (Fig. 24b). Altered expression was observed for 113 genes following tumor inoculation. A panel of genes of interest were defined based on their fold change, level of significance (false-discovery rate corrected), and expression stability. The 10 target genes (Fig. 17b-k, normalized to an average of 5 reference genes) included: S100 Calcium Binding Protein A8 (S100a8), S100 Calcium Binding Protein A9 (S100a9), Peptidoglycan Recognition Protein 1 (Pglyrp1), Lactotransferrin (Ltf), Cathelicidin Antimicrobial Peptide (Camp), Elastase 2 (Ela2), Chitinase (Chi3l3), Bone Morphogenetic Protein 15 (Bmp15), C-C Motif Chemokine Ligand 22 (Ccl22), C-C Motif Chemokine Receptor 7 (Ccr7). Unsupervised hierarchical clustering of these genes (Fig. 17k) based on Euclidean distance and average linkage produced a complete separation between healthy and diseased cohorts, with the gene expression in the tissue infiltrate of tumor-bearing mice progressing away from healthy controls as a function of time. This clustering identified distinct gene groups (i.e., S100a8 / S100a9 / Pglyrp1) that displayed similar expression profiles. Validation in C57BL/6 mice inoculated with a metastatic derivative of the E0771 line, denoted as Lu.2 (Fig. 25), showed similar gene expression changes and clustering, with 80% (8/10) of altered genes progressing in a similar direction (Fig. 26) as those in the BALB/c-4T1 model.

The changes in expression of specific genes within the implant-derived tissue suggest the adoption of an immunosuppressive and tumor cell hospitable environment, which is reflective of the natural preMN and supports its function as a SynDx. The increases in S100a8/9 are hallmarks of pre-metastatic and metastatic niche formation in metastasis-targeted organs (e.g., lungs) and myeloid-derived suppressor cell (MDSC) immunosuppressive functionality.⁴ S100a8/9 expression leads to T cell inhibitory/cytotoxic byproducts (e.g., reactive oxygen species) and tumor cell proliferation.^{15,16} Unlike the increase in S100a8/9, the increase in Pglyrp1 has not yet been associated with the preMN, though its correlation with S100a8/9 was previously associated with bone-marrow derived granulocytic MDSCs and Th17 to regulatory T (T_{REG}) cell transdifferentiation.^{17,18} Increases in Ela2, Camp, and Ltf were the greatest overall magnitude changes within this tissue at Day 21. Ltf and Camp are upregulated in granulocytic MDSCs as compared to neutrophils and increased levels of Ela2 and Camp have been identified as drivers of metastasis, with Ela2 associated with poor clinical outcomes and endocrine treatment failure.^{19,20} Chi3l3 expression progressively increased in the tissue infiltrate, which is supported by recent reports from chitinase knockout mice showing decreased metastasis.²¹ A decrease in Ccr7 would limit the chemoattraction of T cells, which is reflective of the decrease in CD8+ and CD4+ T cells in tissue at Days 14 and 21, respectively.^{12,22} Ccl22 expression can be upregulated in metastatic target organs and attracts T_{REGS} in oncogenesis, thus this decrease in expression during disease progression was unexpected.²³

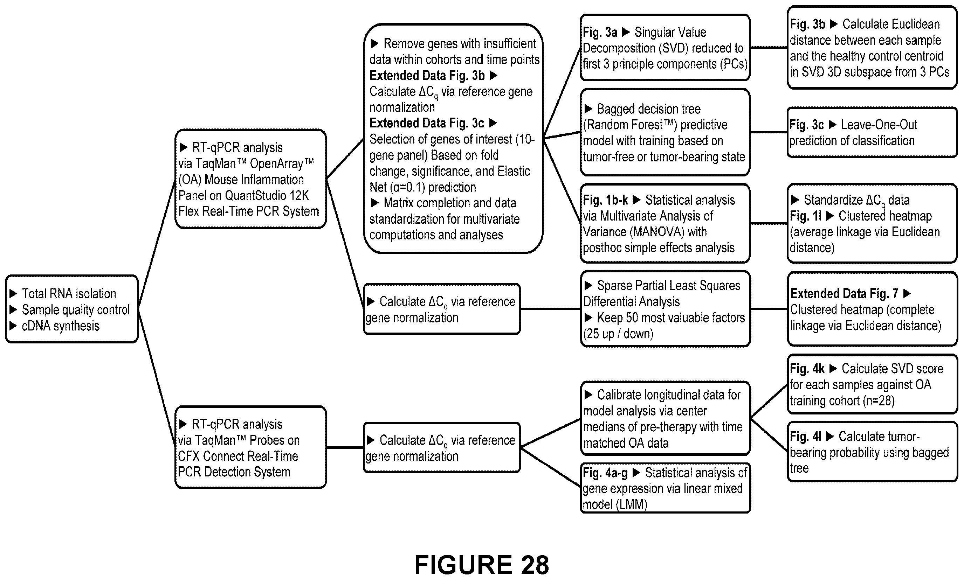

A powerful use of multi-gene panels in medicine is the computational reduction to single metric scores and predictions, often referred to as signatures, which have guided clinical management in breast cancer patients using primary tumor tissue assays.²⁴ The 10-gene panel selected from the OpenArray^{™} RT-qPCR analysis (Fig. 17) was reduced to a single metric and predictive model for the probability that a mouse was either tumor-bearing (TB) or a healthy control (HC). Three computational techniques were investigated in parallel based on the knowledge that a spectrum of regression and modeling approaches provides more robust interpretations of data and biological networks (Fig. 28).²⁵ First, data dimensionality was reduced via an unsupervised matrix factorization (singular value decomposition, SVD, Fig. 18a-b)²⁶, which linearly transformed the data into eigengenes and eigenarrays that indicate gene significance and sample grouping, respectively. The SVD separation of the samples in three-dimensional (3D) subspace (Fig. 18a) established a scoring metric by setting a 3D reference point at the centroid of the healthy controls and calculating Euclidean distance to each sample data point (Fig. 18b, scores are scaled between 0 and 1). Second, supervised machine learning created a predictive model via a bootstrap aggregated (bagged) decision tree ensemble (i.e. Random Forest^{™})²⁷, which used a forest of decision trees (n=5000) based on a random selection of a gene from the 10-gene panel to predict a mouse status as healthy or diseased. For each sample a prediction score (Fig. 18c) was determined via leave-one-out analysis. Third, a supervised approach using sparse partial least squares discriminant analysis (sPLS-DA)²⁸, was employed using the entire OpenArray^{™} data set for assessment of variable selection and classification (Fig. 29), which showed that 7 of 10 genes from the 10-gene panel were highly valuable for the stratification of samples (Fig. 29). Collectively, the output from these computational pipelines showed consensus in prediction (Fig. 18d), with progression of disease evidenced by the significant changes identified from Day 7 through Day 21.

The gene expression signatures were interrogated following therapy to test if the signature obtained from disease progression data (Figs. 17 & 18) maintained utility in the context of a therapeutic response. We investigated the potential of the signatures to differentiate therapeutically responsive and resistant mice using a mouse model where the tumor-bearing mammary gland was excised, with tissue biopsied from SynDx before and after treatment. BALB/c mice were implanted with multiple implants (n=8 per mouse) in their subcutaneous space to facilitate individualized, longitudinal tracking (Fig. 24d) following 4T1 tumor inoculation. Fourteen days following inoculation, tissue from the implant was biopsied prior to removal of the mammary gland to align with the Day 14 data in Figures 17 & 18. Immediately following biopsy (identified as Day 0 in Fig. 19), the tumor-bearing mammary gland was completely excised. Additionally, in healthy control mice a control excision of the mammary gland was performed as indicated in Fig. 19. At weekly intervals following mammary gland excision (Days 7, 14, and 21 post-excision in Fig. 19) a single biopsy was retrieved per mouse. At Day 7, following tumor-bearing mammary gland excision (Fig. 19A), a regression towards healthy baseline of all the genes with increased expression (S100a8, S100a9, Pglyrp1, Camp, Ltf, Chi3l3, and Ela2) was observed, which was a striking shift in the implant gene expression relative to the untreated disease course (Day 21, Fig. 17). This regression towards healthy continued over the next two weeks (Days 14 and 21 post-surgery) for responsive mice that survived long-term with no signs of recurrence at the study end (survival > 70 days following therapy). However, redirection of gene expressions towards diseased levels was observed for mice resistant to therapy (survival < 32 days following therapy). Signature analysis for each animal (Fig. 19i-k) also showed a shift towards healthy 7 days after therapy for all mice, however, the average prediction remained significantly elevated above the healthy control baseline for mice that would develop a recurrence. The difference was most notable 21 days after mammary gland excision, where the trajectory for surviving mice continued a downward trend while the resistant mice increased. Lastly, to improve divergence between resistant and responsive mice a revised 4-gene (S100a8, S100a9, Pglyrp1, Ltf) signature was constructed to test separation (Fig. 19l-m). ROC curve analysis of the training and therapy cohorts indicated a capacity of all computational signatures to separate resistant from responsive and healthy control mice (Fig. 19o). Interestingly, patient primary tumor sample analysis matched with survival data (Fig. 31) revealed some conserved inflammatory mechanisms that were readily captured by SynDx and may identify the timing of recurrence that would not be possible through the analysis of primary tumors used by platforms like Oncotype DX and Mammaprint.^{29,30}

To contextualize our findings, we analyzed the gene expression of blood leukocytes and lung biopsies. Liquid biopsies (Fig. 20) represent a relatively easy to access source for molecular analysis, while solid organ (e.g., lung) biopsies (Fig. 21) are arguably more valuable, yet acquisition poses greater patient risk. While 9 of the 10 genes from the panel were expressed at some level in blood, the pattern of expression differed from that of the implant-derived tissue. These dynamics were particularly evident for S100a8, S100a9, and Pglyrp1, which have a poor correlation to the disease stage and cannot distinguish disease progression in blood. Notably, these 3 factors (S100a8, S100a9, and Pglyrp1) in implant-derived tissue were most prominent in distinguishing therapy response and resistance (Fig. 19f-h). Importantly, the gene expression patterns in the lung at Day 21 closely mimicked that observed in the implant-derived tissue for all genes in the panel.

Herein the utility of SynDx developed through biopsy of tissue in microporous polymer implants during disease course in multiple pre-clinical models of metastatic breast cancer is demonstrated. Using SynDx as surrogates for metastatic sites in diseased vital organs, a high-throughput gene expression platform is implemented and signatures to track disease progression and enable longitudinal monitoring of individual mice before and after treatment are developed. Collectively, these data indicate that the tissue within implants is dynamic, with context-dependent profiles that reflect disease course and therapeutic response. Broadly, the concept of a synthesizing tissues and sites necessary for effective diagnostics may have substantial transdisciplinary potential when extended to other disorders with immunologically driven or affected components.

### EXAMPLE 6

This example describes the methods used for the experiments of Example 5.

*Microporous PCL scaffold fabrication:* Preparation of microporous PCL scaffolds for implantation was performed as previously described in studies that included material characterization via mechanical testing, scanning electron microscopy, and porosity calculations. Briefly, PCL microspheres were prepared by emulsifying (homogenization at 10,000 rpm for 1 minute) a 6% (w/w) solution of PCL (Lactel Absorbable Polymers) in dichloromethane in a 10% poly(vinyl alcohol) solution, which was then stirred in DI water for 3 hours. Particles were collected by centrifugation, serially washed with MilliQ filtered water, then lyophilized. Salt porogen (NACI) with a size range of 250-425 µm was selected through sieving. PCL microspheres and NaCl were then mixed in a 1:30 (w/w) ratio and pressed at 1,500 psi in a stainless-steel die (International Crystal Laboratories) for 45 seconds. The volume and die size used results in a PCL/NaCl disc that is 5 mm wide and 2 mm thick (Fig. 24a). PCL/NaCl discs were then heated on glass slides on a hot plate set to 135°C for 5 minutes per side. NaCl was then removed via leaching in MilliQ water for 1.5 hours. The resulting microporous PCL scaffolds were disinfected in 70% ethanol then serially rinsed in sterile filtered MilliQ water. Scaffolds were dried on a sterile gauze and stored at -80°C until time of implantation.

*Animals and scaffold implantation:* Microporous PCL scaffolds were implanted in the subcutaneous space of female mice to facilitate synthetic niche development for subsequent isolation. All animal studies were performed in accordance with institutional guidelines and protocols approved by the University of Michigan Institutional Animal Care and Use Committee. Female mice of the BALB/c and C57BL/6 strains were purchased from Jackson Laboratories at an age of 8 weeks. For implantation, mice were administered the analgesic Carprofen (5mg/kg) prior to and 24 hours after surgery. Mice were anesthetized under isoflurane (2.0-2.5%) and assessed by toe pinch reflex for level of sedation. The upper dorsal area above the scapula was then shaved and prepared using betadine and ethanol swabs. An incision (approximate 7-10 mm from anterior to posterior) was made in the upper dorsal area. A subcutaneous cutdown created a pocket in the lateral direction on the left and right sides of the mouse. A single scaffold was then inserted into each pocket. Two scaffolds were implanted for mice used to identify the initial gene expression changes and develop a signature. The incision line in these mice was closed with 7 mm stainless-steel wound clips (Roboz Surgical Instrument Co.). An array of eight scaffolds was implanted in mice used for longitudinal monitoring of disease course after therapy. Skin in these mice was closed with suture (MONOCRYL - poliglecaprone 25, Ethicon, Inc.).

*Metastatic tumor cell lines and animal inoculation:* Orthotopic inoculation of tumor cells was performed 2 weeks after scaffold implantation. 4T1-luc2-tdTomato cells were obtained from Perkin Elmer which had been authenticated previously via short tandem repeat (STR) and comparison with ATCC STR database. E0771 GFP+ cells were a kind gift from the Laboratory of Gary and Kathryn Luker at the University of Michigan Center for Molecular Imaging. To develop a more aggressive metastatic cell line, we employed a serial inoculation strategy that began when a C57BL/6 mouse inoculated with the parental line exhibited secondary metastatic growths at many sites including: lung, brain, mesentery, and diaphragm. Cells derived from the original lung (Lu.1) and brain (Br.1) metastases were then inoculated via intracardiac injection, which resulted in brain and lung metastases that were again isolated as Lu.2 and Br.2. Both Lu.1 and Lu.2 showed a stronger inclination for metastatic colonization of the lung (Fig. 25e), similar to reports on 4T1 phenotype. 4T1-luc2-tdTomato (BALB/c) or E0771 Lu.2 (C57BL/6) syngeneic tumor cells were orthotopically inoculated at a density of 40,000 cells/µL for a total of 800,000 in 20 µL of sterile Dulbecco's phosphate buffer saline (DPBS; Gibco). Inoculation was performed by making a small incision (5mm dorsal to ventral) above the 4^{th} right mammary fat pad. The mammary fat pad was exposed, injected, then the skin was closed with tissue adhesive (3M Vetbond^{™}). For healthy mice that were inoculated at late time points to study the dynamics at Day 70 and 133 the inoculation process was replicated with the inoculation being performed in the 4^{th} left mammary fat pad, as the right mammary fat pad had already been resected as a control.

*Biopsies of scaffold, blood, and lung tissue:* Microporous scaffolds, blood leukocytes, and lung tissue were isolated to study gene expression changes due to disease progression. Microporous scaffolds that had been implanted for either 7, 14, or 21 days following inoculation of primary tumors (as indicated in figures) were surgically biopsied following isoflurane (2.0-2.5%) sedation by making an incision adjacent to the implant site and removing the implant without taking surrounding tissue. For core-needle biopsy (CNB) of the synthetic niche a disposable CNB tool (Bard^{®} Mission^{®} Disposable Core Biopsy Instrument) was inserted through the skin and used to isolate a portion of the synthetic niche (Fig. 23b), which enables minimally invasive retrieval of samples. Blood leukocytes were isolated via an intracardiac blood draw stabilized by EDTA. Erythrocyte lysis in EDTA-treated blood samples was performed with Ammonium-Chloride-Potassium (ACK) Lysing Buffer (Gibco) with serial washes in DPBS. Lung tissue from time-matched healthy control and tumor-bearing mice at Day 21 were isolated for endpoint comparative analysis following euthanasia.

*Surgical resection of primary tumor and longitudinal tracking:* To test the responsiveness of the synthetic niche to therapeutic treatment mammary gland removal was performed on the 4T1 primary tumor that had been inoculated in the 4^{th} right mammary fat pad of BALB/c mice. In this model, the primary tumor was resected 14 days after inoculation, which aligns with the time point for the initial set of experiments to study the progressive changes in the synthetic niche as a function of disease course. The skin above the primary tumor was prepared by swabbing with betadine and ethanol. A dermal incision was made adjacent to the primary tumor for the length of the primary tumor and mammary fat pad (approximately 1 cm). Large vessels surrounding the primary tumor were cauterized using a Gemini Cautery System (Roboz Surgical Instrument Co.). Iris scissors were used to cut connective tissue to separate the mammary fat pad and contained primary tumor from the superficial dermal and underlying body cavity wall. The dermal incision was closed using suture (MONOCRYL - poliglecaprone 25, Ethicon, Inc.). Following tumor resection and mammary gland removal, animal health was monitored daily for activity and responsiveness, including posture, mobility, body weight, grooming behavior, and respiratory conditions. Animals were euthanized if found in a moribund condition or when a primary tumor regrowth was positively identified.

*RNA isolation, purity, integrity, and cDNA synthesis:* Total RNA was isolated from synthetic niches, lung, and blood leukocytes. Frozen (-80°C), surgically biopsied scaffolds were immersed in monophasic solution of phenol and guanidine isothiocyanate (TRIzol^{®} reagent, Thermo Fisher Scientific, Waltham, MA) at 4°C, then homogenized with a rotor stator homogenizer (T25, S25N-8G-ST dispersing element, IKA^{®} Works, Inc.). Samples were then centrifuged at 10,000g to remove particulate, and the TRlzol^{®} containing genomic content from the scaffold was then processed in a silica-based matrix spin column (Direct-zol^{™} RNA Miniprep Plus, Zymo Research Corp., Irvine, CA) with DNase I treatment to isolate total RNA. Concentration (modified Beer-Lambert at 260nm) and purity (260nm/280nm absorbance ratio) of the RNA isolation was assessed by light absorbance via a NanoDrop 2000c (Thermo Fisher Scientific). Additionally, RNA integrity number (RIN) analysis was obtained through RNA fragment analysis (RNA 6000 Nano Kit on a 2100 Bioanalyzer, Agilent Technologies, Inc., Santa Clara, CA). RIN for samples used in high-throughput RT-qPCR ranged from 8.2-9.7. Generation of first strand cDNA was performed through reverse transcription (RT, SuperScript^{™} VILO^{™} cDNA Synthesis Kit, Thermo Fisher Scientific). For all RT-qPCR of synthetic niche, RT was performed with an RNA concentration of 200 ng/µL. cDNA was either used immediately for RT-qPCR or stored at -80°C. Frozen lung tissue biopsy RNA was isolated in similar fashion to synthetic niches with RT performed at an RNA concentration of 200 ng/µL. Blood leukocyte RNA was isolated by thoroughly mixing blood leukocytes following ACK lysis in TRIzol, then processing for RNA and performing RT at 100 ng/µL due to limited quantities of total RNA. RNA concentration was increased to 200 ng/µL (niches and lungs) or 100 ng/µL (blood) using a RNA clean-up kit (RNA Clean & Concentrator-5, Zymo Research Corp.) for samples below these thresholds.

*OpenArray^{™} high-throughput RT-qPCR:* Synthetic niche gene expression was analyzed with OpenArray^{®} (OA), a high-throughput RT-qPCR platform that analyzes 648 genes per sample in parallel, in accordance with the standard OA protocol. All materials used in this section were purchased from Thermo Fisher Scientific. Briefly, the genes for this study were focused on the inflammatory pathways and included a panel of 16 reference (housekeeping) genes per sample (Applied Biosystems^{™} TaqMan^{™} OA Mouse Inflammation Panel, Cat. No. 4475393). cDNA from synthetic niches biopsied from tumor-free healthy controls (n=14 total healthy) and tumor-bearing mice (n=14 total diseased) across the three time points of 7 (n=3), 14 (n=8), and 21 (n=3) days were mixed with 2X TaqMan^{®} OA Real-Time Master Mix then loaded onto the OA panels via a robotic OA AccuFill^{™} System in an order that staggered conditions and time points across the panels analyzed. The OA RT-qPCR run (QuantStudio 12K Flex Real-Time PCR System, ThermoFisher Scientific) and sample quality control were performed by the Affymetrix Group at the University of Michigan DNA Sequencing Core. In accordance with OA standard operating procedure, C_{q} was calculated as Cᵣₜ, a curve-specific method. Complete matrices of raw data for non-normalized (C_{q}), reference gene normalized (ΔC_{q}), and fold change calculations between tumor-bearing and healthy (ΔΔC_{q}) at Days 7, 14, and 21 are available and organized according to the RT-PCR GEOarchive template.

*Analysis of gene expression and selection of genes of interest:* Gene expressions from OA was screened to identify genes of interest during disease course. First, genes with insufficient data within synthetic niche samples (missing greater than 4/14 per cohort or 2/3 per time-point) were dropped from the study, resulting in 559 genes for full analysis. Next the 16 reference genes were ranked according to their expression stability compared to each other and as a function of experimental design (NormFinder Algorithm), which led to the selection of Gapdh, Tbp, Ywhaz, Hmbs, and Ubc. ΔC_{q} values were calculated against the average of the reference genes, centered on median of time-matched healthy controls, then standardized for cluster and multivariate statistical analysis. From ΔC_{q} data fold change, significance (uncorrected p and false-discovery rate corrected q), at Days 7, 14, and 21 were calculated. For computational analysis ΔC_{q} values were log₂ transformed and centered on the healthy-control time-matched median as reflected in the figure box plots, which show the median, 25th-75th percentiles and most extreme data points not considered outliers (outliers are visually indicated by +). Management of non-detects within the OA data was handled in two manners: for multivariate statistical analysis non-detects were interpolated based on the ΔC_{q} average across all cohorts and time points, and for signature construction non-detects were interpolated based on the ΔC_{q} average for a specific cohort at a specific time point.

*10-gene panel RT-qPCR analysis in 96-well format:* Experiments for signature validation, analysis of blood, lung and post-excision monitoring was performed by RT-qPCR analysis in a 96-well plate format using matched Taqman^{®} probes from the OA platform. Like the OA RT-qPCR, samples were staged within plates alternating between healthy and diseased. The same 5 reference genes (Gapdh, Tbp, Ywhaz, Hmbs, Ubc) were run in parallel with the 10 target genes of interest (Bmp15, Camp, Ccl22, Ccr7, Chi3l3, Ela2, Ltf, Pglyrp1, S100a8, S100a9) for all studies in BALB/c mice. Ywhaz and Ubc were only used for the C57BL/6-E0771 analysis due to poor detection and poor stability in Gapdh, Tbp, and Hmbs reference genes. Analysis was performed on a CFX Connect^{™} Real-Time PCR Detection System (Bio-Rad Laboratories, Inc., Hercules, CA) with CFX Manager Software that calculated the C_{q} values based on the regression analysis mode, which applies a multivariable, nonlinear regression model to each well trace. For signature computation and multivariate statistics, non-detects were interpolated based on ΔC_{q} average across all cohorts and time points to limit predictive bias, which could be exaggerated by use of a static, arbitrary ΔC_{q}. Non-detects were left blank for univariate, multiple comparison, and linear mixed model analysis. Synthetic niche gene expression dynamics and blood gene expression dynamics were compared by goodness of fit using a normalized root mean square error cost function on a linear polynomial curve fit between Days 7, 14, and 21. Cohort centering was used to align the longitudinal data from the therapy study for subsequent signature analysis. The median of the therapy cohorts Day 0 pre-excision was centered on the OA Day 14 data median, which was experimentally equivalent. The calibration factor used for aligning therapy cohorts Day 0 to OA Day 7 was applied to all successive time points.

*Gene expression dimensionality reduction and classification:* The source data and code for the computational pipeline (Fig. 27) within this section are publicly accessible as indicated in the Data and Code Availability sections. To screen for the genes of interest an Elastic Net regularization, a variation of the LASSO (least absolute shrinkage and selection operator) algorithm with an α=0.1 and leave-one-out cross validation, was employed using the Matlab lasso function on the ΔC_{q} of the 559 genes with sufficient data. This data indicated the predictive nature of genes for tumor-bearing classification at specific time points (Fig. 24b). Clustering of samples was performed with the Matlab clustergram function on standardized ΔC_{q} data. Dendograms indicate clustered genes and samples, in which samples are indicated on the x-axis and genes expressed are indicated on the y-axis with a complete linkage and a display range of -3.5 to +3.5. Unsupervised dimensionality reduction was performed using singular value decomposition (X=USV^{T}) with the Matlab svds function on the 10-gene and 4-gene panels standardized ΔC_{q} values centered on the time-matched healthy controls. The first 3 principle components (PCs) were computed from the first 3 left singular vectors (columns of U, eigenassays) and singular values (diagonal matrix S). Unsupervised separation of the samples was visualized by plotting the PCs in 3D scatter, which was quantified by calculating the 3D Euclidean distance to each sample from the centroid of all the healthy-controls. Distance calculations were scaled between 0 and 1. A supervised machine learning algorithm, bootstrap aggregated (bagged) decision tree ensemble (i.e., Random Forest^{™}), was used to construct a predictive model based on the 10-gene and 4-gene panels standardized ΔC_{q} values centered on the time-matched healthy controls. The model was constructed using the Matlab core fitcensemble function with the Bag method. Decision tree depth was limited by the number of splits to two times the number of input predictors (genes). The number of learning cycles for final model was set to 5000 cycles. A partitioned model and leave-one-out cross-validation was performed using the Matlab crossval and kfoldPredict functions, which returned the posterior probability for classification of each sample. A second supervised learning approach, Sparse Partial Least Squares Discriminant Analysis (sPLS-DA) was employed using the MixOmics package in R, to process the ΔC_{q} from 632 target genes at Days 7, 14, and 21. For this analysis ΔC_{q} was normalized to a combination of the 8 most stable reference genes (Hmbs, Gusb, Ubc, Ywhaz, Cdkn1a, Tbp, Gapdh, Hprt1), yet was not log₂ transformed or centered. This reduction in processing was done to improve confidence in the selected genes of interest. For cluster analysis the 50 (25 positive and 25 negative) most valuable genes for classification were retained. For sample and gene clustering the R cim function was used. Receiver Operating Characteristic (ROC) curves were used to compute the true positive rate (sensitivity) against the false positive rate (specificity) to determine diagnostic cutoff points and model accuracy for training cohort (n=28) data with SVD and bagged tree as test variables and tumor-bearing designation as the state variable. The therapy cohort from post-excision Days 7 to 21 was analyzed by the 10-gene panel SVD, 10-gene panel bagged tree prediction, 4-gene (S100a8, S100a9, Pglyrp1, and Ltf) cluster SVD, 4-gene cluster bagged tree prediction as test conditions with the therapy resistance designation as the state variable. Area under the curve with confidence intervals are listed within the figure legend.

*Statistics:* This section details the statistical tests used to evaluate the hypotheses. Error bars are defined within figure legends, with all line plots indicating standard error of the mean. The exact n for each test is detailed in the legends along with the methods for multiple comparisons corrections. All n throughout the manuscript indicate biological replicates from different mice. During the initial screen of the OA data and selection of genes of interest, a false-discovery rate corrected two-tailed t-test using a linear step-up procedure to account for all genes analyzed (559 total following elimination of genes that did not contain sufficient sample) contrasted samples from diseased mice and healthy time-matched controls. These tests were carried out in Matlab using the mattest and mafdr functions. All statistical indicators within the manuscript were computed using packages and syntax in the Statistical Package for the Social Sciences (SPSS, International Business Machines Co., Armonk, NY). Multiple comparisons analysis for comparisons with an individual time point were compared via an independent Student's two-tailed t-test with Dunn-Šidák correction for multiple comparisons resulting in a p<0.0051 being considered significant. Two-way multivariate analysis of variance (MANOVA) for genes of interest and reduced dependent variables (SVD score or bagged tree prediction) were performed within the generalized linear model (GLM) syntax package using the BY command for condition and time. First, Pillai's Trace statistic (p<0.05) was used to determine MANOVA significant interaction effects. Second, fixed-effects ANOVA was used to assess univariate interaction effects. Last, following determination of multivariate and univariate interaction effects, a post-hoc simple effects analysis was computed with Šidák correction from an initial p<0.05 for multiple comparisons to determine within groups over time and between groups at Days 7, 14, and 21. To analyze the differences within mice tracked longitudinally before and after a therapeutic tumor excision a Linear Mixed Model (LMM) was employed. The SPSS syntax for the LMM is available in the prepress repository. First, two-way ANOVA within the LMM was assessed for significant (p<0.05) or trending (p<0.1) interactions. Last, post hoc simple effects analysis indicated significant differences (p<0.05) within groups over time and between groups at Days 7, 14, and 21. Within the figures the focus for within group effects was how the gene expression, SVD, or bagged tree data changed from pre-excision (Day 0) to post-excision (Days 7, 14, and 21).

*Breast cancer patient microarray Kaplan-Meier correlation:* A repository of human patient microarray data was analyzed for gene expressions correlations with high or low gene expression and patient recurrence free survival (RFS). Microarray data from Gene Expression Omnibus (GEO) via Kaplan-Meier Plotter (KMPlot, kmplot.com/analysis/) and prognosis data were queried for genes selected for the 10-gene panel from the synthetic niche to determine the expression level relevance in primary tumors on the clinical outcome of systemically untreated breast cancer patients. Separation of high and low gene expression profiles was automatically divided by the median expression of the genes for all samples (n=818). Redundant and outlier microarrays were excluded, and only breast cancer patients that were systemically untreated were included. Data outputs for Hazard Ratio (HR) and logrank significance are indicated in plots with multiple comparisons corrected using the KMPlotter tool for false-discovery rate (FDR) with FDR 10% corrected significance (p<0.011) and (**) FDR 5% corrected significance (p<0.00099).

*Protocol availability and life science reporting summary:* Many of the experimental protocols including scaffold fabrication, RNA isolation, and RT-qPCR follow previously published or manufacturer recommended guidelines and additional specifics may be located in the life science reporting summary.

### EXAMPLE 7

This example describes an exemplary method of treating a subject.

A polymer scaffold comprising PCL is implanted into a subject following the treatment of a primary tumor (e.g., a malignant growth in the breast) according to standard of therapy (surgery, chemotherapy, radiation). The polymer scaffold is implanted by either surgical insertion or through minimally-invasive placement via a trocar. After insertion, a tissue sample is obtained from the scaffold through a core-needle biopsy to establish a baseline result for the patient. The same core-needle biopsy approach is used at scheduled intervals (e.g., every 3 months) to generate a metastatic potential score and thereby monitor the patient for early-onset metastatic events. To attain the metastatic potential score, the cellular components of the implant are processed to extract total RNA through use of a standard Trizol extraction protocol and purification technique using a silica based filter and DNase. (Zymo Research Directzol and Qiagen RNeasy kits are both suitable options, though others exist.)

The purified RNA is processed via qRT-PCR for a panel of genes associated with disease progression (as detailed in the data) using TaqMan probes or SYBR-green, based detection. The expression of the genes in the panel is analyzed by an amplification curve regression analysis. Gene expression is normalized to a panel of housekeeping genes (e.g., Gapdh, Hmbs, Tbp, Ubc, Ywhaz) which is averaged for each sample (as demonstrated and common in clinical practice). Gene expression data is processed to generate a variety of graphical outputs, including a heatmap to graphically represent a patient's readout compared to healthy and diseased control groups, as well as the patient's previous readouts. Each is expressed as a comparison to healthy controls, thus providing insights on a patient's similarity or dissimilarity with healthy or matched diseased samples. The patient results are also classified as either pre-metastatic, early metastatic, or late metastatic through machine learning algorithms or linear regression (e.g., using singular value decomposition and bootstrap aggregated decision trees).

The sample analysis can provide a range of scores, with a low score indicating healthy, an intermediate score indicating an early stage of disease, such as pre-metastatic, and a high score indicating metastases. The observation of an intermediate score that is distinct from a healthy control leads to the application of therapies that target the immune cells at a metastatic niche, such as phosphodiesterase 5 (PDE-5) or COX-2 inhibitors that inhibit the functionality of myeloid derived suppressor cells. The observation of a high score or one that is distinct from a healthy control is indicative of high metastatic potential and disease conditioning, and thus, anti-metastatic disease therapy is warranted for the subject. If a high score or signature of metastatic disease is observed, the subject is determined as needing treatment targeting the metastatic cells, such as, for instance, PARP inhibitors (e.g., Olaparib) or alternative chemotherapies (e.g. Gemcitabine). Additionally, or alternatively, the subject may be treated with more specific drugs targeting metastatic pathways that are currently available for clinical use or are in the drug discovery pipeline. The implant environment is reassessed for metastatic potential following treatments to identify the efficacy of a specific approach in suppressing the metastasis promoting environment.

One to three months following treatment in response to a low (e.g., PDE-5) or high score (e.g., Olaparib), a biopsy from the scaffold is taken, the cells from the biopsy are processed, RNA is isolated, and gene expression analysis is performed as described above. The observation that the patient's readout is more concordant with a healthy control indicates that the treatment with the chosen drug is effective, whereas a readout indicating concordance with the disease state indicates that a different treatment strategy, e.g., treatment with eribulin mesylate, should be implemented. If the specific treatment plan for the patient does not result in a reduction in the metastatic potential score, ongoing readouts from the scaffold can serve to direct patient care towards alternative targeted management plans. Follow up analysis as described is performed regularly (e.g., every 3 months) until a patient's condition plateaus (e.g., patient experiences 9 months at a continuous readout, no progression of metastatic events) at a level consistent with disease remission or stability. The implant may remain in the patient for future tests as necessary.

### REFERENCES

1 Kaplan et al., Nature 438, 820-827, doi:10.1038/nature04186 (2005).
2 Giles et al., Cancer Res, doi:10.1158/0008-5472.CAN-15-0204 (2015).
3 Hiratsuka et al., Nat Cell Biol 8, 1369-1375, doi:10.1038/ncb1507 (2006).
4 Sinha et al., The Journal of Immunology 181, 4666-4675, doi:10.4049/jimmunol.181.7.4666 (2008).
5 Oliva et al., Sci Transl Med 7, 272ra211, doi:10.1126/scitranslmed.aaa1616 (2015).
6 Socarras et al., PLoS One 9, e110945, doi:10.1371/journal.pone.0110945 (2014).
7 Vegas et al., Nat Biotechnol, doi:10.1038/nbt.3462 (2016).
8 Harris et al., J Clin Oncol 34, 1134-1150, doi:10.1200/JCO.2015.65.2289 (2016).
9 Minn et al., J Clin Invest 115, 44-55, doi:10.1172/JCI22320 (2005).
10 Luzzi et al., Am J Pathol 153, 865-873, doi:10.1016/S0002-9440(10)65628-3 (1998).
11 Azarin et al., Nat Commun 6, 8094, doi:10.1038/ncomms9094 (2015).
12 Rao et al., Cancer Research 76, 5209-5218, doi:10.1158/0008-5472.Can-15-2106 (2016).
13 Aguado et al., Acta Biomater 33, 13-24, doi:10.1016/j.actbio.2016.01.043 (2016).
14 Bersani et al., Cancer Res 74, 7229-7238, doi:10.1158/0008-5472.CAN-14-1809 (2014).
15 Cheng et al., J Exp Med 205, 2235-2249, doi:10.1084/jem.20080132 (2008).
16 Hiratsuka et al,. Nat Cell Biol 10, 1349-1355, doi:10.1038/ncb1794 (2008).
17 Ouzounova et al., Nat Commun 8, 14979, doi:10.1038/ncomms14979 (2017).
18 Downs-Canner et al., Nat Commun 8, 14649, doi:10.1038/ncomms14649 (2017).
19 Weber et al., Breast Cancer Res 11, R6, doi:10.1186/bcr2221 (2009).
20 Youn et al., J Leukoc Biol 91, 167-181, doi:10.1189/jlb.0311177 (2012).
21 Kim et al. Nat Commun 9, 503, doi:10.1038/s41467-017-02731-6 (2018).
22 Muller et al., Nature 410, 50-56, doi:10.1038/35065016 (2001).
23 Curiel et al., Nat Med 10, 942-949, doi:10.1038/nm1093 (2004).
24 Kwa et al., Nat Rev Clin Oncol 14, 595-610, doi:10.1038/nrclinonc.2017.74 (2017).
25 Janes et al., Curr Opin Chem Biol 10, 73-80, doi:10.1016/j.cbpa.2005.12.016 (2006).
26 Alter et al., Proceedings of the National Academy of Sciences 97, 10101-10106, doi:10.1073/pnas.97.18.10101 (2000).
27 Breiman, Machine Learning 45, 5-32, doi:10.1023/a:1010933404324 (2001).
28 Decker et al., BiotechnolBioeng 114, 2085-2095, doi:10.1002/bit.26293 (2017).
29 Paik et al., N Engl J Med 351, 2817-2826, doi:10.1056/NEJMoa041588 (2004).
30 van de Vijver et al., N Engl J Med 347, 1999-2009, doi:10.1056/NEJMoa021967 (2002).

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any nonclaimed element as essential to the practice of the disclosure.

Preferred embodiments of this disclosure are described herein, including the best mode known to the inventors for carrying out the disclosure. Variations of those preferred embodiments may become apparent to those of ordinary skill in the art upon reading the foregoing description. The inventors expect skilled artisans to employ such variations as appropriate,. Moreover, any combination of the above-described elements in all possible variations thereof is encompassed by the disclosure unless otherwise indicated herein or otherwise clearly contradicted by context.

## Claims

1. A method for determining a subject's metastatic potential, or determining a tumor's or cancer's metastatic stage, or determining a subject's need for metastatic disease therapy, or determining treatment for a subject with a tumor or cancer, or determining a treatment's efficacy for treating a metastatic disease, or detecting metastatic disease or a predisposition thereto, in a subject in need thereof,
the method comprising:
measuring levels of expression of a panel of genes, RNAs, or proteins in a test sample that has been obtained from a synthetically-engineered pre-metastatic niche (pMN) implanted in the subject, wherein the test sample comprises immune cells, stromal cells, or a combination thereof;
wherein the measured expression levels of the panel of genes, RNAs, or proteins in the test sample is compared to control levels or to the level of expression of the panel of genes, RNAs, or proteins in an earlier sample that has been obtained from the synthetically-engineered pMN at an earlier time point,
and wherein the panel of genes comprises one or more of S100 Calcium Binding Protein A8 (S100a8), S100 Calcium Binding Protein A9 (S100a9), Peptidoglycan Recognition Protein 1 (Pglyrpl), Lactotransferrin (Ltf), Cathelicidin Antimicrobial Peptide (Camp), Elastase 2 (Ela2), Chitinase (Chi3l3), Bone Morphogenetic Protein 15 (Bmp15), C-C Motif Chemokine Ligand 22 (Ccl22), and C-C Motif Chemokine Receptor 7 (Ccr7), or
the panel of RNAs comprises one or more RNAs encoded by S100 Calcium Binding Protein A8 (S100a8), S100 Calcium Binding Protein A9 (S100a9), Peptidoglycan Recognition Protein 1 (Pglyrpl), Lactotransferrin (Ltf), Cathelicidin Antimicrobial Peptide (Camp), Elastase 2 (Ela2), Chitinase (Chi3l3), Bone Morphogenetic Protein 15 (Bmp15), C-C Motif Chemokine Ligand 22 (Ccl22), and C-C Motif Chemokine Receptor 7 (Ccr7), or
the panel of proteins comprises one or more cytokines and/or one or more chemokines.

2. The method of claim 1, wherein the method is for determining a treatment's efficacy for treating a metastatic disease, and wherein the earlier sample was obtained before the treatment and the test sample was obtained after the treatment,
wherein optionally the treatment was surgical removal of a tumor, or radiation therapy, or administration of a compound.

3. The method of any one of the preceding claims, wherein the measured expression levels are compared to control levels.

4. The method of any one of the preceding claims, wherein the control levels of the genes, RNAs, or proteins are of a subject known to have metastatic disease or of a subject known to not have metastatic disease.

5. The method of any one of the preceding claims, wherein the measured expression levels of the panel of genes, RNAs, or proteins form a pMN expression signature and wherein the pMN expression signature is processed through a decomposition algorithm to obtain a single score for gene expression and/or a machine learning algorithm to obtain a score of prediction of disease state,
wherein optionally the decomposition algorithm is a singular value decomposition and/or the machine learning algorithm is a random forest generation.

6. The method of claim 5, wherein the single score for gene expression and the score of prediction of disease state are combined to provide a combined score of metastatic potential.

7. The method of any one of the preceding claims, wherein the control levels of the genes, RNAs, or proteins form a control pMN expression signature indicative of metastatic disease,
wherein optionally the control pMN expression signature indicative of metastatic disease is processed through a decomposition algorithm to obtain a single score for gene expression and/or a machine learning algorithm to obtain a score of prediction of disease state.

8. The method of claim 7, wherein the decomposition algorithm is a singular value decomposition or wherein the machine learning algorithm is a random forest generation.

9. The method of claim 7 or claim 8, wherein the control pMN expression signature is processed through a decomposition algorithm to obtain a single control score for gene expression and a machine learning algorithm to obtain a control score of prediction of disease state,
wherein optionally the single control score for gene expression and the control score of prediction of disease state are combined to provide a combined control score.

10. The method of claim 9, wherein the combined score of metastatic potential is compared to the combined control score to determine the subject's metastatic potential.

11. The method of any one of the preceding claims, wherein the control levels of the genes, RNAs, or proteins form a control pMN expression signature indicative of no metastatic disease,
wherein optionally the control pMN expression signature indicative of no metastatic disease is processed through a decomposition algorithm to obtain a single score for gene expression and/or a machine learning algorithm to obtain a score of prediction of disease state.

12. The method of claim 11, wherein the decomposition algorithm is a singular value decomposition and/or wherein the machine learning algorithm is a random forest generation.

13. The method of claim 11 or claim 12, wherein the single control score for gene expression and the control score of prediction of disease state are combined to provide a combined control score of no metastatic disease,
wherein optionally the combined score of metastatic potential is compared to the combined control score of no metastatic disease to determine the subject's metastatic potential.

14. The method of claim 13, wherein:
when the combined score of metastatic potential is low, relative to the combined control score, the subject's metastatic potential is low and/or the subject is deemed healthy, when the combined score of metastatic potential is intermediate, relative to the combined control score, the subject's metastatic potential indicates an early stage of metastatic disease, and when the combined score of metastatic potential is high, relative to the combined control score, the subject's metastatic potential indicates metastatic disease, or
when the combined score of metastatic potential is intermediate, relative to the combined control score, the subject's metastatic potential indicates an early stage of metastatic disease, and the subject is determined as needing a therapy that targets the immune cells at the metastatic niche, such as phosphodiesterase 5 (PDE-5) or COX-2 inhibitors that inhibit the functionality of myeloid derived suppressor cells, or
when the combined score of metastatic potential is high, relative to the combined control score, the subject's metastatic potential indicates metastatic disease, and the subject is determined as needing an anti-metastatic disease treatment, wherein optionally the anti-metastatic disease treatment comprises a PARP inhibitor or Gemcitabine.

15. The method of any one of the preceding claims,
wherein optionally the immune cells comprise macrophages, dendritic cells, MDSCs, neutrophils, monocytes, helper T cells, cytotoxic T cells, B cells, NK cells, CD45+ cells, or a combination of two or more of the foregoing;
and wherein optionally the stromal cells comprise fibroblasts, endothelial cells, pericytes, or a combination of two or more of the foregoing.

16. The method of any one of the preceding claims, wherein less than 5% of cells in the test sample are cancer cells;
wherein optionally less than 3%, or less than 2%, or less than 1%, or less than 0.5%, or less than 0.1% of cells in the test sample are cancer cells.

17. One or more agents indicated as anti-metastatic disease treatments, for use in a method of treatment of metastasis in a subject, wherein the method of any one of the preceding claims is carried out and wherein the one or more anti-metastatic agents is then administered to the subject based on the subject's metastatic potential.

## Patentansprüche

1. Verfahren zur Bestimmung des metastatischen Potenzials bei einem Subjekt, oder zur Bestimmung des metastatischen Stadiums eines Tumors oder Krebses, oder zur Bestimmung des Bedarfs eines Subjekts an Therapie gegen metastatische Erkrankung, oder zur Bestimmung der Behandlung für ein Subjekt mit einem Tumor oder Krebs, oder zur Bestimmung der Wirksamkeit einer Behandlung zum Behandeln einer metastatischen Erkrankung, oder zum Nachweis von metastatischer Erkrankung oder einer Prädisposition dafür, in einem Subjekt, das dessen bedarf,
wobei das Verfahren umfasst:
Messen der Expressionsniveaus einer Palette von Genen, RNAs oder Proteinen in einer Testprobe, die erhalten wurde aus einer synthetisch erzeugten prämetastatischen Nische (pMN), die in dem Subjekt implantiert wurde, wobei die Testprobe Immunzellen, stromale Zellen oder eine Kombination davon umfasst;
wobei die gemessenen Expressionsniveaus der Palette von Genen, RNAs oder Proteinen in der Testprobe mit Kontrollniveaus oder dem Expressionsniveau der Palette von Genen, RNAs oder Proteinen in einer früheren Probe, die zu einem früheren Zeitpunkt aus der synthetisch erzeugten pMN erhalten wurde, verglichen werden,
und wobei
die Palette von Genen eines oder mehrere von S100-Calcium-Binding-Protein A8 (S100a8), S100-Calcium-Binding-Protein A9 (S100a9), Peptidoglycan-Recognition-Protein 1 (Pglyrpl), Lactotransferrin (Ltf),
Cathelicidin-Antimikrobielles-Peptid (Camp), Elastase 2 (Ela2), Chitinase (Chi3l3), Bone-Morphogenetic-Protein 15 (Bmp15), C-C-Motiv Chemokin-Ligand 22 (Ccl22) und C-C-Motiv Chemokin-Rezeptor 7 (Ccr7) umfasst, oder
die Palette von RNAs eine oder mehrere RNAs umfasst, codiert durch S100-Calcium-Binding-Protein A8 (S100a8), S100-Calcium-Binding-Protein A9 (S100a9), Peptidoglycan-Recognition-Protein 1 (Pglyrpl), Lactotransferrin (Ltf), Cathelicidin-Antimikrobielles-Peptid (Camp), Elastase 2 (Ela2), Chitinase (Chi3l3), Bone-Morphogenetic-Protein 15 (Bmp15), C-C-Motiv Chemokin-Ligand 22 (Ccl22) und C-C-Motiv Chemokin-Rezeptor 7 (Ccr7), oder
die Palette von Proteinen ein oder mehrere Cytokine und/oder ein oder mehrere Chemokine umfasst.

2. Verfahren nach Anspruch 1, wobei das Verfahren zur Bestimmung der Wirksamkeit einer Behandlung zum Behandeln einer metastatischen Erkrankung vorgesehen ist, und wobei die frühere Probe vor der Behandlung erhalten wurde und die Testprobe nach der Behandlung erhalten wurde,
wobei optional die Behandlung in der chirurgischen Entfernung eines Tumors oder einer Strahlentherapie oder der Verabreichung einer Verbindung bestand.

3. Verfahren nach einem der vorhergehenden Ansprüche, wobei die gemessenen Expressionsniveaus mit Kontrollniveaus verglichen werden.

4. Verfahren nach einem der vorhergehenden Ansprüche, wobei die Kontrollniveaus der Gene, RNAs oder Proteine von einem Subjekt, das bekanntermaßen eine metastatische Erkrankung aufweist, oder von einem Subjekt, das bekanntermaßen keine metastatische Erkrankung aufweist, stammen.

5. Verfahren nach einem der vorhergehenden Ansprüche, wobei die gemessenen Expressionsniveaus der Palette von Genen, RNAs oder Proteinen eine pMN-Expressionssignatur bilden und wobei die pMN-Expressionssignatur verarbeitet wird durch einen Dekompositionsalgorithmus zum Erhalten eines Einzelscores für Genexpression und/oder einen Machine-Learning-Algorithmus zum Erhalten eines Scores der Vorhersage des Erkrankungszustands,
wobei es sich optional bei dem Dekompositionsalgorithmus um eine Singulärwertzerlegung und/oder bei dem Machine-Learning-Algorithmus um eine Random-Forest-Erstellung handelt.

6. Verfahren nach Anspruch 5, wobei der Einzelscore für Genexpression und der Score der Vorhersage des Erkrankungszustands kombiniert werden, um einen kombinierten Score des metastatischen Potenzials bereitzustellen.

7. Verfahren nach einem der vorhergehenden Ansprüche, wobei die Kontrollniveaus der Gene, RNAs oder Proteine eine auf metastatische Erkrankung hinweisende Kontroll-pMN-Expressionssignatur bilden,
wobei optional die auf metastatische Erkrankung hinweisende Kontroll-pMN-Expressionssignatur verarbeitet wird durch einen Dekompositionsalgorithmus zum Erhalten eines Einzelscores für Genexpression und/oder einen Machine-Learning-Algorithmus zum Erhalten eines Scores der Vorhersage des Erkrankungszustands.

8. Verfahren nach Anspruch 7, wobei es sich bei dem Dekompositionsalgorithmus um eine Singulärwertzerlegung handelt oder wobei es sich bei dem Machine-Learning-Algorithmus um eine Random-Forest-Erstellung handelt.

9. Verfahren nach Anspruch 7 oder Anspruch 8, wobei die Kontroll-pMN-Expressionssignatur verarbeitet wird durch einen Dekompositionsalgorithmus zum Erhalten eines Einzel-Kontrollscores für Genexpression und einen Machine-Learning-Algorithmus zum Erhalten eines Kontrollscores der Vorhersage des Erkrankungszustands, wobei optional der Einzel-Kontrollscore für Genexpression und der Kontrollscore der Vorhersage des Erkrankungszustands kombiniert werden, um einen kombinierten Kontrollscore bereitzustellen.

10. Verfahren nach Anspruch 9, wobei der kombinierte Score des metastatischen Potenzials mit dem kombinierten Kontrollscore verglichen wird, um das metastatische Potenzial des Subjekts zu bestimmen.

11. Verfahren nach einem der vorhergehenden Ansprüche, wobei die Kontrollniveaus der Gene, RNAs oder Proteine eine Kontroll-pMN-Expressionssignatur bilden, die auf das Fehlen von metastatischer Erkrankung hinweist, wobei optional die auf das Fehlen von metastatischer Erkrankung hinweisende Kontroll-pMN-Expressionssignatur verarbeitet wird durch einen Dekompositionsalgorithmus zum Erhalten eines Einzelscores für Genexpression und/oder einen Machine-Learning-Algorithmus zum Erhalten eines Scores der Vorhersage des Erkrankungszustands.

12. Verfahren nach Anspruch 11, wobei es sich bei dem Dekompositionsalgorithmus um eine Singulärwertzerlegung handelt und/oder wobei es sich bei dem Machine-Learning-Algorithmus um eine Random-Forest-Erstellung handelt.

13. Verfahren nach Anspruch 11 oder Anspruch 12, wobei der Einzel-Kontrollscore für Genexpression und der Kontrollscore der Vorhersage des Erkrankungszustands kombiniert werden, um einen kombinierten Kontrollscore des Fehlens von metastatischer Erkrankung bereitzustellen,
wobei optional der kombinierte Score des metastatischen Potenzials mit dem kombinierten Kontrollscore des Fehlens von metastatischer Erkrankung verglichen wird, um das metastatische Potenzial des Subjekts zu bestimmen.

14. Verfahren nach Anspruch 13, wobei:
wenn der kombinierte Score des metastatischen Potenzials im Vergleich zum kombinierten Kontrollscore niedrig ist, das metastatische Potenzial des Subjekts niedrig ist und/oder das Subjekt als gesund erachtet wird, wenn der kombinierte Score des metastatischen Potenzials im Vergleich zum kombinierten Kontrollscore mittelgroß ist, das metastatische Potenzial des Subjekts auf metastatische Erkrankung im Frühstadium hinweist, und wenn der kombinierte Score des metastatischen Potenzials im Vergleich zum kombinierten Kontrollscore hoch ist, das metastatische Potenzial des Subjekts auf metastatische Erkrankung hinweist, oder
wenn der kombinierte Score des metastatischen Potenzials im Vergleich zum kombinierten Kontrollscore mittelgroß ist, das metastatische Potenzial des Subjekts auf metastatische Erkrankung im Frühstadium hinweist, und es festgestellt wird, dass das Subjekt einer Therapie bedarf, die auf die Immunzellen an der metastatischen Nische abzielt, wie etwa Phosphodiesterase-5- (PDE-5)- oder COX-2-Hemmer, die die Funktionalität myeloidabgeleiteter Suppressorzellen hemmen, oder
wenn der kombinierte Score des metastatischen Potenzials im Vergleich zum kombinierten Kontrollscore hoch ist, das metastatische Potenzial des Subjekts auf eine metastatische Erkrankung hinweist, und es festgestellt wird, dass das Subjekt einer Behandlung gegen metastatische Erkrankung bedarf, wobei die Behandlung gegen metastatische Erkrankung optional einen PARP-Hemmer oder Gemcitabin umfasst.

15. Verfahren nach einem der vorhergehenden Ansprüche, wobei die Immunzellen optional Makrophagen, dendritische Zellen, MDSCs, Neutrophile, Monozyten, T-Helferzellen, zytotoxische T-Zellen, B-Zellen, NK-Zellen, CD45+-Zellen oder eine Kombination aus zwei oder mehreren der Vorgenannten umfassen;
und wobei die Stromazellen optional Fibroblasten, Endothelzellen, Perizyten oder eine Kombination aus zwei oder mehreren der Vorgenannten umfassen.

16. Verfahren nach einem der vorhergehenden Ansprüche, wobei weniger als 5 % der Zellen in der Testprobe Krebszellen sind;
wobei optional weniger als 3 % oder weniger als 2 % oder weniger als 1 % oder weniger als 0,5 % oder weniger als 0,1 % der Zellen in der Testprobe Krebszellen sind.

17. Ein oder mehrere Mittel, die als Behandlungen gegen metastatische Erkrankung indiziert sind, zur Verwendung in einem Verfahren zur Behandlung von Metastasierung bei einem Subjekt, wobei das Verfahren nach einem der vorhergehenden Ansprüche durchgeführt wird und wobei das eine oder die mehreren Mittel gegen Metastasierung dann basierend auf dem metastatischen Potenzial des Subjekts dem Subjekt verabreicht werden.

## Revendications

1. Procédé de détermination du potentiel métastatique d'un sujet, ou de détermination du stade métastatique d'une tumeur ou d'un cancer, ou de détermination du besoin pour un sujet d'une thérapie contre une maladie métastatique, ou de détermination du traitement d'un sujet atteint d'une tumeur ou d'un cancer, ou de détermination de l'efficacité d'un traitement pour traiter une maladie métastatique, ou de détection d'une maladie métastatique ou d'une prédisposition à celle-ci, chez un sujet en ayant besoin,
le procédé comprenant :
la mesure des taux d'expression d'un panel de gènes, d'ARN ou de protéines dans un échantillon d'essai qui a été obtenu à partir d'une niche prémétastatique (pMN) synthétiquement modifiée implantée chez le sujet, l'échantillon d'essai comprenant des cellules immunitaires, des cellules stromales ou une combinaison de celles-ci ;
dans lequel les taux d'expression mesurés du panel de gènes, d'ARN ou de protéines dans l'échantillon d'essai sont comparés à des taux de contrôle ou au taux d'expression du panel de gènes, d'ARN ou de protéines dans un échantillon antérieur qui a été obtenu à partir du pMN synthétiquement modifié à un moment précédent, et dans lequel
le panel de gènes comprend l'un ou plusieurs des suivants : protéine de liaison au calcium S100 A8 (S100a8), protéine de liaison au calcium S100 A9 (S100a9), protéine de reconnaissance du peptidoglycane 1 (Pglyrpl), lactotransferrine (Ltf), peptide antimicrobien de cathélicidine (Camp), élastase 2 (Ela2), chitinase (Chi3l3), protéine morphogénétique osseuse 15 (Bmp15), ligand de chimiokine à motif C-C 22 (Ccl22) et récepteur de chimiokine à motif C-C 7 (Ccr7), ou
le panel d'ARN comprend un ou plusieurs ARN codés par la protéine de liaison au calcium S100 A8 (S100a8), la protéine de liaison au calcium S100 A9 (S100a9), la protéine de reconnaissance du peptidoglycane 1 (Pglyrpl), la lactotransferrine (Ltf), le peptide antimicrobien de cathélicidine (Camp), l'élastase 2 (Ela2), la chitinase (Chi3l3), la protéine morphogénétique osseuse 15 (Bmp15), le ligand de chimiokine à motif C-C 22 (Ccl22) et le récepteur de chimiokine à motif C-C 7 (Ccr7), ou
le panel de protéines comprend une ou plusieurs cytokines et/ou une ou plusieurs chimiokines.

2. Procédé selon la revendication 1, le procédé étant destiné à déterminer l'efficacité d'un traitement pour traiter une maladie métastatique, et dans lequel l'échantillon précédent a été obtenu avant le traitement et l'échantillon d'essai a été obtenu après le traitement,
le traitement pouvant éventuellement être une ablation chirurgicale d'une tumeur, ou une radiothérapie, ou l'administration d'un composé.

3. Procédé selon l'une quelconque des revendications précédentes, dans lequel les taux d'expression mesurés sont comparés à des taux de contrôle.

4. Procédé selon l'une quelconque des revendications précédentes, dans lequel les taux de contrôle des gènes, ARN ou protéines sont ceux d'un sujet connu pour avoir une maladie métastatique ou d'un sujet connu pour ne pas avoir de maladie métastatique.

5. Procédé selon l'une quelconque des revendications précédentes, dans lequel les taux d'expression mesurés du panel de gènes, d'ARN ou de protéines forment une signature d'expression pMN et dans lequel la signature d'expression de pMN est traitée au moyen d'un algorithme de décomposition pour obtenir un score unique pour l'expression génique et/ou un algorithme d'apprentissage automatique pour obtenir un score de prédiction de l'état de la maladie,
dans lequel l'algorithme de décomposition est éventuellement une décomposition en valeur singulière et/ou l'algorithme d'apprentissage automatique est une génération de forêt aléatoire.

6. Procédé selon la revendication 5, dans lequel le score unique pour l'expression génique et le score de prédiction de l'état de la maladie sont combinés pour fournir un score combiné de potentiel métastatique.

7. Procédé selon l'une quelconque des revendications précédentes, dans lequel les taux de contrôle des gènes, des ARN ou des protéines forment une signature d'expression de pMN de contrôle indiquant une maladie métastatique,
dans lequel la signature d'expression de pMN de contrôle indiquant une maladie métastatique est éventuellement traitée par un algorithme de décomposition pour obtenir un score unique pour l'expression génique et/ou un algorithme d'apprentissage automatique pour obtenir un score de prédiction de l'état de la maladie.

8. Procédé selon la revendication 7, dans lequel l'algorithme de décomposition est une décomposition en valeur singulière ou dans lequel l'algorithme d'apprentissage automatique est une génération de forêt aléatoire.

9. Procédé selon la revendication 7 ou la revendication 8, dans lequel la signature d'expression de pMN de contrôle est traitée par un algorithme de décomposition pour obtenir un score de contrôle unique pour l'expression génique et un algorithme d'apprentissage automatique pour obtenir un score de contrôle de prédiction de l'état de la maladie,
dans lequel le score de contrôle unique pour l'expression génique et le score de contrôle de prédiction de l'état de la maladie sont éventuellement combinés pour fournir un score de contrôle combiné.

10. Procédé selon la revendication 9, dans lequel le score combiné de potentiel métastatique est comparé au score de contrôle combiné pour déterminer le potentiel métastatique du sujet.

11. Procédé selon l'une quelconque des revendications précédentes, dans lequel les taux de contrôle des gènes, des ARN ou des protéines forment une signature d'expression de pMN de contrôle indiquant l'absence de maladie métastatique,
dans lequel la signature d'expression de pMN de contrôle indiquant l'absence de maladie métastatique est éventuellement traitée par un algorithme de décomposition pour obtenir un score unique pour l'expression génique et/ou un algorithme d'apprentissage automatique pour obtenir un score de prédiction de l'état de la maladie.

12. Procédé selon la revendication 11, dans lequel l'algorithme de décomposition est une décomposition en valeur singulière et/ou dans lequel l'algorithme d'apprentissage automatique est une génération de forêt aléatoire.

13. Procédé selon la revendication 11 ou la revendication 12, dans lequel le score de contrôle unique pour l'expression génique et le score de contrôle de prédiction de l'état de la maladie sont combinés pour fournir un score de contrôle combiné de l'absence de potentiel métastatique.
dans lequel le score combiné de potentiel métastatique est éventuellement comparé au score de contrôle combiné de l'absence de maladie métastatique pour déterminer le potentiel métastatique du sujet.

14. Procédé selon la revendication 13, dans lequel :
lorsque le score combiné de potentiel métastatique est faible, par rapport au score de contrôle combiné, le potentiel métastatique du sujet est faible et/ou le sujet est considéré comme sain, lorsque le score combiné de potentiel métastatique est intermédiaire, par rapport au score de contrôle combiné, le potentiel métastatique du sujet indique un stade précoce de maladie métastatique, et lorsque le score combiné du potentiel métastatique est élevé, par rapport au score de contrôle combiné, le potentiel métastatique du sujet indique une maladie métastatique, ou
lorsque le score combiné de potentiel métastatique est intermédiaire, par rapport au score de contrôle combiné, le potentiel métastatique du sujet indique un stade précoce de maladie métastatique, et il est déterminé que le sujet a besoin d'une thérapie qui cible les cellules immunitaires au niveau de la niche métastatique, telle que des inhibiteurs de phosphodiestérase 5 (PDE-5) ou de COX-2 qui inhibent la fonctionnalité des cellules myéloïdes suppressives, ou
lorsque le score combiné de potentiel métastatique est élevé, par rapport au score de contrôle combiné, le potentiel métastatique du sujet indique une maladie métastatique, et le sujet est déterminé comme ayant besoin d'un traitement antimétastatique, le traitement antimétastatique comprenant éventuellement un inhibiteur de PARP ou la gemcitabine.

15. Procédé selon l'une quelconque des revendications précédentes,
dans lequel les cellules immunitaires comprennent éventuellement des macrophages, des cellules dendritiques, des MDSC, des neutrophiles, des monocytes, des lymphocytes T auxiliaires, des lymphocytes T cytotoxiques, des lymphocytes B, des cellules NK, des cellules CD45+ ou une combinaison d'au moins deux parmi ceux-ci ;
et dans lequel les cellules stromales comprennent éventuellement des fibroblastes, des cellules endothéliales, des péricytes ou une combinaison d'au moins deux parmi ceux-ci.

16. Procédé selon l'une quelconque des revendications précédentes, dans lequel moins de 5 % des cellules dans l'échantillon d'essai sont des cellules cancéreuses ; dans lequel, éventuellement, moins de 3 %, ou moins de 2 %, ou moins de 1 %, ou moins de 0,5 %, ou moins de 0,1 % des cellules dans l'échantillon d'essai sont des cellules cancéreuses.

17. Un ou plusieurs agents indiqués comme traitements antimétastatiques d'une maladie, destinés à être utilisés dans un procédé de traitement des métastases chez un sujet, le procédé selon l'une quelconque des revendications précédentes étant mis en œuvre et les un ou plusieurs agents antimétastatiques étant ensuite administrés au sujet en fonction du potentiel métastatique du sujet.
